# EUROPEAN PATENT SPECIFICATION

(11) **EP 4 358 675 B1**
(45) Date of publication and mention of the grant of the patent: **29.04.2026**
(21) Application number: 23199908.7
(22) Date of filing: 26.09.2023
(51) Int. Cl.: H10K 39/34, H10K 59/13, H10K 59/35, H10K 85/60, H10K 30/20, C07D 471/06, C07D 493/06, C07D 517/16, C07D 517/22

(54) **ELECTRONIC APPARATUS INCLUDING ORGANIC PHOTODIODE AND ORGANIC LIGHT-EMITTING DEVICE**
ELEKTRONISCHE VORRICHTUNG MIT ORGANISCHER PHOTODIODE UND ORGANISCHE LICHTEMITTIERENDE VORRICHTUNG
APPAREIL ÉLECTRONIQUE COMPRENANT UNE PHOTODIODE ORGANIQUE ET DISPOSITIF ÉLECTROLUMINESCENT ORGANIQUE

(30) Priority: 11.10.2022 KR 20220130026
(43) Date of publication of application: 24.04.2024
(73) Proprietor: Samsung Display Co., Ltd., Yongin-si, Gyeonggi-do 17113 (KR)
(72) Inventor: JUNG, Jinsoo, 17113 Yongin-si (KR); YOON, Seokgyu, 17113 Yongin-si (KR); CHOI, Minsoo, 17113 Yongin-si (KR); CHOI, Youngeun, 17113 Yongin-si (KR)
(74) Representative: Marks & Clerk LLP

(56) References cited:
- WO-A1-2021/220141
- US-A1- 2022 173 174

## Description

### BACKGROUND

### 1. Field

One or more embodiments of the present disclosure relate to an electronic apparatus including an organic photodiode and an organic light-emitting device.

### 2. Description of the Related Art

Organic photodiodes (OPDs) use an organic semiconductor to absorb incident light, and convert it into a current. OPDs are advantageous over inorganic photodiodes, such as silicon photodetectors, in terms of ease of processing, cost reduction, application of flexible devices, and mass production, due to the characteristics of organic materials. In addition, by having a high extinction coefficient and being able to selectively absorb light in a set or specific wavelength according to a molecular structure, organic materials are very advantageous in terms of sensitivity improvement, wavelength selectivity, and high integration.

The structures of OPDs are very similar to those of organic light-emitting devices (OLEDs). In both devices, a photoactive layer or an emission layer may be included between a hole transport layer and an electron transport layer that are located between electrodes. Using such structural similarity, an OPD may be integrated into an OLED and used for fingerprint recognition.

US 2022/173174 A1 relates to a display device, a display module and an electronic device including such a display device.

WO 2021/220141 A1 relates to a display device, a display module and an electronic apparatus including such a display device.

### SUMMARY

One or more embodiments of the present disclosure include an electronic apparatus including an organic photodiode having improved external quantum efficiency (EQE) and an organic light-emitting device.

Additional aspects of embodiments will be set forth in part in the description which follows and, in part, will be apparent from the description, or may be learned by practice of the presented embodiments of the disclosure.

According to one or more embodiments, an electronic apparatus includes:
a substrate, and
a plurality of organic light-emitting devices and an organic photodiode that are arranged on the substrate.

The organic photodiode sequentially includes a first electrode, a first auxiliary layer, a photoactive layer, and a common electrode.

The plurality of the organic light-emitting devices include a first light-emitting device, a second light-emitting device, and a third light-emitting device.

The first light-emitting device sequentially includes a second electrode, a second auxiliary layer, a first emission layer, and a common electrode.

The second light-emitting device sequentially includes a third electrode, a third auxiliary layer, a second emission layer, and a common electrode.

The third light-emitting device sequentially includes a fourth electrode, a fourth auxiliary layer, a third emission layer, and a common electrode.

The photoactive layer has a thickness in a range of about 200 Å to about 300 Å.

The second auxiliary layer, the third auxiliary layer, and the fourth auxiliary layer have different thicknesses from each other.

The first auxiliary layer has an identical thickness to a thickness of one selected from the second auxiliary layer, the third auxiliary layer, and the fourth auxiliary layer.

In an embodiment, the first emission layer to third emission layer may each have a thickness in a range of 150 Å to 500 Å.

Thicknesses of the second auxiliary layer, the third auxiliary layer, and the fourth auxiliary layer may each be proportional to the wavelength of light emitted from each of the first light-emitting device, the second light-emitting device, and the third light-emitting device.

In an embodiment, the first light-emitting device may emit red light, the second light-emitting device may emit green light, and the third light-emitting device may emit blue light.

In an embodiment, a resonance distance of the organic photodiode may be identical to a resonance distance of the second light-emitting device. The first auxiliary layer may have an identical thickness to a thickness of the second auxiliary layer.

The organic photodiode may detect light emitted from the second light-emitting device and reflected from a subject.

The photoactive layer may include a p-type semiconductor and an n-type semiconductor.

The p-type semiconductor and the n-type semiconductor of the photoactive layer may have a planar heterojunction structure.

The p-type semiconductor and the n-type semiconductor of the photoactive layer may have a bulk heterojunction structure.

In an embodiment, the p-type semiconductor may include a donor compound represented by Formula 110: 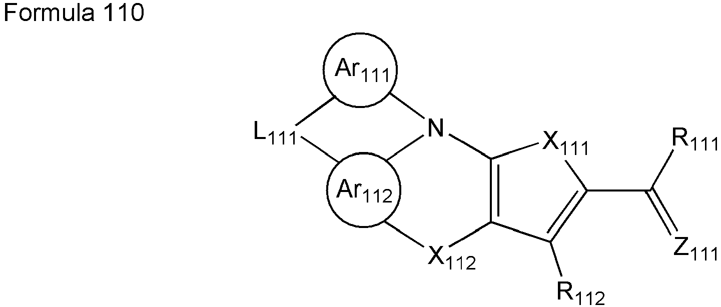 wherein, in Formula 110,
Ar₁₁₁ and Ar₁₁₂ are each independently a C₆-C₃₀ arylene group that is unsubstituted or substituted with at least one R₁₀ₐ or a C₃-C₃₀ heteroarylene group that is unsubstituted or substituted with at least one R₁₀ₐ,
X₁₁₁ is selected from -Se-, -Te-, -S(=O)-, -S(=O)₂-, -N(Q₁₁₁)-, -B(Q₁₁₁)-, - C(Q₁₁₁)(Q₁₁₂)-, -Si(Q₁₁₁)(Q₁₁₂)-, and -Ge(Q₁₁₁)(Q₁₁₂)-,
X₁₁₂ and L₁₁₁ are each independently selected from -O-, -S-, -Se-, -Te-, - S(=O)-, -S(=O)₂-, -N(Q₁₁₁)-, -B(Q₁₁₁)-, -C(Q₁₁₁)(Q₁₁₂)-, -Si(Q₁₁₁)(Q₁₁₂)-, -Ge(Q₁₁₁)(Q₁₁₂)-, -(C(Q₁₁₁)=C(Q₁₁₂))-, and -(C(Q₁₁₁)=N))-,
when L₁₁₁ is selected from -N(Q₁₁₁)-, -B(Q₁₁₁)-, -C(Q₁₁₁)(Q₁₁₂)-, - Si(Q₁₁₁)(Q₁₁₂)-, -Ge(Q₁₁₁)(Q₁₁₂)-, -(C(Q₁₁₁)=C(Q₁₁₂))-, and -(C(Q₁₁₁)=N))-, L₁₁₁ is optionally linked to Ar₁₁₁ or Ar₁₁₂ to form a condensed ring,
Z₁₁₁ is a C₆-C₃₀ carbocyclic group that is unsubstituted or substituted with at least one R₁₀ₐ and has at least one functional group selected from C=O, C=S, C=Se, and C=Te, or a C₁-C₃₀ heterocyclic group that is unsubstituted or substituted with at least one R₁₀ₐ and has at least one functional group selected from C=O, C=S, C=Se, and C=Te,
R₁₁₁ to R₁₁₂ are each independently hydrogen, deuterium, a halogen, a cyano group, a nitro group, a hydroxy group, a C₁-C₃₀ alkyl group unsubstituted or substituted with at least one R₁₀ₐ, a C₁-C₃₀ alkoxy group unsubstituted or substituted with at least one R₁₀ₐ, a C₆-C₃₀ aryl group unsubstituted or substituted with at least one R₁₀ₐ, a C₃-C₃₀ heteroaryl group unsubstituted or substituted with at least one R₁₀ₐ, a C₂-C₃₀ acyl group unsubstituted or substituted with at least one R₁₀ₐ, or any combination thereof,
R₁₀ₐ is:
deuterium, -F, -Cl, -Br, -I, a hydroxyl group, a cyano group, or a nitro group; a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, C₆-C₆₀ aryloxy group, a C₆-C₆₀ arylthio group, a C₁-C₆₀ heteroaryloxy group, a C₁-C₆₀ heteroarylthio group, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), -P(=O)(Q₁₁)(Q₁₂), or any combination thereof,
a C₃-C₆₀ carbocyclic group, a C₁-C₆₀ heterocyclic group, a C₆-C₆₀ aryloxy group, a C₆-C₆₀ arylthio group, a C₁-C₆₀ heteroaryloxy group, or a C₁-C₆₀ heteroarylthio group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₁-C₆₀ heteroaryloxy group, a C₁-C₆₀ heteroarylthio group, -Si(Q₂₁)(Q₂₂)(Q₂₃), -N(Q₂₁)(Q₂₂), - B(Q₂₁)(Q₂₂), -C(=O)(Q₂₁), -S(=O)₂(Q₂₁), -P(=O)(Q₂₁)(Q₂₂), or any combination thereof, or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁₁ to Q₁₃, Q₂₁ to Q₂₃, Q₃₁ to Q₃₃, Q₁₁₁, and Q₁₁₂ are each independently hydrogen, deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

In an embodiment, Z₁₁₁ in Formula 110 may be a group represented by one selected from Formulae 111A to 111F: 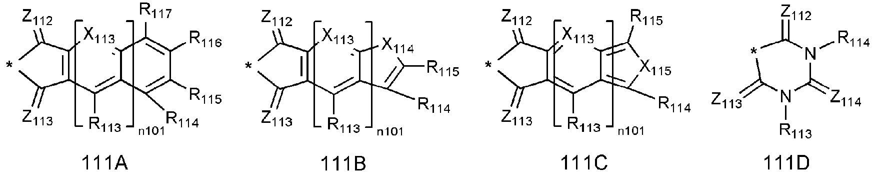 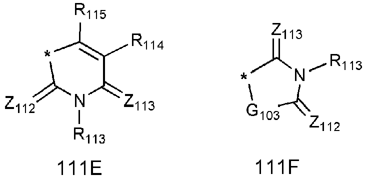 wherein, in Formulae 111A to 111F,
Z₁₁₂ to Z₁₁₄ are each independently O, S, Se, or Te,
G₁₀₃ may be O, S, Se, or Te,
X₁₁₃ is N or C(Q₁₁₃), and
X₁₁₄ and X₁₁₅ are each independently O, S, Se, Te, Si(Q₁₁₁)(Q₁₁₂), or Ge(Q₁₁₁)(Q₁₁₂).
n101 is an integer from 0 to 3,
R₁₁₃ to R₁₁₇ are each independently as defined with respect to R₁₂₁, and
Q₁₁₁ to Q₁₁₃ are each independently as defined with respect to Q₁₁₁.

In an embodiment, the compound represented by Formula 110 may be a compound selected from compounds of Groups 1 to 3.

In an embodiment, the n-type semiconductor may include 3,4,9,10-perylenetetracarboxylic dianhydride (PTCDA), N,N'-di(propoxyethyl)perylene-3,4,9,10-tetracarboxylic diimide (PTCDI), naphthalene tetracarboxylic anhydride (NTCDA), naphthalene tetracarboxylic diimide (NTCDI), and/or a derivative thereof, a fullerene derivative, or any combination thereof.

For example, the n-type semiconductor may include a compound represented by Formula 120: 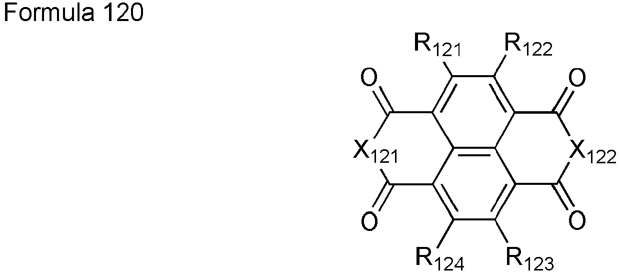 wherein, in Formula 120,
X₁₂₁ and X₁₂₂ are each independently O or NR₁₂₅, and
R₁₂₁ to R₁₂₄ and R₁₂₅ are each independently hydrogen, deuterium, a halogen, a cyano group, a nitro group, a hydroxy group, a C₁-C₃₀ alkyl group substituted or unsubstituted with at least one R₁₀ₐ, a C₆-C₃₀ aryl group substituted or unsubstituted with at least one R₁₀ₐ, a C₃-C₃₀ heteroaryl group substituted or unsubstituted with at least one R₁₀ₐ, or any combination thereof.

A first common layer may be included between the first electrode and the first auxiliary layer, between the second electrode and the second auxiliary layer, between the third electrode and the third auxiliary layer, and between the fourth electrode and the fourth auxiliary layer.

A second common layer may be included between the photoactive layer and the common electrode, between the first emission layer and the common electrode, between the second emission layer and the common electrode, and between the third emission layer and the common electrode.

The first common layer may include at least one selected from a hole injection layer, a hole transport layer, and an electron blocking layer.

The second common layer may include at least one selected from a buffer layer, a hole blocking layer, an electron transport layer, and an electron injection layer.

The first auxiliary layer may be in direct contact with the first emission layer, the second auxiliary layer may be in direct contact with the second emission layer, the third auxiliary layer may be in direct contact with the third emission layer, and the fourth auxiliary layer may be in direct contact with the photoactive layer.

A material for forming the first auxiliary layer, the second auxiliary layer, the third auxiliary layer, and the fourth auxiliary layer may include, for example, a fluorene-based compound, a carbazole-based compound, an arylamine-based compound, a dibenzofuran-based compound, a dibenzothiophene-based compound, and/or a dibenzosilole-based compound.

In an embodiment, the plurality of the organic light-emitting devices and the organic photodiode may form a pixel, and the electronic apparatus may include a plurality of the pixels.

In an embodiment, the electronic apparatus may include a plurality of the pixels, and the plurality of the pixels may all include the organic photodiode.

In an embodiment, the electronic apparatus may further include a fourth light-emitting device, and the fourth light-emitting device may be identical to one selected from the first light-emitting device, the second light-emitting device, and the third light-emitting device.

In an embodiment, the organic photodiode may be arranged adjacent to the second light-emitting device.

The electronic apparatus may be a display apparatus.

At least some of the above and other features of the invention are set out in the claims.

### BRIEF DESCRIPTION OF THE DRAWINGS

The above and other aspects and features of certain embodiments of the disclosure will be more apparent from the following description taken in conjunction with the accompanying drawings, in which:
FIG. 1 is a schematic cross-sectional view of an organic photodiode according to an embodiment;
FIG. 2 is a schematic cross-sectional view of an organic photodiode according to another embodiment;
FIG. 3 is a schematic cross-sectional view of an electronic apparatus according to an embodiment;
FIG. 4 is a schematic cross-sectional view of an electronic apparatus according to another embodiment;
FIG. 5 is a view of an electronic apparatus according to an embodiment; and
FIG. 6 is a view of an electronic apparatus according to another embodiment.

### DETAILED DESCRIPTION

Reference will now be made in more detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of embodiments of the present description. As used herein, the term "and/or" includes any and all combinations of one or more of the associated listed items. Throughout the disclosure, the expression "at least one of a, b or c" indicates only a, only b, only c, both a and b, both a and c, both b and c, all of a, b, and c, or variations thereof.

As the present disclosure allows for various suitable changes and numerous embodiments, example embodiments will be illustrated in the drawings and described in more detail in the written description. An effect and a characteristic of the disclosure, and a method of accomplishing these will be apparent when referring to embodiments described with reference to the drawings. The subject matter of the disclosure may, however, be embodied in many different forms and should not be construed as limited to the embodiments set forth herein.

It will be understood that although the terms "first," "second," etc. used herein may be used herein to describe various components, these components should not be limited by these terms. These components are only used to distinguish one component from another.

An expression used in the singular encompasses the expression of the plural, unless it has a clearly different meaning in the context.

In the following embodiments, when various components such as layers, films, regions, plates, etc. are said to be "on" another component, this may include not only a case in which other components are "immediately on" the layers, films, regions, or plates, but also a case in which other components may be placed therebetween. Sizes of elements in the drawings may be exaggerated for convenience of explanation. In other words, because sizes and thicknesses of components in the drawings may be arbitrarily illustrated for convenience of explanation, the following embodiments are not limited thereto.

It will be further understood that the terms "includes" and/or "comprises" used herein specify the presence of stated features or elements, but do not preclude the presence or addition of one or more other features or elements. Unless defined otherwise, the terms "include or have" may refer to both the case of consisting of features or components described in a specification and the case of further including other components.

### Organic photodiode

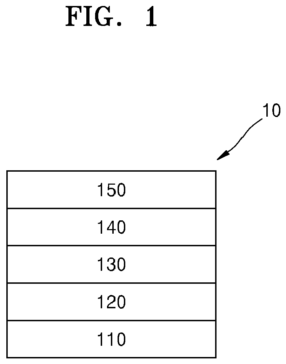

Hereinafter, an organic photodiode according to an embodiment will be described in more detail in reference to FIGS. 1-2.

### Description of FIGS. 1-2

An organic photodiode included in an electronic apparatus will be described with reference to FIGS. 1-2.

FIG. 1 is a schematic cross-sectional view of an organic photodiode 10 according to an embodiment.

Referring to FIG. 1, the organic photodiode 10 according to an embodiment sequentially includes a first electrode 110, a hole transport region 120, a photoactive layer 130, an electron transport region 140, and a second electrode 150.

One selected from the first electrode 110 and the second electrode 150 may be an anode, and the other may be a cathode. For example, the first electrode 110 may be an anode, and the second electrode 150 may be a cathode.

The photoactive layer 130 may absorb incident light to form excitons, and charges are separated from the excitons to form electrons and holes.

The holes generated in the photoactive layer 130 may move to the first electrode 110 through the hole transport region 120, and the electrons generated in the photoactive layer 130 may move to the second electrode 150 through the electron transport region 140, thereby generating photocurrent and detecting light.

In an embodiment, the photoactive layer 130 may include a p-type semiconductor layer including a p-type semiconductor and an n-type semiconductor layer including the n-type semiconductor, wherein the p-type semiconductor layer and the n-type semiconductor layer form a PN junction. The PN junction may include, for example, a planar heterojunction or a bulk heterojunction.

Because the p-type semiconductor acts as an electron donor, and the n-type semiconductor acts as an electron acceptor, excitons can be efficiently separated into holes and electrons by photo-induced charge separation occurring at the interface between the p-type semiconductor layer and the n-type semiconductor layer. Furthermore, as the photoactive layer is separated into the p-type semiconductor layer and the n-type semiconductor layer, the holes and electrons generated at the interface may be easily captured and moved.

In one or more embodiments, the photoactive layer 130 may be a mixed layer in which the p-type semiconductor and the n-type semiconductor are mixed together. In this case, the p-type semiconductor and the n-type semiconductor may be co-deposited.

The p-type semiconductor may include a compound serving as an electron donor.

In an embodiment, the donor layer, e.g., a p-type semiconductor, may include a donor compound represented by Formula 110: 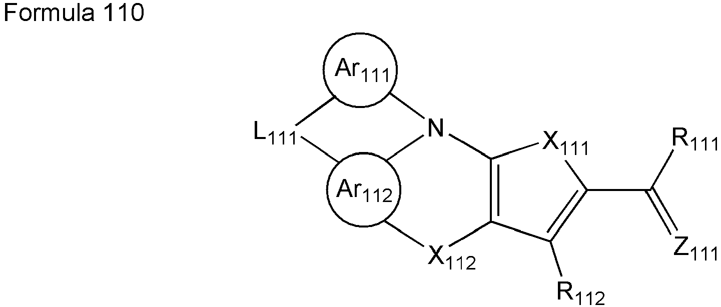 wherein, in Formula 110,
Ar₁₁₁ and Ar₁₁₂ are each independently a C₆-C₃₀ (*e.g.* C₆-C₂₀) arylene group that is unsubstituted or substituted with at least one R₁₀ₐ or a C₃-C₃₀ (*e.g.* C₃-C₂₀) heteroarylene group that is unsubstituted or substituted with at least one R₁₀ₐ,
X₁₁₁ is selected from -Se-, -Te-, -S(=O)-, -S(=O)₂-, -N(Q₁₁₁)-, -B(Q₁₁₁)-, - C(Q₁₁₁)(Q₁₁₂)-, -Si(Q₁₁₁)(Q₁₁₂)-, and -Ge(Q₁₁₁)(Q₁₁₂)-,
X₁₁₂ and L₁₁₁ are each independently selected from -O-, -S-, -Se-, -Te-, - S(=O)-, -S(=O)₂-, -N(Q₁₁₁)-, -B(Q₁₁₁)-, -C(Q₁₁₁)(Q₁₁₂)-, -Si(Q₁₁₁)(Q₁₁₂)-, -Ge(Q₁₁₁)(Q₁₁₂)-, -(C(Q₁₁₁)=C(Q₁₁₂))-, and -(C(Q₁₁₁)=N))-,
when L₁₁₁ is selected from -N(Q₁₁₁)-, -B(Q₁₁₁)-, -C(Q₁₁₁)(Q₁₁₂)-, - Si(Q₁₁₁)(Q₁₁₂)-, -Ge(Q₁₁₁)(Q₁₁₂)-, -(C(Q₁₁₁)=C(Q₁₁₂))-, and -(C(Q₁₁₁)=N))-, L₁₁₁ is optionally linked to Ar₁₁₁ or Ar₁₁₂ to form a condensed ring,
Z₁₁₁ is a C₆-C₃₀ (*e.g.* C₆-C₂₀) carbocyclic group that is unsubstituted or substituted with at least one R₁₀ₐ and has at least one functional group selected from C=O, C=S, C=Se, and C=Te, or a C₁-C₃₀ (*e.g.* C₁-C₂₀) heterocyclic group that is unsubstituted or substituted with at least one R₁₀ₐ and has at least one functional group selected from C=O, C=S, C=Se, and C=Te, and
R₁₁₁ to R₁₁₂ are each independently hydrogen, deuterium, a halogen, a cyano group, a nitro group, a hydroxy group, a C₁-C₃₀ (*e.g.* C₁-C₂₀) alkyl group unsubstituted or substituted with at least one R₁₀ₐ, a C₁-C₃₀ (*e.g.* C₁-C₂₀) alkoxy group unsubstituted or substituted with at least one R₁₀ₐ, a C₆-C₃₀ (*e.g.* C₆-C₂₀) aryl group unsubstituted or substituted with at least one R₁₀ₐ, a C₃-C₃₀ (*e.g.* C₃-C₂₀) heteroaryl group unsubstituted or substituted with at least one R₁₀ₐ, a C₂-C₃₀ (*e.g.* C₂-C₂₀) acyl group unsubstituted or substituted with at least one R₁₀ₐ, or any combination thereof.
R₁₀ₐ is:
deuterium, -F, -Cl, -Br, -I, a hydroxyl group, a cyano group, or a nitro group; a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, or a C₁-C₆₀ (*e.g.* C₁-C₂₀) alkoxy group, each unsubstituted or substituted with deuterium, -F, -Cl, -Br, -I, a hydroxyl group, a cyano group, a nitro group, a C₃-C₆₀ (*e.g.* C₃-C₃₀) carbocyclic group, a C₁-C₆₀ (*e.g.* C₁-C₂₀) heterocyclic group, C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryloxy group, a C₁-C₆₀ (*e.g.* C₁-C₂₀) heteroarylthio group, - Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), - P(=O)(Q₁₁)(Q₁₂), or any combination thereof;
a C₃-C₆₀ (*e.g.* C₃-C₃₀) carbocyclic group, a C₁-C₆₀ (*e.g.* C₁-C₂₀) heterocyclic group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryloxy group, or a C₁-C₆₀ (*e.g.* C₁-C₂₀) heteroarylthio group, each unsubstituted or substituted with deuterium, -F, -Cl, -Br, -I, a hydroxyl group, a cyano group, a nitro group, a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g.* C₁-C₂₀) alkoxy group, a C₃-C₆₀ (*e.g.* C₃-C₃₀) carbocyclic group, a C₁-C₆₀ (*e.g.* C₁-C₂₀) heterocyclic group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryloxy group, a C₁-C₆₀ (*e.g.* C₁-C₂₀) heteroarylthio group, - Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), - P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or - P(=O)(Q₃₁)(Q₃₂), and
Q₁₁ to Q₁₃, Q₂₁ to Q₂₃, Q₃₁ to Q₃₃, Q₁₁₁, and Q₁₁₂ are each independently: hydrogen; deuterium; -F; -Cl; -Br; -I; a hydroxyl group; a cyano group; a nitro group; a C₁-C₆₀ (*e.g.* C₁-C₂₀) alkyl group; a C₂-C₆₀ (e.g. C₂-C₂₀) alkenyl group; a C₂-C₆₀ (*e.g.* C₂-C₂₀) alkynyl group; a C₁-C₆₀ (*e.g.* C₁-C₂₀) alkoxy group; or a C₃-C₆₀ (*e.g.* C₃-C₂₀) carbocyclic group or a C₁-C₆₀ (*e.g.* C₁-C₂₀) heterocyclic group, each unsubstituted or substituted with deuterium, -F, a cyano group, a C₁-C₆₀ (e*.g.* C₁-C₂₀) alkyl group, a C₁-C₆₀ (*e.g.* C₁-C₂₀) alkoxy group, a phenyl group, a biphenyl group, or any combination thereof.

In an embodiment, Z₁₁₁ in Formula 110 may be a group represented by one selected from Formulae 111A to 111F: 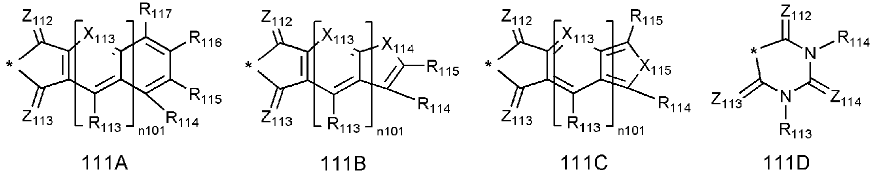 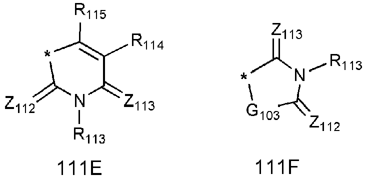 wherein, in Formulae 111A to 111F,
Z₁₁₂ to Z₁₁₄ are each independently O, S, Se, or Te,
X₁₁₃ is N or C(Q₁₁₃),
X₁₁₄ and X₁₁₅ are each independently O, S, Se, Te, Si(Q₁₁₁)(Q₁₁₂), or Ge(Q₁₁₁)(Q₁₁₂),
n101 is an integer from 0 to 3,
R₁₁₃ to R₁₁₇ are each independently as defined with respect to R₁₂₁, and
Q₁₁₁ to Q₁₁₃ are each independently as defined with respect to Q₁₁₁.

In an embodiment, the compound represented by Formula 110 may be a compound selected from compounds of Groups 1 to 3:

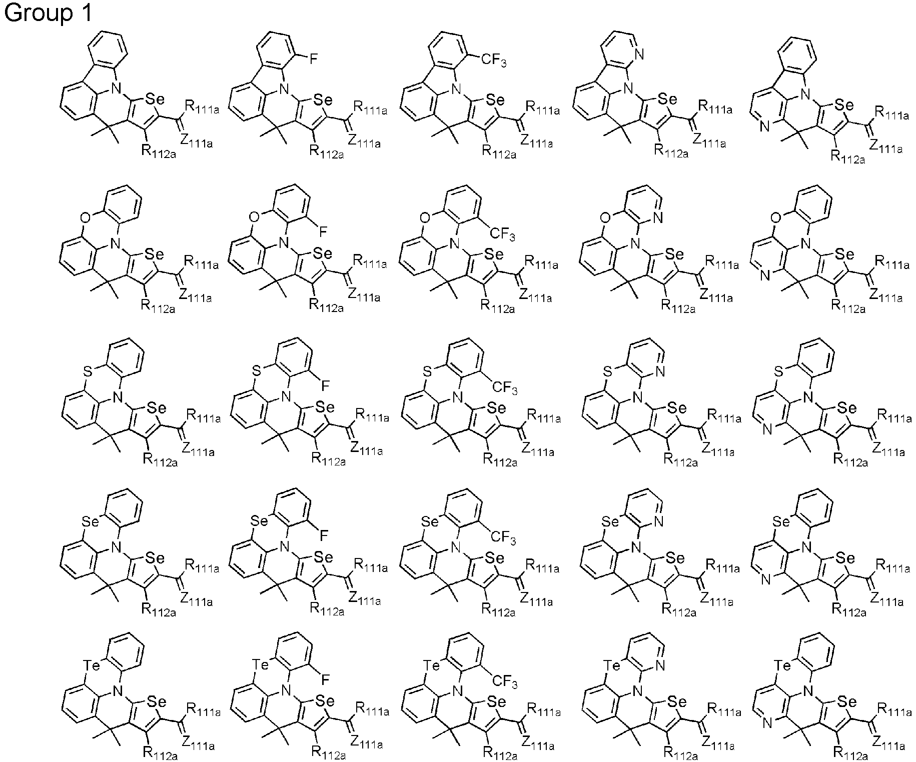

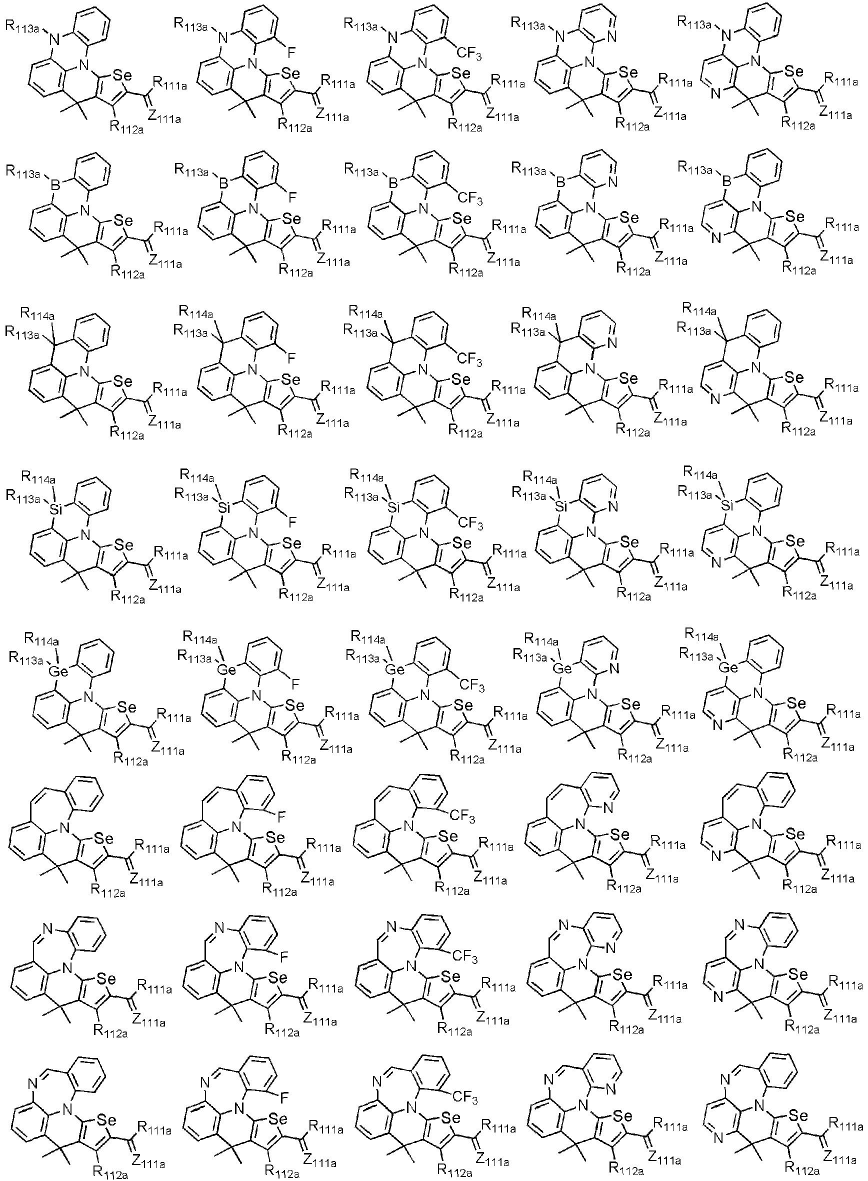

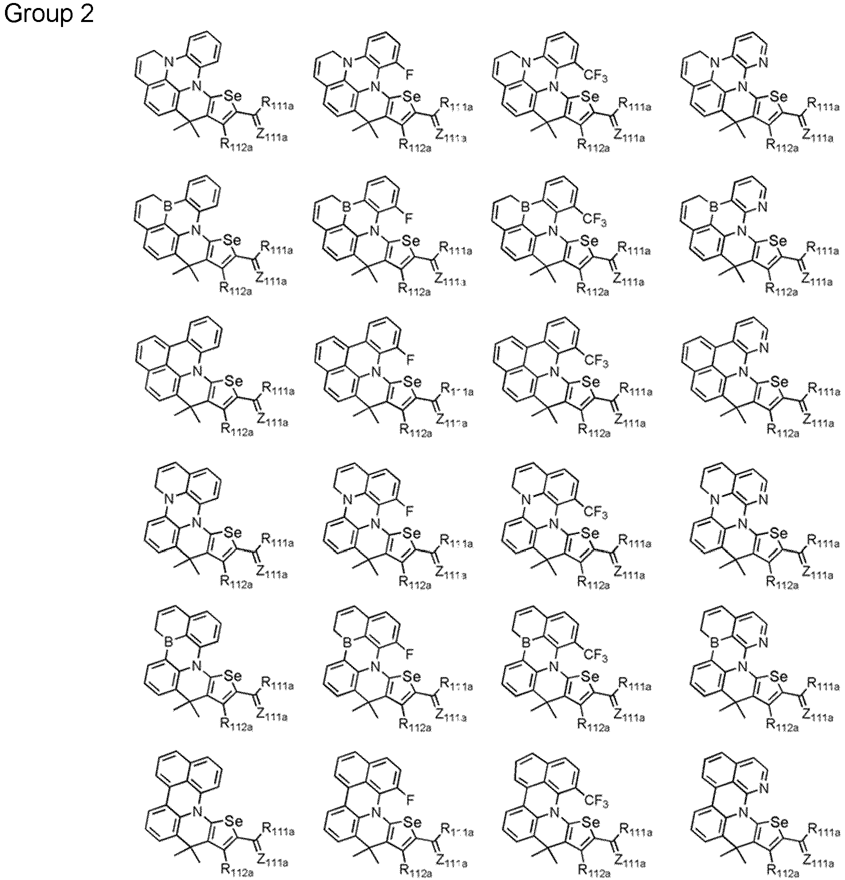

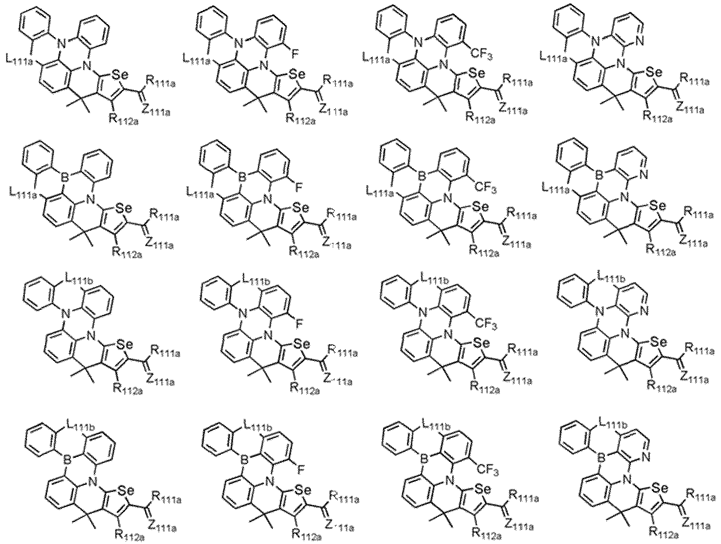

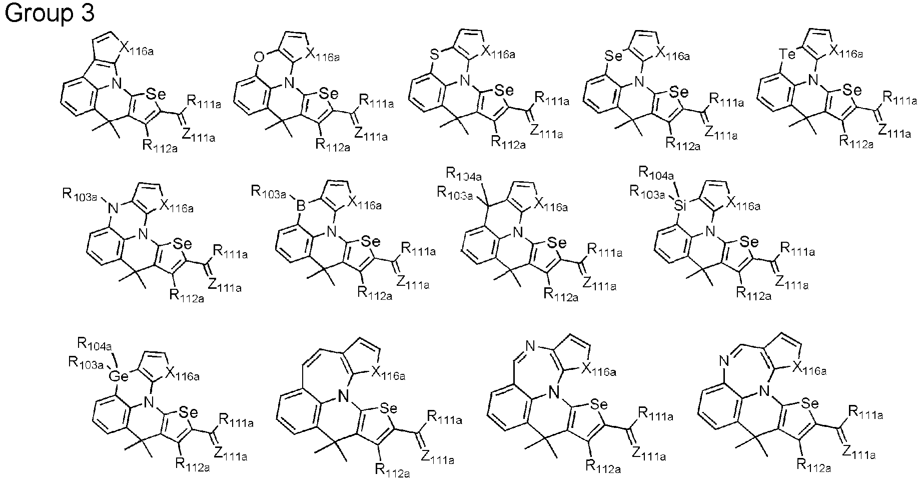

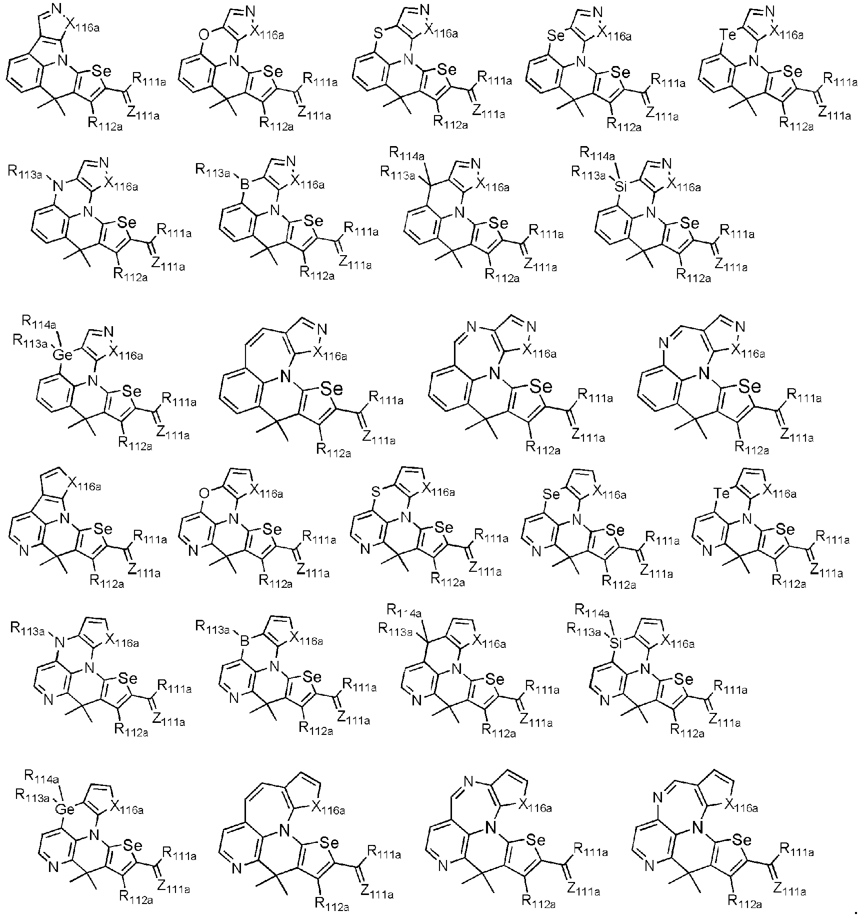

In the compounds of Groups 1 to 3,
R₁₀₃ₐ, R₁₀₄ₐ, and R₁₁₁ₐ to R₁₁₄ₐ are each independently as defined with respect to R₁₁₁,
L₁₁₁ₐ and L_{111b} are each independently as described for L₁₁₁,
Z₁₁₁ₐ is as defined with respect to Z₁₁₁, and
X₁₁₆ₐ is as defined with respect to X₁₁₂.

In an embodiment, R₁₀₃ₐ, R₁₀₄ₐ, and R₁₁₁ₐ to R₁₁₄ₐ may each independently be hydrogen, a C₁-C₃₀ (*e.g.* C₁-C₂₀) alkyl group, a C₆-C₃₀ (*e.g.* C₆-C₂₀) aryl group, or a C₃-C₃₀ (*e.g*. C₃-C₂₀) heteroaryl group. For example, R₁₁₁ₐ and R₁₁₂ₐ may each independently be hydrogen. For example, R₁₀₃ₐ, R₁₀₄ₐ, R₁₁₃ₐ and R₁₁₄ₐ may each independently be hydrogen, a methyl group, or a phenyl group.

In an embodiment, Z₁₁₁ₐ may be a group represented by Formula 111A. For example, in Formula 111A, Z₁₁₂ and Z₁₁₃ may each independently be O, X₁₁₃ may be N or CH, and R₁₁₃ to R₁₁₇ may each independently be hydrogen.

The n-type semiconductor may include a compound serving as an electron acceptor. For example, the n-type semiconductor may include 3,4,9,10-perylenetetracarboxylic dianhydride (PTCDA), N,N'-di(propoxyethyl)perylene-3,4,9,10-tetracarboxylic diimide (PTCDI), naphthalene tetracarboxylic anhydride (NTCDA), naphthalenetetracarboxylic diimide (NTCDI), and/or a derivative thereof, a fullerene derivative, or any combination thereof.

In an embodiment, the n-type semiconductor may include a compound represented by Formula 120: 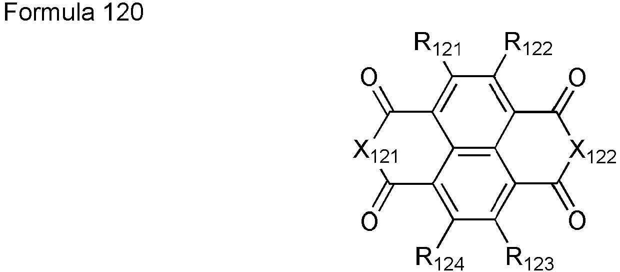 wherein, in Formula 120,
X₁₂₁ and X₁₂₂ are each independently O or NR₁₂₅, and
R₁₂₁ to R₁₂₄ and R₁₂₅ are each independently hydrogen, deuterium, a halogen, a cyano group, a nitro group, a hydroxy group, a C₁-C₃₀ (*e.g.* C₁-C₂₀) alkyl group substituted or unsubstituted with at least one R₁₀ₐ, a C₆-C₃₀ *(*e.g. C₆-C₂₀) aryl group substituted or unsubstituted with at least one R₁₀ₐ, a C₃-C₃₀ (*e.g.* C₃-C₂₀) heteroaryl group substituted or unsubstituted with at least one R₁₀ₐ, or any combination thereof.

In an embodiment, the compound represented by Formula 120 may be a compound represented by Formula 121 or 122: 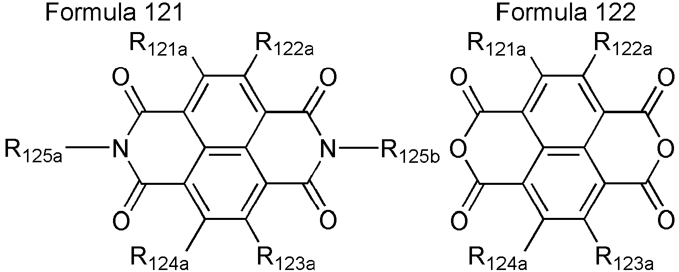 wherein, in Formulae 121 and 122,
R₁₂₁ₐ to R₁₂₄ₐ are each independently hydrogen, deuterium, a halogen, a cyano group, a C₁-C₃₀ (*e.g.* C₁-C₂₀) alkyl group, a C₆-C₃₀ (*e.g.* C₆-C₂₀) aryl group, or a C₃-C₃₀ (*e.g.* C₃-C₂₀) heteroaryl group, and
R₁₂₅ₐ and R_{125b} are each independently: hydrogen, deuterium, a halogen, or a cyano group; a C₁-C₃₀ (*e.g.* C₁-C₂₀) alkyl group that is unsubstituted or substituted with at least one R₁₀ₐ; a C₆-C₃₀ (*e.g.* C₆-C₂₀) aryl group that is unsubstituted or substituted with at least one R₁₀ₐ or a C₃-C₃₀ (*e.g.* C₃-C₂₀) heteroaryl group that is unsubstituted or substituted with at least one R₁₀ₐ.

In an embodiment, R₁₂₁ₐ to R₁₂₄ₐ may each independently be hydrogen, deuterium, a halogen, a cyano group, a methyl group, or a phenyl group. For example, R₁₂₁ₐ to R₁₂₄ₐ may each independently be hydrogen.

In an embodiment, R₁₂₅ₐ and R_{125b} may each independently be: hydrogen, a halogen, or a cyano group; a C₁-C₃₀ (*e.g.* C₁-C₂₀) alkyl group; or a C₆-C₃₀ (*e.g.* C₆-C₂₀) aryl group that is substituted with an alkyl group substituted with a halogen, a cyano group, an alkyl group, or a halogen.

In an embodiment, the compound represented by Formula 120 may be selected from the following compounds:

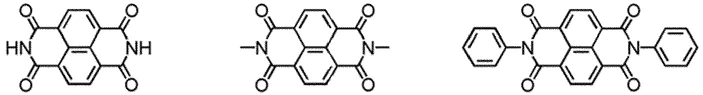

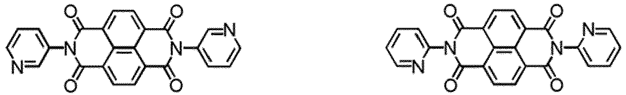

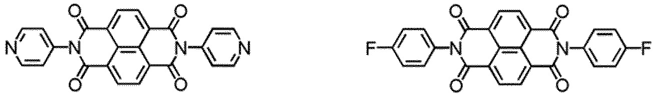

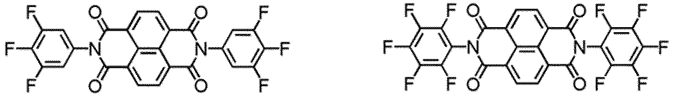

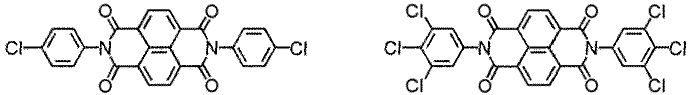

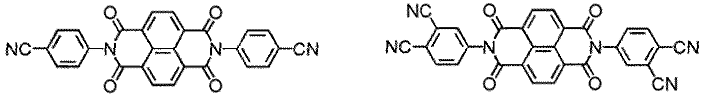

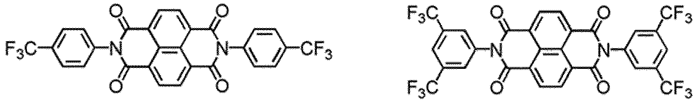

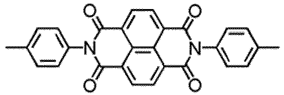

The film quality of the photoactive layer may affect the external quantum efficiency of the organic photodiode. In the organic photodiode 10 according to an embodiment, the photoactive layer 130 is formed as a high-quality thin film, and thus the external quantum efficiency may be further improved as compared with existing organic photodiodes. A thickness of the photoactive layer 130 is 200 Å to 300 Å.

The hole transport region 120 may further include an auxiliary layer that is in contact with the photoactive layer 130. The auxiliary layer may serve to improve light absorption efficiency by compensating for an optical resonance distance according to a wavelength of light incident on the photoactive layer 130. In addition, the auxiliary layer may serve to smooth the movement of holes by lowering an energy barrier of holes moving in the direction toward the hole transport layer and the anode.

The auxiliary layer may be, for example, formed of a material, such as a fluorene-based compound, a carbazole-based compound, an arylamine-based compound, a dibenzofuran-based compound, a dibenzothiophene-based compound, and/or a dibenzosilole-based compound. In an embodiment, the auxiliary layer may include (or consist of) a compound having a refractive index in a range of 1.7 to 2.0.

The first electrode 110, the second electrode 150, the hole transport region 120, and the electron transport region 140 of the organic photodiode 10 may each be formed of the same materials as materials for forming an anode, a cathode, a hole transport region, and an electron transport region of an organic light-emitting device, respectively.

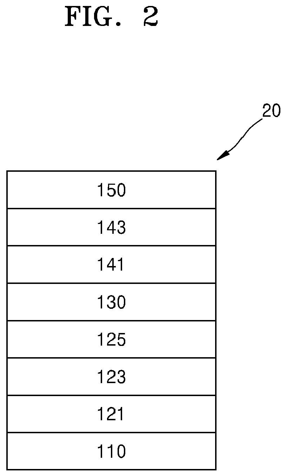

FIG. 2 is a schematic cross-sectional view of an organic photodiode 20 according to another embodiment.

Referring to FIG. 2, a hole injection layer 121, a hole transport layer 123, and an auxiliary layer 125 are arranged between the first electrode 110 and the photoactive layer 130. Here, the photoactive layer 130 is in direct contact with the auxiliary layer 125, and the auxiliary layer 125 is in direct contact with the hole transport layer 123. As used herein, the term "direct contact" may mean direct physical contact between two elements with no other intervening elements between the two elements. In an embodiment, the organic photodiode 20 may further include an electron blocking layer between the photoactive layer 130 and the auxiliary layer 125.

An electron transport layer 141 and an electron injection layer 143 are arranged between the photoactive layer 130 and the second electrode 150. In an embodiment, the organic photodiode 20 may further include a hole blocking layer between the photoactive layer 130 and the electron transport layer 141.

A description of the photoactive layer 130 of the organic photodiode 20 may refer to that of the photoactive layer 130 of the organic photodiode 10.

Hereinafter, the layers constituting the organic photodiodes 10 and 20 of FIGS. 1-2 will be described in more detail.

### First electrode 110

In FIGS. 1-2, a substrate may be additionally arranged under the first electrode 110 and/or on the second electrode 150. In an embodiment, as the substrate, a glass substrate and/or a plastic substrate may be used. In one or more embodiments, the substrate may be a flexible substrate, and may include plastics having excellent heat resistance and durability, such as polyimide, polyethylene terephthalate (PET), polycarbonate, polyethylene naphthalate, polyarylate (PAR), polyetherimide, or any combination thereof.

The first electrode 110 may be formed by, for example, depositing and/or sputtering a material for forming the first electrode 110 on the substrate. When the first electrode 110 is an anode, the material for forming the first electrode 110 may be a high-work function material.

The first electrode 110 may be a reflective electrode, a semi-transmissive electrode, or a transmissive electrode. When the first electrode 110 is a transmissive electrode, indium tin oxide (ITO), indium zinc oxide (IZO), tin oxide (SnO₂), zinc oxide (ZnO), or any combination thereof may be used as a material for forming the first electrode 110. In one or more embodiments, when the first electrode 110 is a semi-transmissive electrode or a reflective electrode, magnesium (Mg), silver (Ag), aluminium (Al), aluminium-lithium (Al-Li), calcium (Ca), magnesium-indium (Mg-In), magnesium-silver (Mg-Ag), or any combinations thereof may be used as a material for forming the first electrode 110.

The first electrode 110 may have a single-layer structure consisting of a single layer or a multi-layer structure including a plurality of layers. For example, the first electrode 110 may have a three-layer structure of ITO/Ag/ITO.

### Charge auxiliary layer

The organic photodiode 10 or 20 according to an embodiment may include a charge auxiliary layer that facilitates the transport of holes and electrons separated from the photoactive layer 130. The charge auxiliary layer may include a hole injection layer and a hole transport layer to facilitate the transport of holes, and an electron transport layer and an electron injection layer to facilitate the transport of electrons.

### Hole transport region

The charge auxiliary layer(s) arranged between the first electrode 110 and the photoactive layer 130 may be collectively referred to as the hole transport region 120. The hole transport region 120 may facilitate the transport of holes generated in the photoactive layer 130.

The hole transport region 120 may include a hole transport layer, a hole injection layer, an electron blocking layer, or a combination thereof.

The hole transport region 120 may include a hole transport material. For example, the hole transport material may include a compound represented by Formula 201, a compound represented by Formula 202, or any combination thereof: 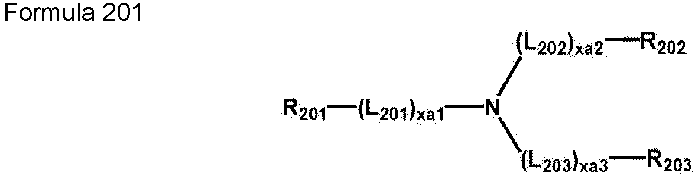 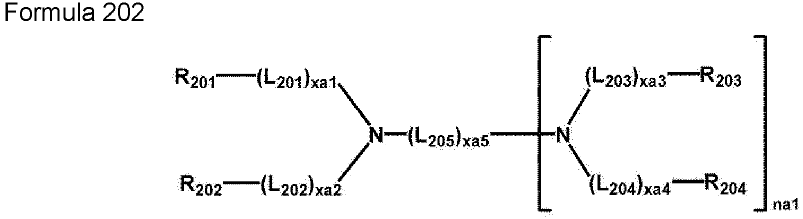 wherein, in Formulae 201 and 202,
L₂₀₁ to L₂₀₄ may each independently be a C₃-C₆₀ (*e.g.* C₃-C₃₀) carbocyclic group that is unsubstituted or substituted with at least one R₁₀ₐ or a C₁-C₆₀ (e.g. C₁-C₂₀) heterocyclic group that is unsubstituted or substituted with at least one R₁₀ₐ,
L₂₀₅ may be *-O-*', *-S-*', *-N(Q₂₀₁)-*', a C₁-C₂₀ (e.g. C₁-C₁₀) alkylene group unsubstituted or substituted with at least one R₁₀ₐ, a C₂-C₂₀ (e.g. C₂-C₁₀) alkenylene group unsubstituted or substituted with at least one R₁₀ₐ, a C₃-C₆₀ (*e.g.* C₃-C₃₀) carbocyclic group unsubstituted or substituted with at least one R₁₀ₐ, or a C₁-C₆₀ (e.g. C₁-C₂₀) heterocyclic group unsubstituted or substituted with at least one R₁₀ₐ,
xa1 to xa4 may each independently be an integer from 0 to 5,
xa5 may be an integer from 1 to 10,
R₂₀₁ to R₂₀₄ and Q₂₀₁ may each independently be a C₃-C₆₀ (*e.g.* C₃-C₃₀) carbocyclic group unsubstituted or substituted with at least one R₁₀ₐ or a C₁-C₆₀ (*e.g.* C₁-C₂₀) heterocyclic group unsubstituted or substituted with at least one R₁₀ₐ,
R₂₀₁ and R₂₀₂ may optionally be bonded to each other via a single bond, a C₁-C₅ alkylene group unsubstituted or substituted with at least one R₁₀ₐ, or a C₂-C₅ alkenylene group unsubstituted or substituted with at least one R₁₀ₐ, to form a C₈-C₆₀ (*e.g.* C₃-C₃₀) polycyclic group (for example, a carbazole group, etc.) unsubstituted or substituted with at least one R₁₀ₐ (for example, Compound HT16, etc.),
R₂₀₃ and R₂₀₄ may optionally be linked to each other, via a single bond, a C₁-C₅ alkylene group unsubstituted or substituted with at least one R₁₀ₐ, or a C₂-C₅ alkenylene group unsubstituted or substituted with at least one R₁₀ₐ, to form a C₈-C₆₀ (*e.g.* C₃-C₃₀) polycyclic group unsubstituted or substituted with at least one R₁₀ₐ, and
na1 may be an integer from 1 to 4.

For example, each of Formulae 201 and 202 may include at least one selected from groups represented by Formulae CY201 to CY217:

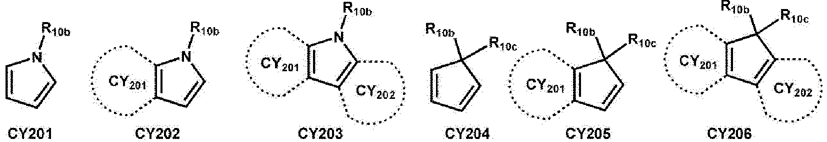

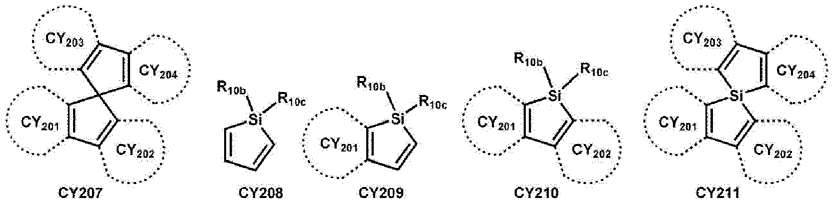

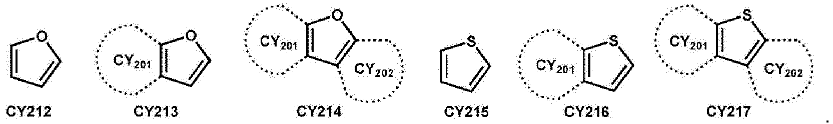

R_{10b} and R_{10c} in Formulae CY201 to CY217 may each be as defined with respect to R₁₀ₐ, ring CY₂₀₁ to ring CY₂₀₄ may each independently be a C₃-C₂₀ carbocyclic group or a C₁-C₂₀ heterocyclic group, and at least one hydrogen in Formulae CY201 to CY217 may be unsubstituted or substituted with R₁₀ₐ.

In an embodiment, in Formulae CY201 to CY217, ring CY₂₀₁ to ring CY₂₀₄ may each independently be a benzene group, a naphthalene group, a phenanthrene group, or an anthracene group.

In one or more embodiments, each of Formulae 201 and 202 may include at least one selected from the groups represented by Formulae CY201 to CY203.

In one or more embodiments, Formula 201 may include at least one selected from the groups represented by Formulae CY201 to CY203 and at least one selected from the groups represented by Formulae CY204 to CY217.

In one or more embodiments, in Formula 201, xa1 may be 1, R₂₀₁ may be one selected from the groups represented by Formulae CY201 to CY203, xa2 may be 0, and R₂₀₂ may be one selected from the groups represented by Formulae CY204 to CY207.

In one or more embodiments, each of Formulae 201 and 202 may not include the groups represented by Formulae CY201 to CY203.

In one or more embodiments, each of Formulae 201 and 202 may not include the groups represented by Formulae CY201 to CY203, and may include at least one selected from the groups represented by Formulae CY204 to CY217.

In one or more embodiments, each of Formulae 201 and 202 may not include the groups represented by Formulae CY201 to CY217.

For example, the hole transport material may include one selected from Compounds HT1 to HT46, m-MTDATA, TDATA, 2-TNATA, NPB(NPD), β-NPB, TPD, Spiro-TPD, Spiro-NPB, methylated NPB, TAPC, HMTPD, 4,4',4"-tris(N-carbazolyl)triphenylamine (TCTA), polyaniline/dodecylbenzenesulfonic acid (PANI/DBSA), poly(3,4-ethylenedioxythiophene)/poly(4-styrenesulfonate) (PEDOT/PSS), polyaniline/camphor sulfonic acid (PANI/CSA), polyaniline/poly(4-styrenesulfonate) (PANI/PSS), or any combination thereof:

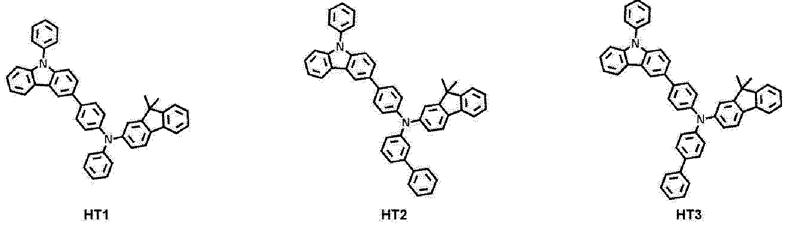

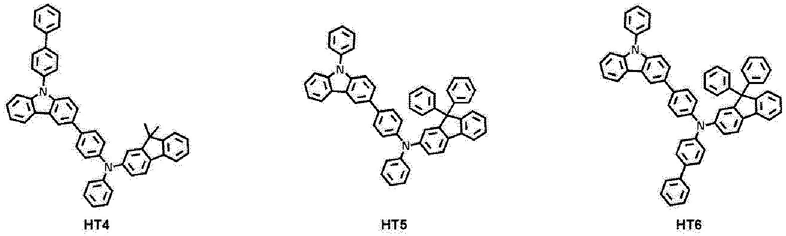

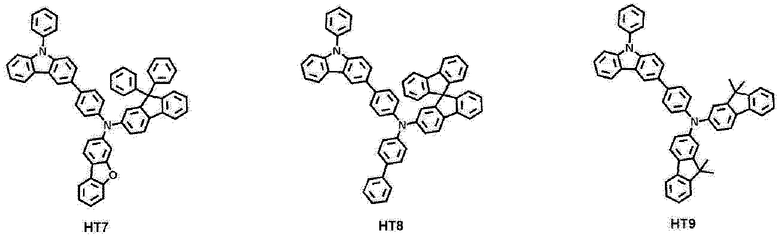

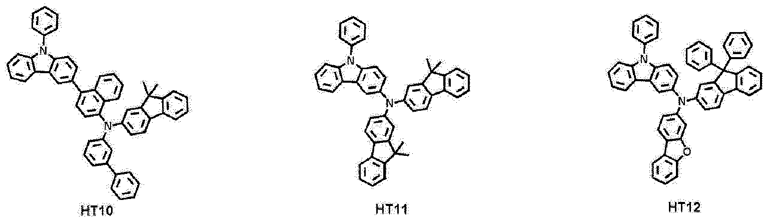

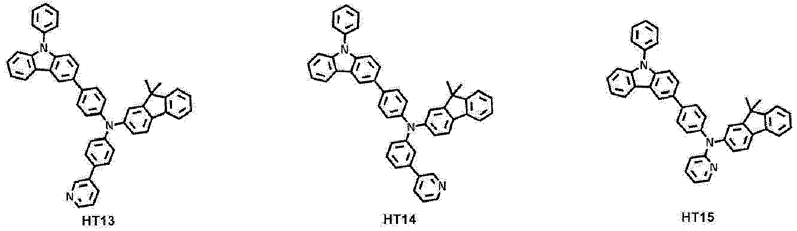

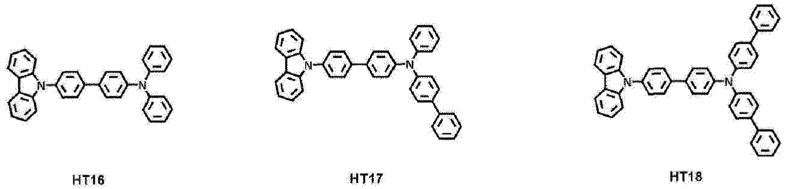

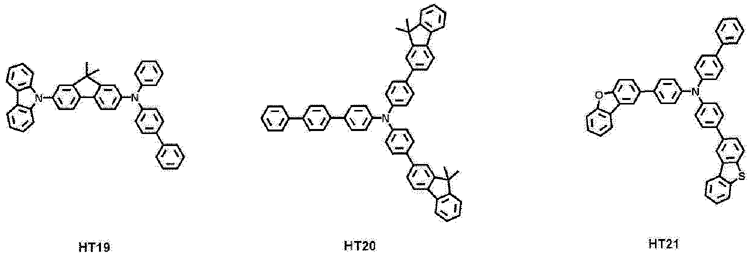

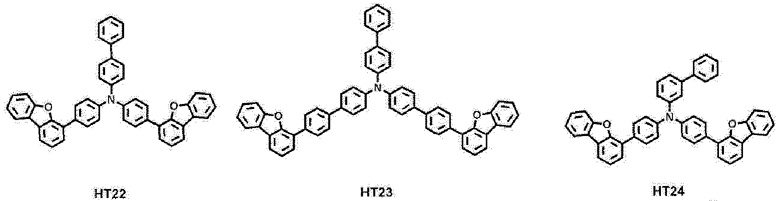

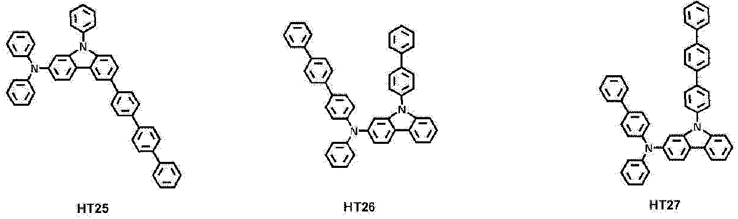

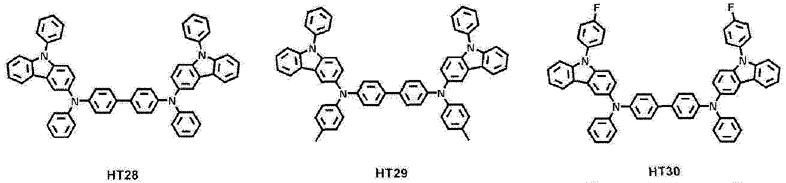

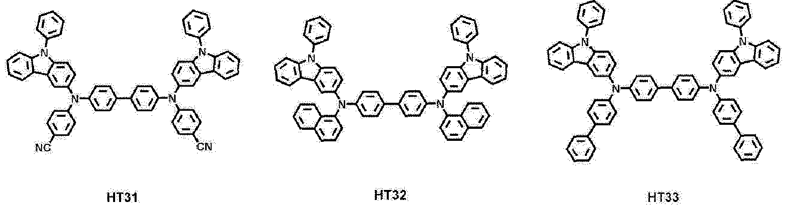

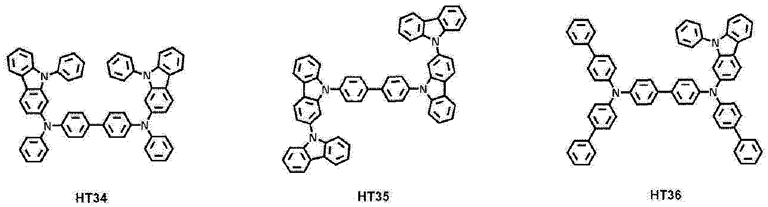

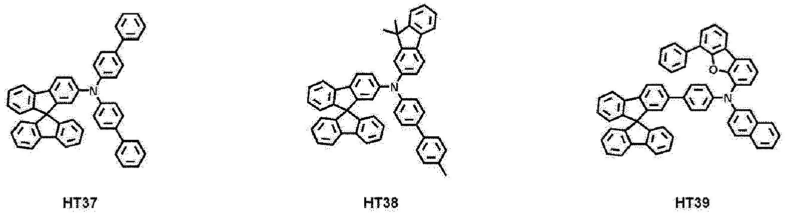

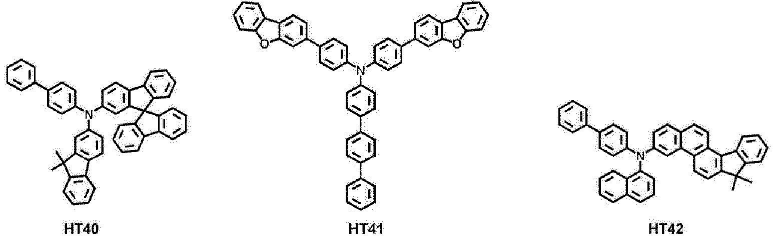

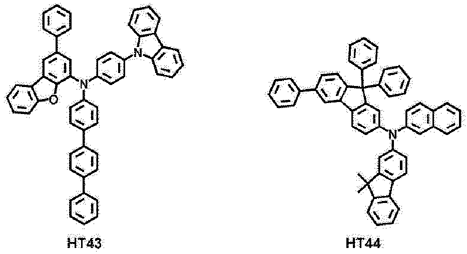

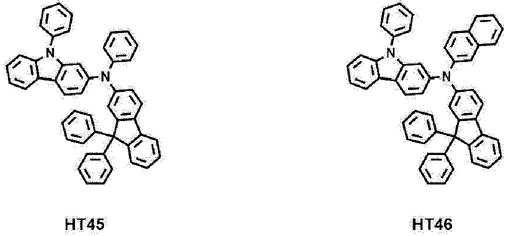

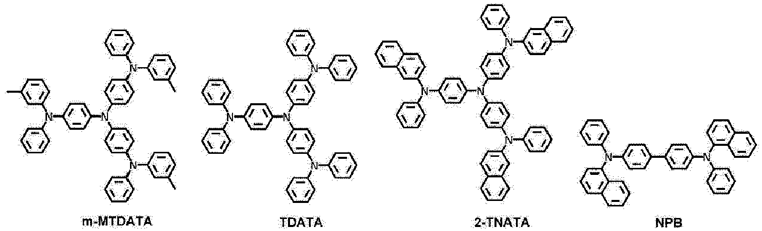

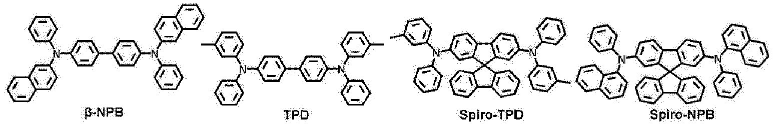

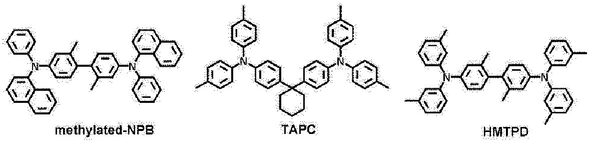

A thickness of the hole transport region 120 may be in a range of about 50 Å to about 10,000 Å, for example, about 100 Å to about 4,000 Å. When the hole transport region 120 includes a hole injection layer, a hole transport layer, or any combination thereof, a thickness of the hole injection layer may be in a range of about 100 Å to about 9,000 Å, for example, about 100 Å to about 1,000 Å, and a thickness of the hole transport layer may be in a range of about 50 Å to about 2,000 Å, for example, about 100 Å to about 1,500 Å. When the thicknesses of the hole transport region 130, the hole injection layer, and the hole transport layer are within these ranges, suitable or satisfactory hole transporting characteristics may be obtained without a substantial increase in driving voltage.

The electron blocking layer may be a layer that prevents or reduces electron leakage from the photoactive layer 130 to the hole transport region 120. The aforementioned hole transport material may be included in the electron blocking layer.

### p-dopant

The hole transport region 120 may further include, in addition to these materials, a charge-generation material for the improvement of conductive properties (e.g., electrically conductive properties). The charge-generation material may be uniformly or non-uniformly dispersed in the hole transport region (for example, in the form of a single layer consisting of a charge-generation material).

The charge-generation material may be, for example, a p-dopant.

For example, the p-dopant may have a lowest unoccupied molecular orbital (LUMO) energy level of less than or equal to about -3.5 eV.

In an embodiment, the p-dopant may include a quinone derivative, a cyano group-containing compound, a compound including element EL1 and element EL2, or any combination thereof.

Examples of the quinone derivative are TCNQ, F4-TCNQ, and the like.

Examples of the cyano group-containing compound are HAT-CN, a compound represented by Formula 221, and the like: 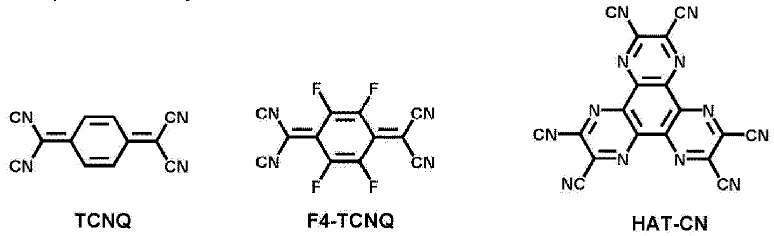 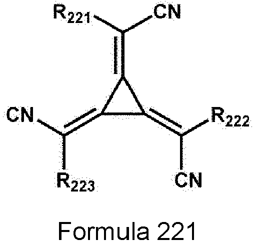 wherein, in Formula 221,
R₂₂₁ to R₂₂₃ may each independently be a C₃-C₆₀ (*e.g.* C₃-C₃₀) carbocyclic group that is unsubstituted or substituted with at least one R₁₀ₐ or a C₁-C₆₀ (*e.g.* C₁-C₂₀) heterocyclic group that is unsubstituted or substituted with at least one R₁₀ₐ, and
at least one selected from R₂₂₁ to R₂₂₃ may each independently be a C₃-C₆₀ (*e.g.* C₃-C₃₀) carbocyclic group or a C₁-C₆₀ (*e.g.* C₁-C₂₀) heterocyclic group, each substituted with: a cyano group; -F; -Cl; -Br; -I; a C₁-C₂₀ alkyl group substituted with a cyano group, -F, -Cl, -Br, -I, or any combination thereof; or any combination thereof.

In the compound including element EL1 and element EL2, element EL1 may be metal, metalloid, or any combination thereof, and element EL2 may be non-metal, metalloid, or any combination thereof.

Examples of the metal are an alkali metal (for example, lithium (Li), sodium (Na), potassium (K), rubidium (Rb), cesium (Cs), etc.); alkaline earth metal (for example, beryllium (Be), magnesium (Mg), calcium (Ca), strontium (Sr), barium (Ba), etc.); transition metal (for example, titanium (Ti), zirconium (Zr), hafnium (Hf), vanadium (V), niobium (Nb), tantalum (Ta), chromium (Cr), molybdenum (Mo), tungsten (W), manganese (Mn), technetium (Tc), rhenium (Re), iron (Fe), ruthenium (Ru), osmium (Os), cobalt (Co), rhodium (Rh), iridium (Ir), nickel (Ni), palladium (Pd), platinum (Pt), copper (Cu), silver (Ag), gold (Au), etc.); post-transition metal (for example, zinc (Zn), indium (In), tin (Sn), etc.); lanthanide metal (for example, lanthanum (La), cerium (Ce), praseodymium (Pr), neodymium (Nd), promethium (Pm), samarium (Sm), europium (Eu), gadolinium (Gd), terbium (Tb), dysprosium (Dy), holmium (Ho), erbium (Er), thulium (Tm), ytterbium (Yb), lutetium (Lu), etc.); and the like.

Examples of the metalloid are silicon (Si), antimony (Sb), tellurium (Te), and the like.

Examples of the non-metal are oxygen (O), halogen (for example, F, Cl, Br, I, etc.), and the like.

Examples of the compound including element EL1 and element EL2 are metal oxide, metal halide (for example, metal fluoride, metal chloride, metal bromide, metal iodide, etc.), metalloid halide (for example, metalloid fluoride, metalloid chloride, metalloid bromide, metalloid iodide, etc.), metal telluride, or any combination thereof.

Examples of the metal oxide are tungsten oxide (for example, WO, W₂O₃, WO₂, WO₃, W₂O₅, etc.), vanadium oxide (for example, VO, V₂O₃, VO₂, V₂O₅, etc.), molybdenum oxide (MoO, Mo₂O₃, MoO₂, MoO₃, Mo₂O₅, etc.), rhenium oxide (for example, ReO₃, etc.), and the like.

Examples of the metal halide are alkali metal halide, alkaline earth metal halide, transition metal halide, post-transition metal halide, lanthanide metal halide, and the like.

Examples of the alkali metal halide are LiF, NaF, KF, RbF, CsF, LiCl, NaCl, KCI, RbCl, CsCl, LiBr, NaBr, KBr, RbBr, CsBr, Lil, Nal, KI, Rbl, CsI, and the like.

Examples of the alkaline earth metal halide are BeF₂, MgF₂, CaF₂, SrF₂, BaF₂, BeCl₂, MgCl₂, CaCl₂, SrCl₂, BaCl₂, BeBr₂, MgBr₂, CaBr₂, SrBr₂, BaBr₂, BeI₂, MgI₂, CaI₂, SrI₂, BaI₂, and the like.

Examples of the transition metal halide are titanium halide (for example, TiF₄, TiCl₄, TiBr₄, TiI₄, etc.), zirconium halide (for example, ZrF₄, ZrCl₄, ZrBr₄, ZrI₄, etc.), hafnium halide (for example, HfF₄, HfCl₄, HfBr₄, HfI₄, etc.), vanadium halide (for example, VF₃, VCl₃, VBr₃, VI₃, etc.), niobium halide (for example, NbF₃, NbCl₃, NbBr₃, NbI₃, etc.), tantalum halide (for example, TaF₃, TaCl₃, TaBr₃, TaI₃, etc.), chromium halide (for example, CrF₃, CrCl₃, CrBr₃, CrI₃, etc.), molybdenum halide (for example, MoF₃, MoCl₃, MoBr₃, MoI₃, etc.), tungsten halide (for example, WF₃, WCl₃, WBr₃, WI₃, etc.), manganese halide (for example, MnF₂, MnCl₂, MnBr₂, MnI₂, etc.), technetium halide (for example, TcF₂, TcCl₂, TcBr₂, TcI₂, etc.), rhenium halide (for example, ReF₂, ReCl₂, ReBr₂, ReI₂, etc.), iron halide (for example, FeF₂, FeCl₂, FeBr₂, FeI₂, etc.), ruthenium halide (for example, RuF₂, RuCl₂, RuBr₂, RuI₂, etc.), osmium halide (for example, OsF₂, OsCl₂, OsBr₂, OsI₂, etc.), cobalt halide (for example, CoF₂, CoCl₂, CoBr₂, CoI₂, etc.), rhodium halide (for example, RhF₂, RhCl₂, RhBr₂, RhI₂, etc.), iridium halide (for example, IrF₂, IrCl₂, IrBr₂, IrI₂, etc.), nickel halide (for example, NiF₂, NiCl₂, NiBr₂, NiI₂, etc.), palladium halide (for example, PdF₂, PdCl₂, PdBr₂, PdI₂, etc.), platinum halide (for example, PtF₂, PtCl₂, PtBr₂, PtI₂, etc.), copper halide (for example, CuF, CuCl, CuBr, Cul, etc.), silver halide (for example, AgF, AgCl, AgBr, AgI, etc.), gold halide (for example, AuF, AuCl, AuBr, Aul, etc.), and the like.

Examples of the post-transition metal halide are zinc halide (for example, ZnF₂, ZnCl₂, ZnBr₂, ZnI₂, etc.), indium halide (for example, InI₃, etc.), tin halide (for example, SnI₂, etc.), and the like.

Examples of the lanthanide metal halide are YbF, YbF₂, YbF₃, SmF₃, YbCl, YbCl₂, YbCl₃ SmCl₃, YbBr, YbBr₂, YbBr₃ SmBr₃, YbI, YbI₂, YbI₃, SmI₃, and the like.

Examples of the metalloid halide are antimony halide (for example, SbCl₅, etc.) and the like.

Examples of the metal telluride are alkali metal telluride (for example, Li₂Te, a Na₂Te, K₂Te, Rb₂Te, Cs₂Te, etc.), alkaline earth metal telluride (for example, BeTe, MgTe, CaTe, SrTe, BaTe, etc.), transition metal telluride (for example, TiTe₂, ZrTe₂, HfTe₂, V₂Te₃, Nb₂Te₃, Ta₂Te₃, Cr₂Te₃, Mo₂Te₃, W₂Te₃, MnTe, TcTe, ReTe, FeTe, RuTe, OsTe, CoTe, RhTe, IrTe, NiTe, PdTe, PtTe, Cu₂Te, CuTe, Ag₂Te, AgTe, Au₂Te, etc.), post-transition metal telluride (for example, ZnTe, etc.), lanthanide metal telluride (for example, LaTe, CeTe, PrTe, NdTe, PmTe, EuTe, GdTe, TbTe, DyTe, HoTe, ErTe, TmTe, YbTe, LuTe, etc.), and the like.

### Electron transport region

The charge auxiliary layer(s) arranged between the photoactive layer 130 and the second electrode 150 may be collectively referred to as the electron transport region 140. The electron transport region 140 may facilitate the transport of electrons generated in the photoactive layer 130.

The electron transport region 140 may have: i) a single-layer structure consisting of a single layer consisting of a single material, ii) a single-layer structure consisting of a single layer consisting of a plurality of different materials, or iii) a multi-layer structure including a plurality of layers including different materials.

The electron transport region 140 may include a buffer layer, a hole blocking layer, an electron control layer, an electron transport layer, an electron injection layer, or any combination thereof.

For example, the electron transport region 140 may have an electron transport layer/electron injection layer structure, a hole blocking layer/electron transport layer/electron injection layer structure, an electron control layer/electron transport layer/electron injection layer structure, or a buffer layer/electron transport layer/electron injection layer structure, wherein constituent layers of each structure are sequentially stacked from the emission layer.

In an embodiment, the electron transport region 140 (for example, the buffer layer, the hole blocking layer, the electron control layer, or the electron transport layer in the electron transport region) may include a metal-free compound including at least one π electron-deficient nitrogen-containing C₁-C₆₀ (e.g. C₁-C₃₀) cyclic group.

For example, the electron transport region 140 may include a compound represented by Formula 601:

Formula 601 [Ar₆₀₁]ₓₑ₁₁-[(L₆₀₁)ₓₑ₁-R₆₀₁]ₓₑ₂₁

wherein, in Formula 601,
Ar₆₀₁ and L₆₀₁ may each independently be a C₃-C₆₀ (*e.g.* C₃-C₃₀) carbocyclic group unsubstituted or substituted with at least one R₁₀ₐ or a C₁-C₆₀ (*e.g.* C₁-C₂₀) heterocyclic group unsubstituted or substituted with at least one R₁₀ₐ,
xe11 may be 1, 2, or 3,
xe1 may be 0, 1, 2, 3, 4, or 5,
R₆₀₁ may be a C₃-C₆₀ (*e.g.* C₃-C₃₀) carbocyclic group unsubstituted or substituted with at least one R₁₀ₐ, a C₁-C₆₀ (*e.g.* C₁-C₂₀) heterocyclic group unsubstituted or substituted with at least one R₁₀ₐ, -Si(Q₆₀₁)(Q₆₀₂)(Q₆₀₃), -C(=O)(Q₆₀₁), - S(=O)₂(Q₅₀₁), or -P(=O)(Q₆₀₁)(Q₆₀₂),
Q₆₀₁ to Q₆₀₃ may each be as defined with respect to Q₁₁,
xe21 may be 1, 2, 3, 4, or 5, and
at least one selected from Ar₆₀₁, L₆₀₁, and R₆₀₁ may each independently be a π electron-deficient nitrogen-containing C₁-C₆₀ (*e.g.* C₁-C₃₀) cyclic group unsubstituted or substituted with at least one R₁₀ₐ.

In an embodiment, when xe11 in Formula 601 is 2 or more, two or more of Ar₆₀₁ may be linked to each other via a single bond.

In an embodiment, Ar₆₀₁ in Formula 601 may be a substituted or unsubstituted anthracene group.

In one or more embodiments, the electron transport region may include a compound represented by Formula 601-1: 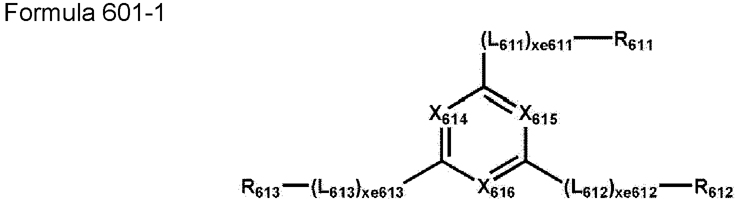 wherein, in Formula 601-1,
X₆₁₄ may be N or C(R₆₁₄), X₆₁₅ may be N or C(R₆₁₅), X₆₁₆ may be N or C(R₆₁₆), and at least one selected from X₆₁₄ to X₆₁₆ may be N,
L₆₁₁ to L₆₁₃ may each be as defined with respect to L₆₀₁,
xe611 to xe613 may each be as defined with respect to in xe1,
R₆₁₁ to R₆₁₃ may each be as defined with respect to R₆₀₁, and
R₆₁₄ to R₆₁₆ may each independently be hydrogen, deuterium, -F, -Cl, -Br, -I, a hydroxyl group, a cyano group, a nitro group, a C₁-C₂₀ (*e.g.* C₁-C₁₀) alkyl group, a C₁-C₂₀ (*e.g.* C₁-C₁₀) alkoxy group, a C₃-C₆₀ (*e.g.* C₃-C₃₀) carbocyclic group unsubstituted or substituted with at least one R₁₀ₐ, or a C₁-C₆₀ (*e.g.* C₁-C₂₀) heterocyclic group unsubstituted or substituted with at least one R₁₀ₐ.

For example, xe1 and xe611 to xe613 in Formulae 601 and 601-1 may each independently be 0, 1, or 2.

In one or more embodiments, the electron transport region may include: one selected from Compounds ET1 to ET45; 2,9-dimethyl-4,7-diphenyl-1,10-phenanthroline (BCP); 4,7-diphenyl-1,10-phenanthroline (Bphen); Alq₃; BAlq; TAZ; NTAZ; or any combination thereof:

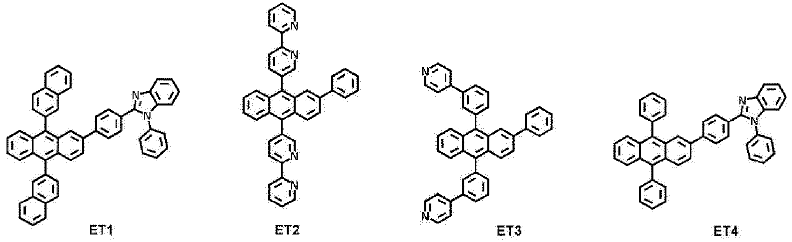

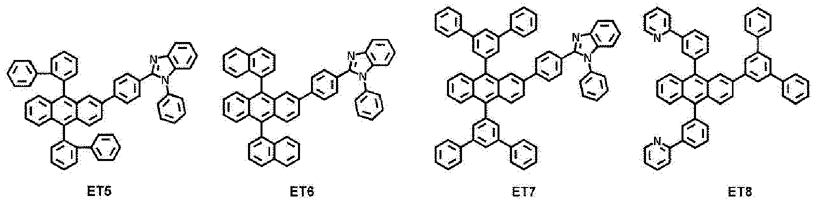

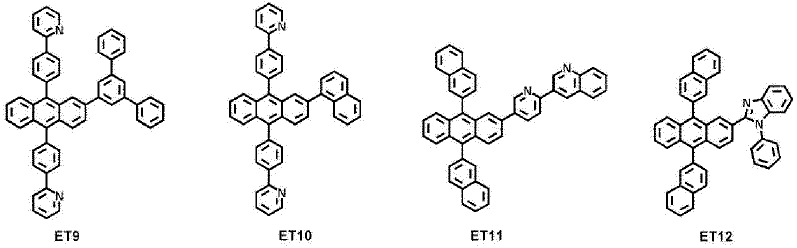

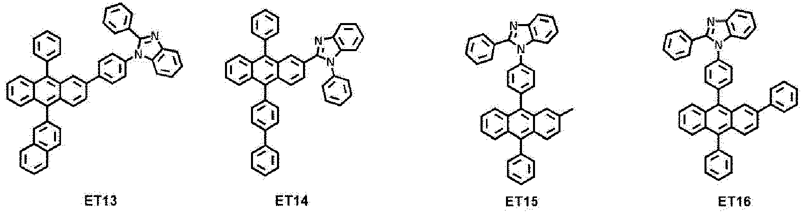

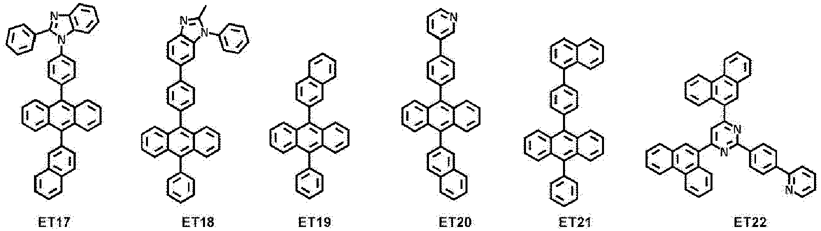

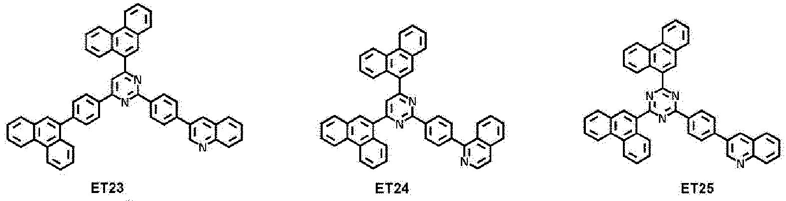

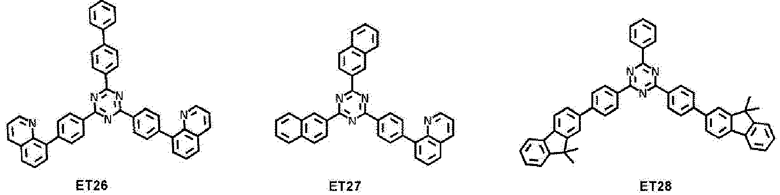

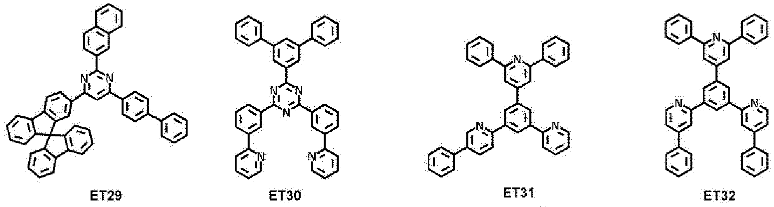

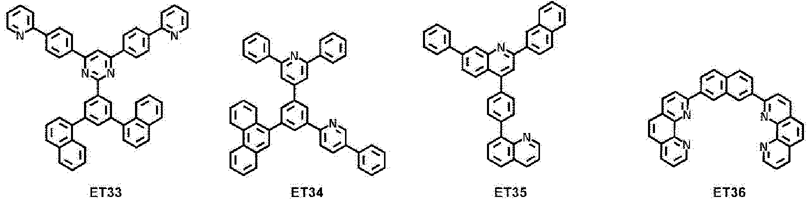

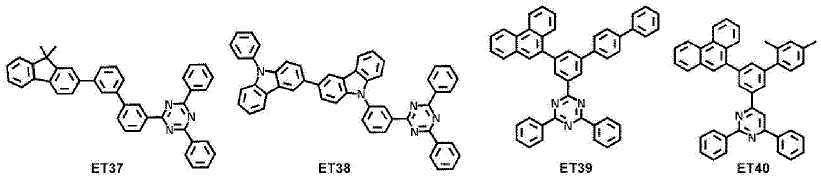

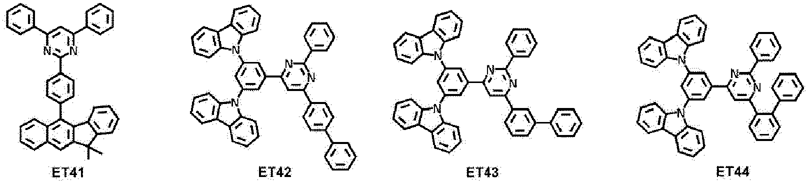

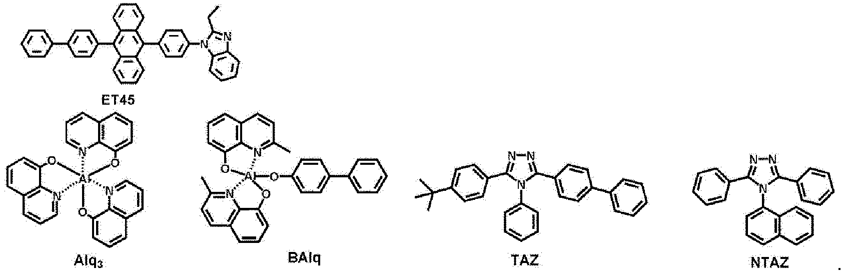

A thickness of the electron transport region 140 may be in a range of about 100 Å to about 5,000 Å, for example, about 160 Å to about 4,000 Å. When the electron transport region 140 includes a buffer layer, a hole blocking layer, an electron control layer, an electron transport layer, or any combination thereof, a thickness of the buffer layer, the hole blocking layer, or the electron control layer may be in a range of about 20 Å to about 1,000 Å, for example, about 30 Å to about 300 Å, and a thickness of the electron transport layer may be in a range of about 100 Å to about 1,000 Å, for example, about 150 Å to about 500 Å. When the thicknesses of the buffer layer, the hole blocking layer, the electron control layer, the electron transport layer, and/or the electron transport region 140 are within these ranges, suitable or satisfactory electron transporting characteristics may be obtained without a substantial increase in driving voltage.

The electron transport region 140 (for example, the electron transport layer in the electron transport region) may further include, in addition to the materials described above, a metal-containing material.

The metal-containing material may include an alkali metal complex, an alkaline earth metal complex, or any combination thereof. The metal ion of an alkali metal complex may be a Li ion, a Na ion, a K ion, a Rb ion, or a Cs ion, and the metal ion of an alkaline earth metal complex may be a Be ion, a Mg ion, a Ca ion, a Sr ion, or a Ba ion. A ligand coordinated with the metal ion of the alkali metal complex or the alkaline earth-metal complex may include a hydroxyquinoline, a hydroxyisoquinoline, a hydroxybenzoquinoline, a hydroxyacridine, a hydroxyphenanthridine, a hydroxyphenyloxazole, a hydroxyphenylthiazole, a hydroxyphenyloxadiazole, a hydroxyphenylthiadiazole, a hydroxyphenylpyridine, a hydroxyphenylbenzimidazole, a hydroxyphenylbenzothiazole, a bipyridine, a phenanthroline, a cyclopentadiene, or any combination thereof.

For example, the metal-containing material may include a Li complex. The Li complex may include, for example, Compound ET-D1 (LiQ) and/or ET-D2:

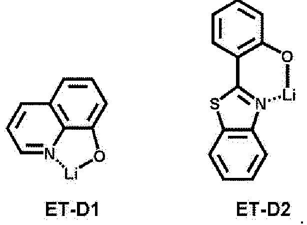

The electron transport region 140 may include an electron injection layer that facilitates the injection of electrons from the second electrode 150. The electron injection layer may directly contact the second electrode 150.

The electron injection layer may have: i) a single-layer structure consisting of a single layer consisting of a single material, ii) a single-layer structure consisting of a single layer consisting of a plurality of different materials, or iii) a multi-layer structure including a plurality of layers including different materials.

The electron injection layer may include an alkali metal, alkaline earth metal, a rare earth metal, an alkali metal-containing compound, alkaline earth metal-containing compound, a rare earth metal-containing compound, an alkali metal complex, an alkaline earth metal complex, a rare earth metal complex, or any combination thereof.

The alkali metal may include Li, Na, K, Rb, Cs, or any combination thereof. The alkaline earth metal may include Mg, Ca, Sr, Ba, or any combination thereof. The rare earth metal may include Sc, Y, Ce, Tb, Yb, Gd, or any combination thereof.

The alkali metal-containing compound, the alkaline earth metal-containing compound, and the rare earth metal-containing compound may be oxides, halides (for example, fluorides, chlorides, bromides, iodides, etc.), or tellurides of the alkali metal, the alkaline earth metal, and the rare earth metal, or any combination thereof.

The alkali metal-containing compound may include: an alkali metal oxide, such as Li₂O, Cs₂O, K₂O, and/or the like; alkali metal halides, such as LiF, NaF, CsF, KF, Lil, Nal, CsI, KI, and/or the like; or any combination thereof. The alkaline earth metal-containing compound may include an alkaline earth metal compound, such as BaO, SrO, CaO, BaₓSr₁₋ₓO (wherein x is a real number satisfying 0<x<1), BaₓCa₁₋ₓO (wherein x is a real number satisfying 0<x<1), and/or the like. The rare earth metal-containing compound may include YbF₃, ScF₃, Sc₂O₃, Y₂O₃, Ce₂O₃, GdF₃, TbF₃, YbI₃, ScI₃, TbI₃, or any combination thereof. In an embodiment, the rare earth metal-containing compound may include lanthanide metal telluride. Examples of the lanthanide metal telluride are LaTe, CeTe, PrTe, NdTe, PmTe, SmTe, EuTe, GdTe, TbTe, DyTe, HoTe, ErTe, TmTe, YbTe, LuTe, La₂Te₃, Ce₂Te₃, Pr₂Te₃, Nd₂Te₃, Pm₂Te₃, Sm₂Te₃, Eu₂Te₃, Gd₂Te₃, Tb₂Te₃, Dy₂Te₃, Ho₂Te₃, Er₂Te₃, Tm₂Te₃, Yb₂Te₃, Lu₂Te₃, and the like.

The alkali metal complex, the alkaline earth-metal complex, and the rare earth metal complex may include i) one selected from ions of the alkali metal, the alkaline earth metal, and the rare earth metal and ii), as a ligand bonded to the metal ion, for example, hydroxyquinoline, hydroxyisoquinoline, hydroxybenzoquinoline, hydroxyacridine, hydroxyphenanthridine, hydroxyphenyloxazole, hydroxyphenylthiazole, hydroxyphenyloxadiazole, hydroxyphenylthiadiazole, hydroxyphenylpyridine, hydroxyphenyl benzimidazole, hydroxyphenylbenzothiazole, bipyridine, phenanthroline, cyclopentadiene, or any combination thereof.

In an embodiment, the electron injection layer may include (or consist of) an alkali metal, an alkaline earth metal, a rare earth metal, an alkali metal-containing compound, an alkaline earth metal-containing compound, a rare earth metal-containing compound, an alkali metal complex, an alkaline earth metal complex, a rare earth metal complex, or any combination thereof, as described above. In one or more embodiments, the electron injection layer may further include an organic material (for example, a compound represented by Formula 601).

In an embodiment, the electron injection layer may include (or consist of) i) an alkali metal-containing compound (for example, alkali metal halide), ii) a) an alkali metal-containing compound (for example, alkali metal halide); and b) an alkali metal, an alkaline earth metal, a rare earth metal, or any combination thereof. For example, the electron injection layer may be a KI:Yb co-deposited layer, an RbI:Yb co-deposited layer, a LiF:Yb co-deposited layer, and/or the like.

When the electron injection layer further includes an organic material, an alkali metal, an alkaline earth metal, a rare earth metal, an alkali metal-containing compound, an alkaline earth metal-containing compound, a rare earth metal-containing compound, an alkali metal complex, an alkaline earth-metal complex, a rare earth metal complex, or any combination thereof may be uniformly or non-uniformly dispersed in a matrix including the organic material.

A thickness of the electron injection layer may be in a range of about 1 Å to about 100 Å, and, for example, about 3 Å to about 90 Å. When the thickness of the electron injection layer is within the ranges above, suitable or satisfactory electron injection characteristics may be obtained without a substantial increase in driving voltage.

### Second electrode 150

The second electrode 150 is arranged on the photoactive layer 130. The second electrode 150 may be a cathode, which is an electron injection electrode, and as a material for forming the second electrode 150, a metal, an alloy, an electrically conductive compound, or any combination thereof, each having a low-work function, may be used.

The second electrode 150 may include lithium (Li), silver (Ag), magnesium (Mg), aluminium (Al), aluminium-lithium (Al-Li), calcium (Ca), magnesium-indium (Mg-In), magnesium-silver (Mg-Ag), ytterbium (Yb), silver-ytterbium (Ag-Yb), ITO, IZO, or any combination thereof. The second electrode 150 may be a transmissive electrode, a semi-transmissive electrode, or a reflective electrode.

The second electrode 150 may have a single-layer structure or a multi-layer structure including a plurality of layers.

### Capping layer

A first capping layer may be arranged outside the first electrode 110, and/or a second capping layer may be arranged outside the second electrode 150.

The first capping layer and/or the second capping layer may prevent or reduce penetration of impurities, such as water, oxygen, and/or the like, into the organic photodiode 10 or 20, thereby increasing the reliability of the organic photodiode 10 or 20.

The first capping layer and the second capping layer may each include a material having a refractive index of 1.6 or more (at a wavelength of 589 nm).

The first capping layer and the second capping layer may each independently be an organic capping layer including an organic material, an inorganic capping layer including an inorganic material, or an organic-inorganic composite capping layer including an organic material and an inorganic material.

At least one selected from the first capping layer and the second capping layer may each independently include a carbocyclic compound, a heterocyclic compound, an amine group-containing compound, a porphine derivative, a phthalocyanine derivative, a naphthalocyanine derivative, an alkali metal complex, an alkaline earth metal complex, or any combination thereof. The carbocyclic compound, the heterocyclic compound, and the amine group-containing compound may optionally be substituted with a substituent including O, N, S, Se, Si, F, Cl, Br, I, or any combination thereof. In an embodiment, at least one selected from the first capping layer and the second capping layer may each independently include an amine group-containing compound.

In one or more embodiments, at least one selected from the first capping layer and the second capping layer may each independently include a compound represented by Formula 201, a compound represented by Formula 202, or any combination thereof.

In one or more embodiments, at least one selected from the first capping layer and the second capping layer may each independently include: one selected from Compounds HT28 to HT33; one selected from Compounds CP1 to CP6; β-NPB; or any combination thereof:

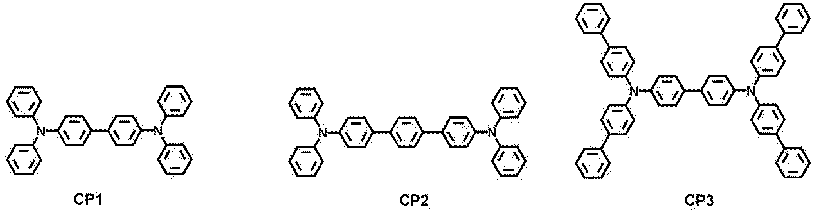

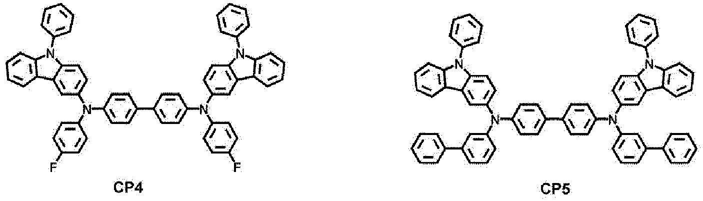

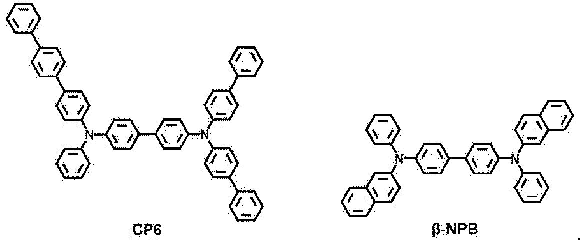

### Electronic apparatus

An electronic apparatus including the organic photodiode and the organic light-emitting device are provided.

The electronic apparatus according to an embodiment may include:
a substrate; and
an organic photodiode and an organic light-emitting device that are arranged on the substrate.

The organic photodiode may sequentially include a first electrode, a first auxiliary layer, a photoactive layer, and a common electrode.

The organic light-emitting device may sequentially include a second electrode, a second auxiliary layer, a first emission layer, and a common electrode.

A thickness of the photoactive layer may be smaller than that of the first emission layer, and
a thickness of the first auxiliary layer is identical to that of the second auxiliary layer.

The first emission layer may have a thickness in a range of 350 Å to 700 Å.

In an embodiment, the organic light-emitting device may emit red light, green light, blue light, or white light. In one or more embodiments, the organic light-emitting device may emit light of different colors other than the aforementioned colors.

A resonance distance of the organic photodiode may be identical to that of the organic light-emitting device.

The organic photodiode may detect light emitted from the organic light-emitting device and reflected from a subject.

### Photoactive layer

The photoactive layer may include a donor compound (e.g., a p-type semiconductor) and an acceptor compound (e.g., an n-type semiconductor).

The photoactive layer may have a planar heterojunction structure of the donor compound and the acceptor compound.

The photoactive layer may have a bulk heterojunction structure of the donor compound and the acceptor compound.

In an embodiment, the donor compound may be a compound represented by Formula 110: 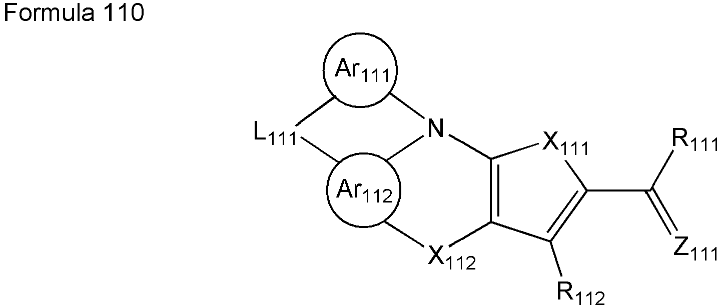 wherein, in Formula 110,
Ar₁₁₁ and Ar₁₁₂ are each independently a C₆-C₃₀ (*e.g.* C₆-C₂₀) arylene group that is unsubstituted or substituted with at least one R₁₀ₐ or a C₃-C₃₀ (*e.g.* C₃-C₂₀) heteroarylene group that is unsubstituted or substituted with at least one R₁₀ₐ,
X₁₁₁ is selected from -Se-, -Te-, -S(=O)-, -S(=O)₂-, -NR₁₁₃-, -BR₁₁₃-, - CR₁₁₃R₁₁₄-, -SiR₁₁₃R₁₁₄-, and -GeR₁₁₃R₁₁₄-,
X₁₁₂ and L₁₁₁ are each independently selected from -O-, -S-, -Se-, -Te-, - S(=O)-, -S(=O)₂-, -, -NR₁₁₃-, -BR₁₁₃-, -CR₁₁₃R₁₁₄-, -SiR₁₁₃R₁₁₄-, -GeR₁₁₃R₁₁₄-, - (C(R₁₁₅)=C(R₁₁₆))-, and -(C(R₁₁₅)=N))-,
when L₁₁₁ is selected from NR₁₁₃-, -BR₁₁₃-, -CR₁₁₃R₁₁₄-, -SiR₁₁₃R₁₁₄-, - GeR₁₁₃R₁₁₄-, -(C(R₁₁₅)=C(R₁₁₆))-, and -(C(R₁₁₅)=N))-, L₁₁₁ is optionally linked to Ar₁₁₁ or Ar₁₁₂ to form a condensed ring,
Z₁₁₁ is a C₆-C₃₀ (*e.g.* C₆-C₂₀) carbocyclic group that is unsubstituted or substituted with at least one R₁₀ₐ and has at least one functional group selected from C=O, C=S, C=Se, and C=Te, or a C₁-C₃₀ (*e.g.* C₁-C₂₀) heterocyclic group that is unsubstituted or substituted with at least one R₁₀ₐ and has at least one functional group selected from C=O, C=S, C=Se, and C=Te, and
R₁₁₁ to R₁₁₆ are each independently hydrogen, deuterium, a halogen, a cyano group, a nitro group, a hydroxy group, a C₁-C₃₀ (*e.g.* C₁-C₂₀) alkyl group unsubstituted or substituted with at least one R₁₀ₐ, a C₁-C₃₀ (*e.g.* C₁-C₂₀) alkoxy group unsubstituted or substituted with at least one R₁₀ₐ, a C₆-C₃₀ (*e.g.* C₆-C₂₀) aryl group unsubstituted or substituted with at least one R₁₀ₐ, a C₃-C₃₀ (*e.g.* C₃-C₂₀) heteroaryl group unsubstituted or substituted with at least one R₁₀ₐ, a C₂-C₃₀ (*e.g.* C₂-C₂₀) acyl group unsubstituted or substituted with at least one R₁₀ₐ, or any combination thereof.

In an embodiment, Z₁₁₁ may be a ring group represented by one selected from Formulae 111A to 111F: 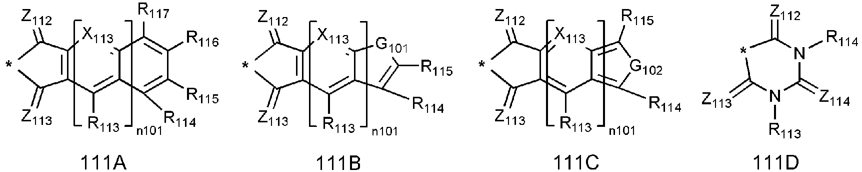 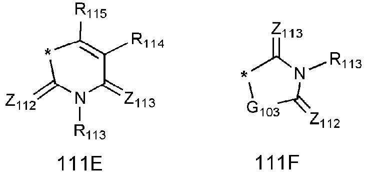 wherein, in Formulae 111A to 111F,
Z₁₁₂ to Z₁₁₄ are each independently O, S, Se, or Te,
X₁₁₃ is N or C(Q₁₁₃),
X₁₁₄ and X₁₁₅ are each independently O, S, Se, Te, Si(Q₁₁₁)(Q₁₁₂), or Ge(Q₁₁₁)(Q₁₁₂),
n101 is an integer from 0 to 3,
R₁₁₃ to R₁₁₇ are each independently as defined with respect to R₁₂₁, and
Q₁₁₁ to Q₁₁₃ are each independently as defined with respect to Q₁₁₁.

### Example compounds

In an embodiment, the compound represented by Formula 110 may be a compound selected from compounds of Groups 1 to 3.

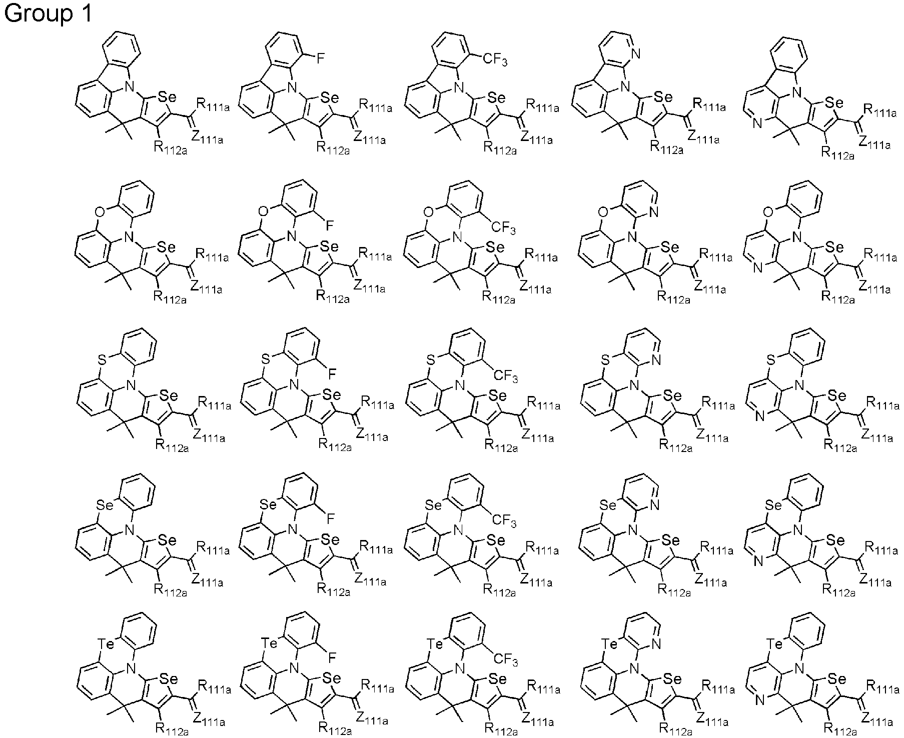

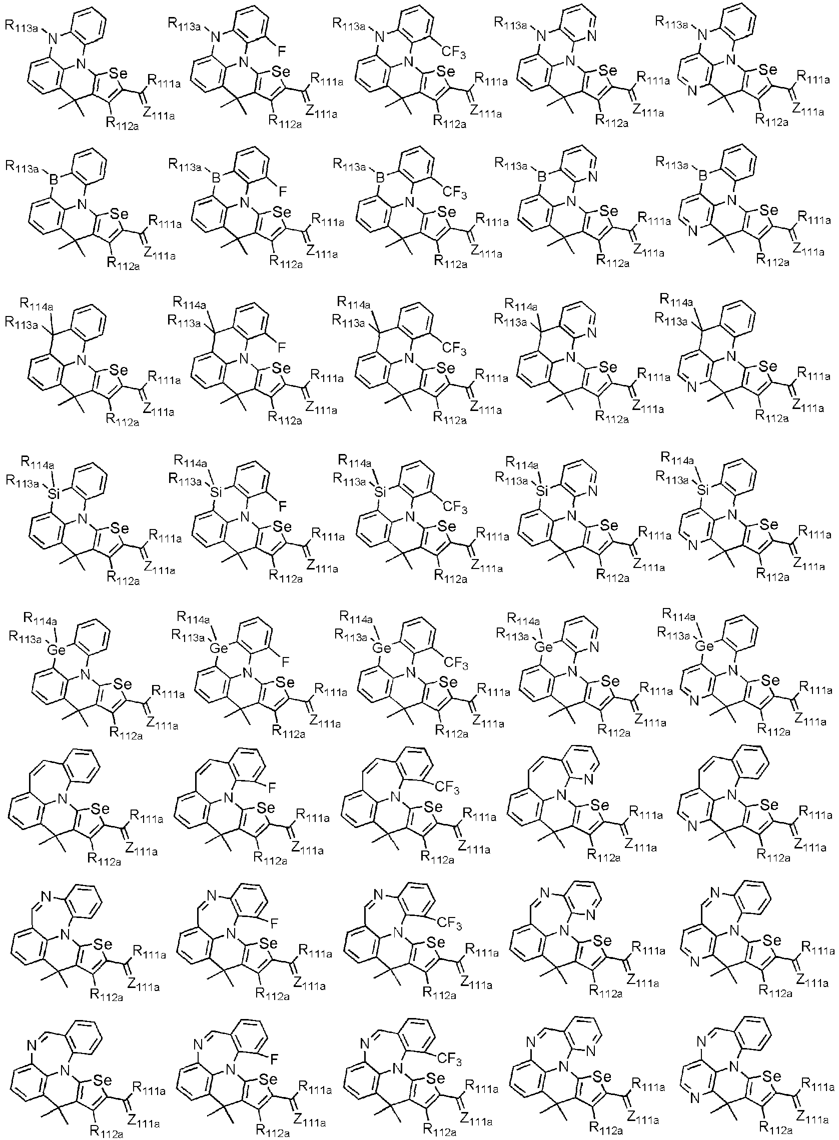

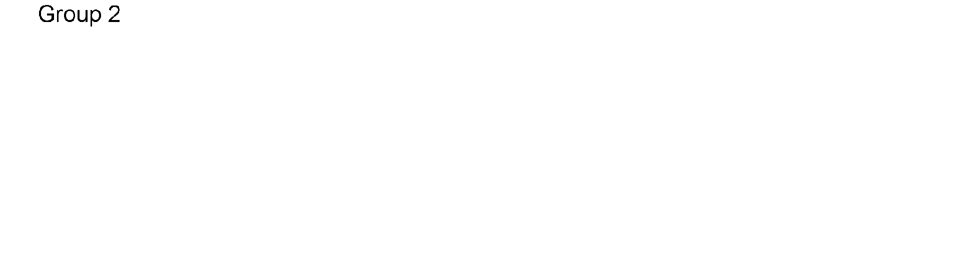

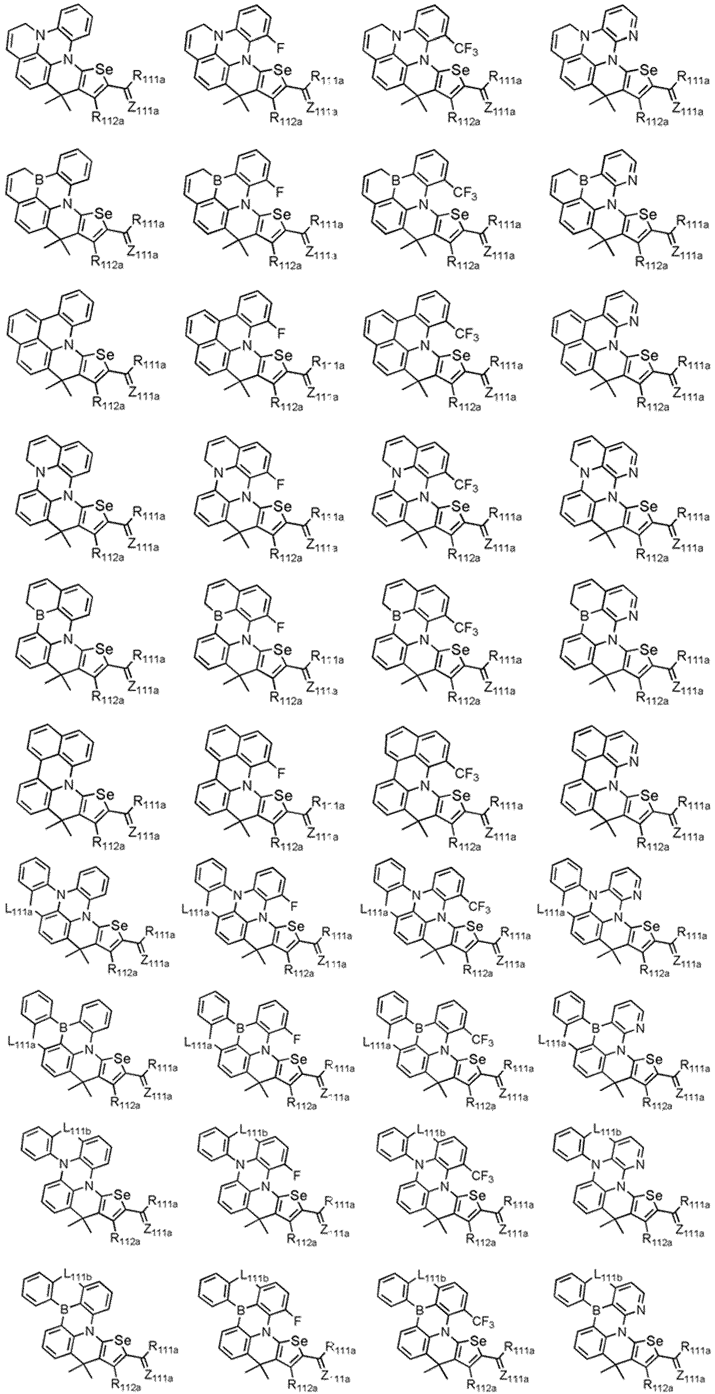

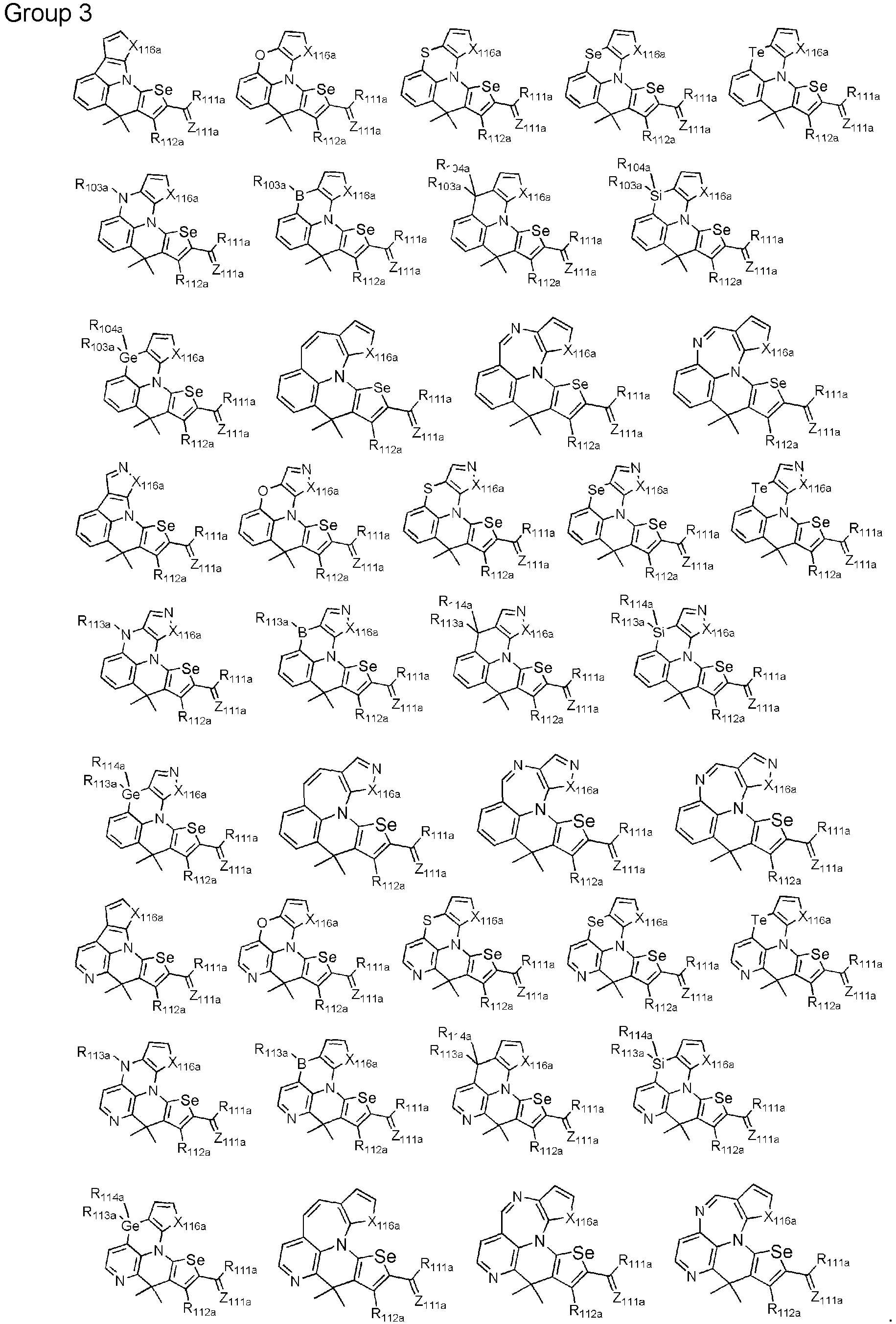

The n-type organic semiconductor may include a compound serving as an electron acceptor. For example, the n-type organic semiconductor may include 3,4,9,10-perylenetetracarboxylic dianhydride (PTCDA), N,N'-di(propoxyethyl)perylene-3,4,9,10-tetracarboxylic diimide (PTCDI), naphthalene tetracarboxylic anhydride (NTCDA), naphthalenetetracarboxylic diimide (NTCDI), and/or a derivative thereof, a fullerene derivative, or any combination thereof.

For example, the n-type semiconductor may include a compound represented by Formula 120: 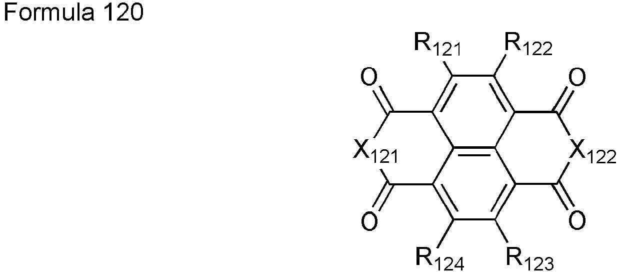 wherein, in Formula 120,
X₁₂₁ and X₁₂₂ are each independently O or NR₁₂₅, and
R₁₂₁ to R₁₂₄ and R₁₂₅ are each independently hydrogen, deuterium, a halogen, a cyano group, a nitro group, a hydroxy group, a C₁-C₃₀ (*e.g.* C₁-C₂₀) alkyl group substituted or unsubstituted with at least one R₁₀ₐ, a C₆-C₃₀ *(*e.g. C₆-C₂₀) aryl group substituted or unsubstituted with at least one R₁₀ₐ, a C₃-C₃₀ (*e.g.* C₃-C₂₀) heteroaryl group substituted or unsubstituted with at least one R₁₀ₐ, or any combination thereof.

In an embodiment, the compound represented by Formula 120 may be a compound represented by Formula 121 or 122: 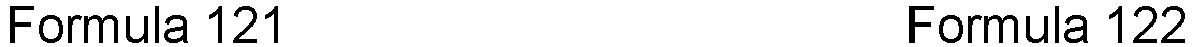 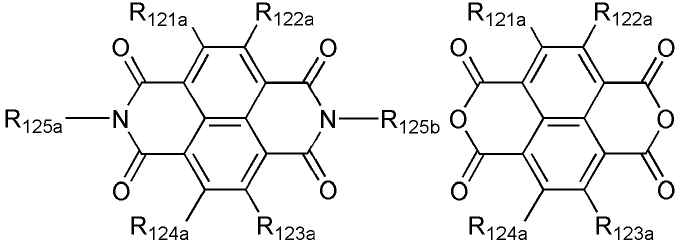 wherein, in Formulae 121 and 122,
R₁₂₁ₐ to R₁₂₄ₐ are each independently hydrogen, deuterium, a halogen, a cyano group, a C₁-C₃₀ (*e.g.* C₁-C₂₀) alkyl group, a C₆-C₃₀ (*e.g.* C₆-C₂₀) aryl group, or a C₃-C₃₀ (*e.g.* C₃-C₂₀) heteroaryl group, and
R₁₂₅ₐ and R_{125b} are each independently: hydrogen, deuterium, a halogen, or a cyano group; a C₁-C₃₀ (*e.g.* C₁-C₂₀) alkyl group that is unsubstituted or substituted with at least one R₁₀ₐ; a C₆-C₃₀ (e.g. C₆-C₂₀) aryl group that is unsubstituted or substituted with at least one R₁₀ₐ or a C₃-C₃₀ (*e.g.* C₃-C₂₀) heteroaryl group that is unsubstituted or substituted with at least one R₁₀ₐ.

In an embodiment, R₁₂₁ₐ to R₁₂₄ₐ may each independently be hydrogen, deuterium, a halogen, a cyano group, a methyl group, or a phenyl group. For example, R₁₂₁ₐ to R₁₂₄ₐ may each independently be hydrogen.

In an embodiment, R₁₂₅ₐ and R_{125b} may each independently be: hydrogen, a halogen, or a cyano group; a C₁-C₃₀ (*e.g.* C₁-C₂₀) alkyl group; or a C₆-C₃₀ (*e.g.* C₆-C₂₀) aryl group that is substituted with an alkyl group substituted with a halogen, a cyano group, an alkyl group, or a halogen.

In an embodiment, the compound represented by Formula 120 may be selected from the following compounds:

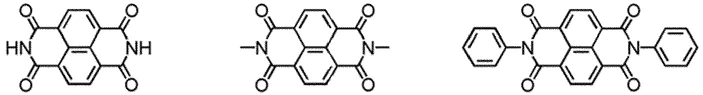

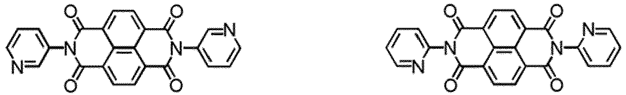

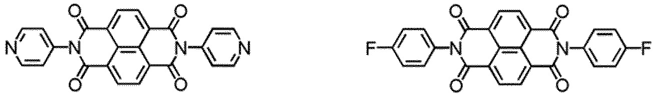

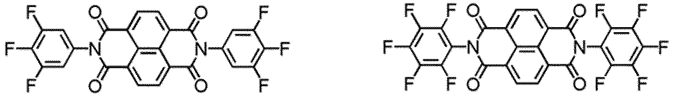

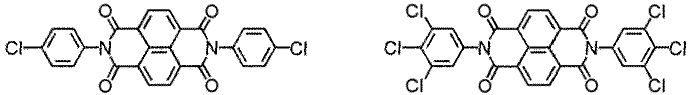

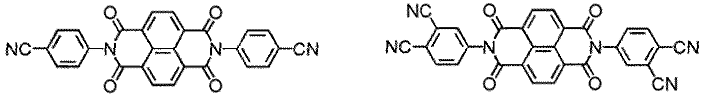

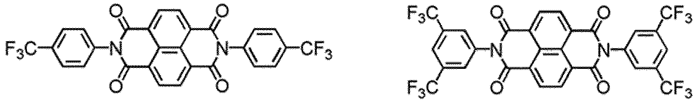

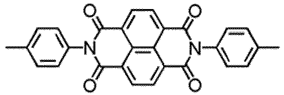

A first common layer may be included between the first electrode and the first auxiliary layer and between the second electrode and the second auxiliary layer.

A second common layer may be included between the photoactive layer and the common electrode and between the first emission layer and the common electrode.

The first common layer may include at least one selected from a hole injection layer, a hole transport layer, and an electron blocking layer.

The second common layer may include at least one selected from a buffer layer, a hole blocking layer, an electron transport layer, and an electron injection layer.

The first auxiliary layer may be in direct contact with the photoactive layer, and
the second auxiliary layer may be in direct contact with the first emission layer.

Material for forming the first auxiliary layer and the second auxiliary layer may include a fluorene-based compound, a carbazole-based compound, an arylamine-based compound, a dibenzofuran-based compound, a dibenzothiophene-based compound, or a dibenzosilole-based compound.

The organic light-emitting device and the organic photodiode may form a pixel, and the electronic apparatus may include a plurality of the pixels.

The organic light-emitting device may include two or more organic light-emitting devices.

The electronic apparatus may be a display apparatus.

The electronic apparatus according to another embodiment includes:
a substrate; and
a plurality of organic light-emitting devices and an organic photodiode that are arranged on the substrate.

The organic photodiode sequentially includes a first electrode, a first auxiliary layer, a photoactive layer, and a common electrode.

The plurality of the organic light-emitting devices include a first light-emitting device, a second light-emitting device, and a third light-emitting device.

The first light-emitting device sequentially includes a second electrode, a second auxiliary layer, a first emission layer, and a common electrode.

The second light-emitting device sequentially includes a third electrode, a third auxiliary layer, a second emission layer, and a common electrode.

The third light-emitting device sequentially includes a fourth electrode, a fourth auxiliary layer, a third emission layer, and a common electrode.

The second auxiliary layer, the third auxiliary layer, and the fourth auxiliary layer have different thicknesses from each other.

The first auxiliary layer has an identical thickness to a thickness of one selected from the second auxiliary layer, the third auxiliary layer, and the fourth auxiliary layer.

The photoactive layer has a thickness in a range of about 200 Å to about 300 Å.

In an embodiment, the first emission layer to third emission layer may each have a thickness in a range of 150 Å to 500 Å.

Thicknesses of the second auxiliary layer, the third auxiliary layer, and the fourth auxiliary layer may each be proportional to the wavelength of light emitted from each of the first light-emitting device, the second light-emitting device, and the third light-emitting device.

In an embodiment, the first light-emitting device may emit red light, the second light-emitting device may emit green light, and the third light-emitting device may emit blue light.

A resonance distance of the organic photodiode may be identical to that of the second light-emitting device. The first auxiliary layer may have an identical thickness to a thickness of the second auxiliary layer.

The organic photodiode may detect light emitted from the second light-emitting device and reflected from a subject.

The photoactive layer may include a p-type semiconductor and an n-type semiconductor.

The photoactive layer may have a planar heterojunction structure of the p-type semiconductor and the n-type semiconductor.

The photoactive layer may have a bulk heterojunction structure of the p-type semiconductor and the n-type semiconductor.

In an embodiment, the p-type semiconductor may include a compound represented by Formula 110, boron subphthalocyanine chloride (SubPc), copper (II) phthalocyanine (CuPc), zinc phthalocyanine (ZnPc), tetraphenyldibenzoperiflanthene (DBP), or any combination thereof.

In an embodiment, the n-type semiconductor may include a compound represented by Formula 120, 3,4,9,10-perylenetetracarboxylic dianhydride (PTCDA), N,N'-di(propoxyethyl)perylene-3,4,9,10-tetracarboxylic diimide (PTCDI), naphthalene tetracarboxylic anhydride (NTCDA), naphthalenetetracarboxylic diimide (NTCDI), and/or a derivative thereof, a fullerene derivative, or any combination thereof.

A first common layer may be included between the first electrode and the first auxiliary layer, between the second electrode and the second auxiliary layer, between the third electrode and the third auxiliary layer, and between the fourth electrode and the fourth auxiliary layer.

A second common layer may be included between the first emission layer and the common electrode, between the second emission layer and the common electrode, between the third emission layer and the common electrode, and between the photoactive layer and the common electrode.

The first common layer may include at least one selected from a hole injection layer, a hole transport layer, and an electron blocking layer.

The second common layer may include at least one selected from a buffer layer, a hole blocking layer, an electron transport layer, and an electron injection layer.

The first auxiliary layer may be in direct contact with the photoactive layer,
the second auxiliary layer may be in direct contact with the first emission layer,
the third auxiliary layer may be in direct contact with the second emission layer, and
the fourth auxiliary layer may be in direct contact with the third emission layer.

A material for forming the first auxiliary layer, the second auxiliary layer, the third auxiliary layer, and the fourth auxiliary layer may include, for example, a fluorene-based compound, a carbazole-based compound, an arylamine-based compound, a dibenzofuran-based compound, a dibenzothiophene-based compound, and/or a dibenzosilole-based compound.

In an embodiment, the plurality of the organic light-emitting devices and the organic photodiode may form a pixel, and the electronic apparatus may include a plurality of the pixels.

In an embodiment, the electronic apparatus may include a plurality of the pixels, and the plurality of the pixels may all include the organic photodiode.

In an embodiment, the electronic apparatus may further include a fourth light-emitting device, and the fourth light-emitting device may be identical to one selected from the first light-emitting device, the second light-emitting device, and the third light-emitting device.

In an embodiment, the organic photodiode may be arranged adjacent to the second light-emitting device.

The electronic apparatus may be a display apparatus.

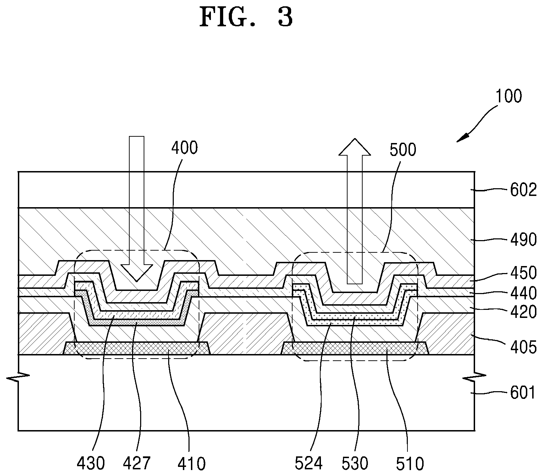

### Description of FIGS. 3-4

FIG. 3 is a schematic cross-sectional view of an electronic apparatus 100 according to an embodiment.

Referring to FIG. 3, the electronic apparatus 100 includes an organic photodiode 400 and an organic light-emitting device 500 that are arranged between two substrates 601 and 602. The organic photodiode 400 includes a first electrode 410, a first common layer 420, a first auxiliary layer 427, a photoactive layer 430, a second common layer 440, and a common electrode 450, and the organic light-emitting device 500 includes a second electrode 510, a first common layer 420, a second auxiliary layer 524, an emission layer 530, a second common layer 440, and a common electrode 450.

The substrates 601 and 602 may each independently be a flexible substrate, a glass substrate, and/or a metal substrate. A buffer layer and a thin-film transistor may be arranged on the substrate 601.

The buffer layer may serve to prevent or reduce infiltration of impurities through the substrate 601 and provide a flat surface on the substrate 601. The thin-film transistor is arranged on the buffer layer, and may include an activation layer, a gate electrode, a source electrode, and a drain electrode.

In an embodiment, the thin-film transistor may be electrically connected to the organic light-emitting device 500 to drive the same. One selected from the second electrode and the drain electrode may be electrically connected to the second electrode 510 of the organic light-emitting device 500.

In one or more embodiments, the thin-film transistor may be electrically connected to the organic photodiode 400. One selected from the second electrode and the drain electrode may be electrically connected to the first electrode 410 of the organic photodiode 400.

In an embodiment, the first electrode 410 may be an anode, and the common electrode 450 may be a cathode. In one or more embodiments, by applying a reverse bias between the first electrode 410 and the common electrode 450 to drive the organic photodiode 400, the electronic apparatus 100 may absorb light incident to the organic photodiode 400, generate a charge, and detect light by extraction as a current.

When the photoactive layer absorbs light, electrons at a highest occupied molecular orbital (HOMO) may be transferred to a LUMO, thereby generating excitons. In the PN bond-based photoactive layer, electron-hole pairs may be formed by transporting excitons, which are generated by absorbing light from a donor (p-type), to the interface of an acceptor and donating the excitons to the acceptor. In the case of an inorganic material, the electrons and the holes may be separated at room temperature because the energy required to separate the electron-hole pairs is only a few meV, whereas, in the case of an organic material, high energy of about 100 meV is required. The separated electrons and holes move to the anode and the cathode, respectively, to generate current. To achieve this, the loss at the interface between the photoactive layer and the electrode should be minimized or reduced, and the electrons and holes separated inside the photoactive layer may move quickly to the electrode.

In an embodiment, the second electrode 510 may be an anode, and the common electrode 450 may be a cathode. In one or more embodiments, in the organic light-emitting device 500, holes injected to the second electrode 510 and electrons injected to the common electrode 450 recombine in the emission layer 530 to form excitons, and the excitons may transition from an excited state to a ground state, thereby generating light.

Descriptions of the first electrode 410 and the second electrode 510 may refer to a description of the anode 110 provided in the specification.

At edges of the first electrode 410 and the second electrode 510, a pixel defining layer 405 may be formed. The pixel defining layer 405 defines a pixel region, and may electrically separate the first electrode 410 and the second electrode 510 from each other. The pixel defining layer 405 may include, for example, various suitable organic insulating materials (for example, silicone-based materials, and/or the like), inorganic insulating materials, and/or organic/inorganic composite insulating materials. The pixel defining layer 405 may be a transmissive film that transmits visible light, or a blocking film that blocks (or reduces transmission of) visible light.

The first common layer 420 and the second common layer 440 may be arranged as common layers over both the organic photodiode 400 and the organic light-emitting device 500. The first common layer 420 may include, for example, a hole injection layer, a hole transport layer, an electron blocking layer, or any combination thereof. The second common layer 440 may include, for example, a buffer layer, a hole blocking layer, an electron transport layer, an electron injection layer, or any combination thereof. The first common layer 420 and the second common layer 440 may each be defined as described herein.

As such, because the electronic apparatus 100 uses common layers in the organic photodiode 400 and the organic light-emitting device 500, these two devices may be arranged in-pixel in the electronic apparatus 100. In this regard, a manufacturing process of the electronic apparatus 100 may be also simplified.

In the organic photodiode 400, the photoactive layer 430 is arranged on the first common layer 420. In the organic light-emitting device 500, the emission layer 530 is arranged on the first common layer 420. The emission layer 530 may be formed by using various suitable luminescent materials generally used in the art. For example, as the luminescent material, an organic material, an inorganic material, or a quantum dot may be used. A description of the photoactive layer 430 may refer to the descriptions provided in the specification, and a description of the emission layer 530 may refer to descriptions provided below.

The second common layer 440 and the common electrode 450 may be formed as common layers over the photoactive 430 and the emission layer 530 throughout the organic photodiode 400 and the organic light-emitting device 500. A description of the common electrode 450 may refer to that of the cathode 150 provided in the specification.

A capping layer may be arranged on the common electrode 450. A material that is used for the capping layer may include an organic material and/or an inorganic material as described above. The capping layer may serve not only to protect the organic photodiode 400 and the organic light-emitting device 500, but also to help light generated from the organic light-emitting device 500 emitted efficiently. A description of the capping layer may refer to that of the capping layer provided in the specification.

An encapsulation portion 490 may be arranged on the capping layer. The encapsulation portion 490 may be arranged on the organic photodiode 400 and the organic light-emitting device 500, and thus may serve to protect the organic photodiode 400 and the organic light-emitting device 500 from moisture and/or oxygen. The encapsulation portion 490 may include: an inorganic film including silicon nitride (SiNₓ), silicon oxide (SiOₓ), indium tin oxide, indium zinc oxide, or any combination thereof; an organic film including polyethylene terephthalate, polyethylene naphthalate, polycarbonate, polyimide, polyethylene sulfonate, polyoxymethylene, polyarylate, hexamethyldisiloxane, an acrylic resin (for example, polymethyl methacrylate, polyacrylic acid, and/or the like), an epoxy-based resin (for example, aliphatic glycidyl ether (AGE), and/or the like), or any combination thereof; or a combination of the inorganic film and the organic film.

The electronic apparatus 100 may be, for example, a display apparatus. Because the electronic apparatus 100 includes both the organic photodiode 400 and the organic light-emitting device 500, the electronic apparatus 100 may be a display apparatus that exhibits a function of light detection.

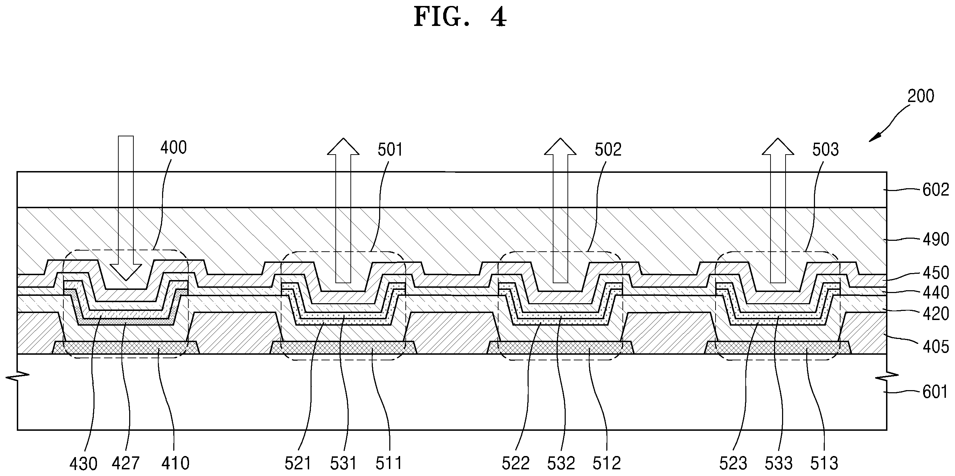

FIG. 4 is a schematic cross-sectional view of an electronic apparatus 200 according to another embodiment.

Referring to FIG. 4, the electronic apparatus 200 includes the organic photodiode 400 and the organic light-emitting devices 501, 502, and 503 that are arranged between two substrates 601 and 602. The electronic apparatus 200 of FIG. 4 is different from the electronic apparatus 100 including one organic light-emitting device in that the electronic apparatus 200 includes a plurality of organic light-emitting devices. The organic light-emitting devices 501, 502, and 503 may each be used to implement full color. Components identical to those of FIG. 3 may refer to the description of the electronic apparatus 200.

The organic photodiode 400 includes a first electrode 410, a first common layer 420, a first auxiliary layer 427, a photoactive layer 430, a second common layer 440, and a common electrode 450.

The first organic light-emitting device 501 includes a second electrode 511, the first common layer 420, a second auxiliary layer 521, a first emission layer 531, the second common layer 440, and the common electrode 450.

The second organic light-emitting device 502 includes a third electrode 512, the first common layer 420, a third auxiliary layer 522, a second emission layer 532, a second common layer 440, and the common electrode 450.

The third organic light-emitting device 503 includes a fourth electrode 513, the first common layer 420, a fourth auxiliary layer 523, a third emission layer 533, the second common layer 440, and the common electrode 450.

The organic photodiode 400 may be electrically connected to the thin-film transistor to drive the organic photodiode 400. The first electrode 410 of the organic photodiode 400 may be electrically connected to one selected from a source electrode and a drain electrode of the thin-film transistor.

Each of the organic light-emitting devices 501, 502, and 503 may be electrically connected to different thin-film transistors to drive the organic light-emitting devices 501, 502, and 503. Each of the second electrode 511, the third electrode 512, and the fourth electrode 513 in the organic light-emitting devices 501, 502, and 503 may be electrically connected to one selected from a source electrode and a drain electrode of different thin-film transistors.

In an embodiment, the second electrode 511, the third electrode 512, and the fourth electrode 513 may each be an anode, and the common electrode 450 may be a cathode. In one or more embodiments, in the organic light-emitting device 501, 502, or 503, holes injected to the second electrode 511, the third electrode 512, or the fourth electrode 513 and electrons injected to the common electrode 450 may recombine in the emission layer 531, 532, or 533 to form excitons, and the excitons may transition from an excited state to a ground state, thereby generating light.

Descriptions of the first electrode 410 and the second electrode 511, the third electrode 512, and the fourth electrode 513 may refer to a description of the anode 110 provided in the specification.

At edges of the first electrode 410 and the second electrode 511, the third electrode 512, and the fourth electrode 513, a pixel defining layer 405 may be formed. The pixel defining layer 405 defines a pixel region, and may electrically separate the first electrode 410 from each of the second electrode 511, the third electrode 512, and the fourth electrode 513. The pixel defining layer 405 may include, for example, various suitable organic insulating materials (for example, silicone-based materials, and/or the like), inorganic insulating materials, and/or organic/inorganic composite insulating materials. The pixel defining layer 405 may be a transmissive film that transmits visible light, or a blocking film that blocks (or reduces transmission of) visible light.

The first common layer 420 and the second common layer 440 may be arranged as common layers over all of the organic photodiode 400 and the first organic light-emitting device 501, the second organic light-emitting device 502, and the third organic light-emitting device 503. The first common layer 420 may include, for example, a hole injection layer, a hole transport layer, an electron blocking layer, or any combination thereof. The second common layer 440 may include, for example, a buffer layer, a hole blocking layer, an electron transport layer, an electron injection layer, or any combination thereof.

On the first common layer 420, the photoactive layer 430 is formed to correspond to the organic photodiode 400.

On the first common layer 420, the emission layers 531, 532, and 533 are formed to correspond to the organic light-emitting devices 501, 502, and 503, respectively.

The first emission layer 531 may emit a first-color light, the second emission layer 532 may emit a second-color light, and the third emission layer 533 may emit a third-color light.

A maximum emission wavelength of the first-color light, a maximum emission wavelength of the second-color light, and a maximum emission wavelength of the third-color light may be identical to or different from each other. For example, each of the maximum emission wavelength of the first-color light and the maximum emission wavelength of the second-color light may be longer than the maximum emission wavelength of the third-color light.

In an embodiment, the first-color light may be red light, the second-color light may be green light, and the third-color light may be blue light, but embodiments of the disclosure are not limited thereto. Therefore, the electronic apparatus 100 may emit full color light. The first-color light, the second-color light, and the third-color light are not limited to red light, green light, and blue light, respectively, and may be any combination of light of different colors, as long as mixed light thereof is white light.

The first emission layer 531, the second emission layer 532, and the third emission layer 533 may each be formed by using various suitable luminescent materials generally used in the art. For example, as the luminescent material, an organic material, an inorganic material, and/or a quantum dot may be used.

The organic photodiode 400, the first organic light-emitting device 501, the second organic light-emitting device 502, and the third organic light-emitting device 503 may all form a pixel, and may each be a sub-pixel constituting the pixel.

In an embodiment, the pixel may include at least one organic photodiode 400.

The second common layer 440 and the common electrode 450 may be sequentially formed over the photoactive layer 430 and the emission layers 531, 532, and 533.

A capping layer may be arranged on the common electrode 450.

An encapsulation portion 490 may be arranged on the capping layer. The encapsulation portion 490 may be arranged on the organic photodiode 400 and the organic light-emitting devices 501, 502, and 503, and thus may serve to protect the organic photodiode 400 and the organic light-emitting devices 501, 502, and 503 from moisture and/or oxygen. The encapsulation portion 490 may include: an inorganic film including silicon nitride (SiNₓ), silicon oxide (SiOₓ), indium tin oxide, indium zinc oxide, or any combination thereof; an organic film including polyethylene terephthalate, polyethylene naphthalate, polycarbonate, polyimide, polyethylene sulfonate, polyoxymethylene, polyarylate, hexamethyldisiloxane, an acrylic resin (for example, polymethyl methacrylate, polyacrylic acid, and/or the like), an epoxy-based resin (for example, aliphatic glycidyl ether (AGE), and/or the like), or any combination thereof; or a combination of the inorganic film and the organic film.

The electronic apparatus 200 may be a display apparatus. Because the electronic apparatus 200 includes all of the organic photodiode 400 and the first organic light-emitting device 501, the second organic light-emitting device 502, and the third organic light-emitting device 503, the electronic apparatus 200 may be a full-color display apparatus that exhibits a function of light detection.

Hereinafter, the emission layers 530, 531, 532, and 533 of FIGS. 3-4 will be described in more detail.

### Emission layer

The emission layer may be a red emission layer, a green emission layer, a blue emission layer, or a white emission layer. The emission layer may have a structure including (or consisting of) a red luminescent material, a green luminescent material, or a blue luminescent material only, or a structure in which these luminescent materials are mixed together.

In an embodiment, the emission layer may include a host and a dopant. The dopant may include a phosphorescent dopant, a fluorescent dopant, or any combination thereof.

An amount of the dopant in the emission layer may be in a range of about 0.01 parts by weight to about 15 parts by weight based on 100 parts by weight of the host.

In one or more embodiments, the emission layer may include a quantum dot.

In one or more embodiments, the emission layer may include a delayed fluorescence material. The delayed fluorescence material may act as a host or a dopant in the emission layer.

### Host

In an embodiment, the host may include a compound represented by Formula 301:

Formula 301 [Ar₃₀₁]_{xb11}-[(L₃₀₁)_{xb1}-R₃₀₁]_{xb21}

wherein, in Formula 301,
Ar₃₀₁ and L₃₀₁ may each independently be a C₃-C₆₀ (e.g. C₃-C₃₀) carbocyclic group that is unsubstituted or substituted with at least one R₁₀ₐ or a C₁-C₆₀ (e.g. C₁-C₂₀) heterocyclic group that is unsubstituted or substituted with at least one R₁₀ₐ,
xb11 may be 1, 2, or 3,
xb1 may be an integer from 0 to 5,
R₃₀₁ may be hydrogen, deuterium, -F, -Cl, -Br, -I, a hydroxyl group, a cyano group, a nitro group, a C₁-C₆₀ (e.g. C₁-C₂₀) alkyl group unsubstituted or substituted with at least one R₁₀ₐ, a C₂-C₆₀ (*e.g.* C₂-C₂₀) alkenyl group unsubstituted or substituted with at least one R₁₀ₐ, a C₂-C₆₀ (*e.g.* C₂-C₂₀) alkynyl group unsubstituted or substituted with at least one R₁₀ₐ, a C₁-C₆₀ (*e.g.* C₁-C₂₀) alkoxy group unsubstituted or substituted with at least one R₁₀ₐ, a C₃-C₆₀ (*e.g.* C₃-C₃₀) carbocyclic group unsubstituted or substituted with at least one R₁₀ₐ, a C₁-C₆₀ (*e.g.* C₁-C₂₀) heterocyclic group unsubstituted or substituted with at least one R₁₀ₐ,-Si(Q₃₀₁)(Q₃₀₂)(Q₃₀₃), -N(Q₃₀₁)(Q₃₀₂), -B(Q₃₀₁)(Q₃₀₂),-C(=O)(Q₃₀₁), -S(=O)₂(Q₃₀₁), or -P(=O)(Q₃₀₁)(Q₃₀₂),
xb21 may be an integer from 1 to 5, and
Q₃₀₁ to Q₃₀₃ may each be as defined with respect to Q₁₁.

For example, when xb11 in Formula 301 is 2 or more, two or more of Ar₃₀₁ may be linked to each other via a single bond.

In one or more embodiments, the host may include a compound represented by Formula 301-1, a compound represented by Formula 301-2, or any combination thereof: 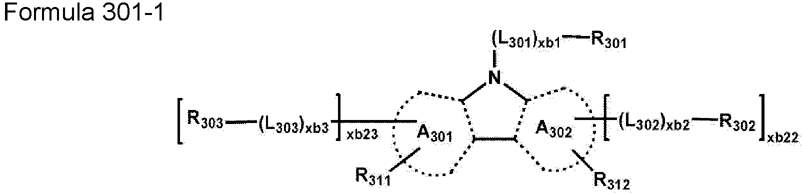 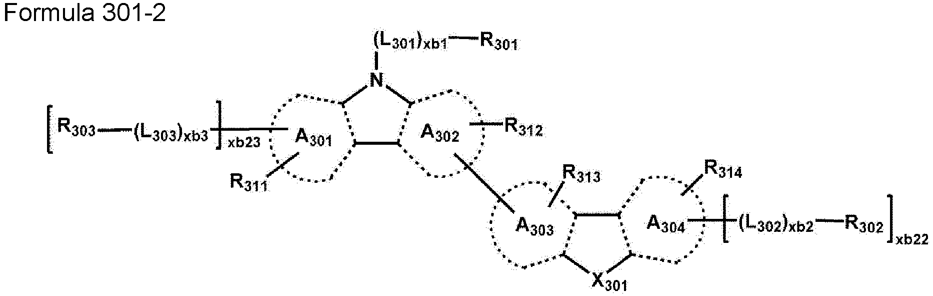 wherein, in Formulae 301-1 and 301-2,
ring A₃₀₁ to ring A₃₀₄ may each independently be a C₃-C₆₀ (e.g. C₃-C₃₀) carbocyclic group unsubstituted or substituted with at least one R₁₀ₐ or a C₁-C₆₀ (e.g. C₁-C₂₀) heterocyclic group unsubstituted or substituted with at least one R₁₀ₐ,
X₃₀₁ may be O, S, N-[(L₃₀₄)_{xb4}-R₃₀₄], C(R₃₀₄)(R₃₀₅), or Si(R₃₀₄)(R₃₀₅),
xb22 and xb23 may each independently be 0, 1, or 2,
L₃₀₁, xb1, and R₃₀₁ may each be as defined herein,
L₃₀₂ to L₃₀₄ may each independently be as defined with respect to L₃₀₁,
xb2 to xb4 may each independently be as defined with respect to xb1, and
R₃₀₂ to R₃₀₅ and R₃₁₁ to R₃₁₄ may each independently be as defined with respect to R₃₀₁.

In one or more embodiments, the host may include an alkali earth metal complex, a post-transition metal complex, or any combination thereof. In one or more embodiments, the host may include a Be complex (for example, Compound H55), an Mg complex, a Zn complex, or any combination thereof.

In an embodiment, the host may include one selected from Compounds H1 to H126, 9,10-di(2-naphthyl)anthracene (ADN), 2-methyl-9,10-bis(naphthalen-2-yl)anthracene (MADN), 9,10-di-(2-naphthyl)-2-t-butyl-anthracene (TBADN), 4,4'-bis(N-carbazolyl)-1,1'-biphenyl (CBP), 1,3-di-9-carbazolylbenzene (mCP), 1,3,5-tri(carbazol-9-yl)benzene (TCP), or any combination thereof:

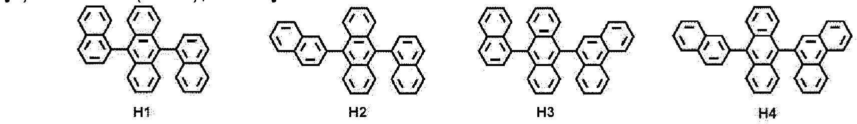

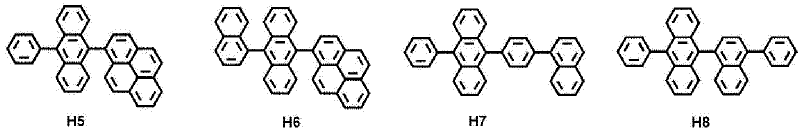

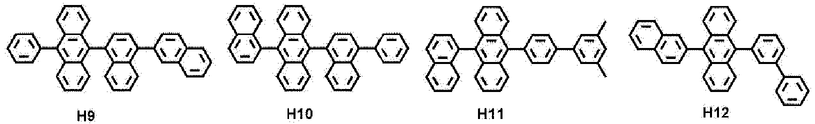

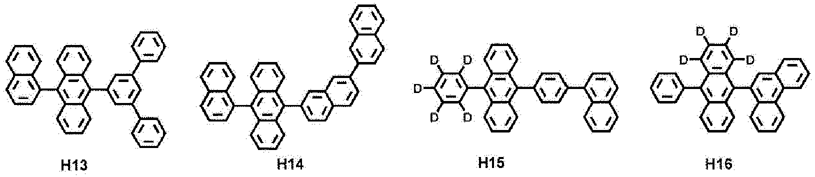

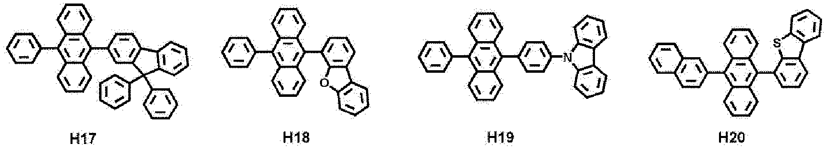

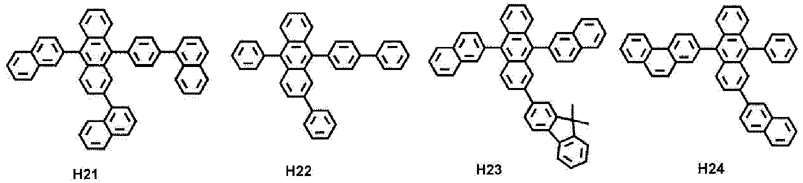

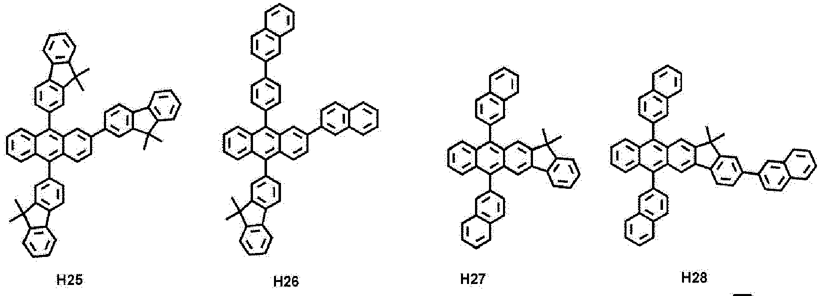

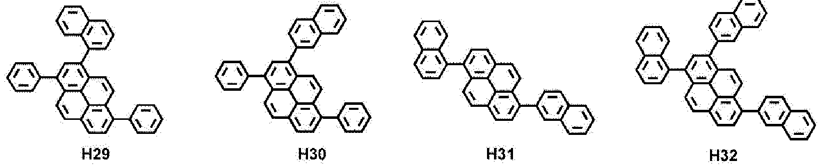

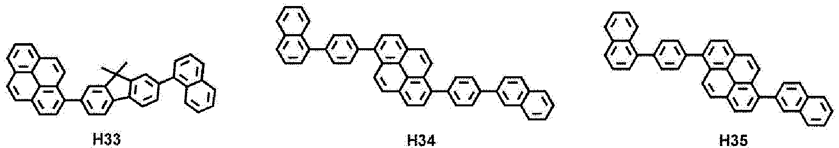

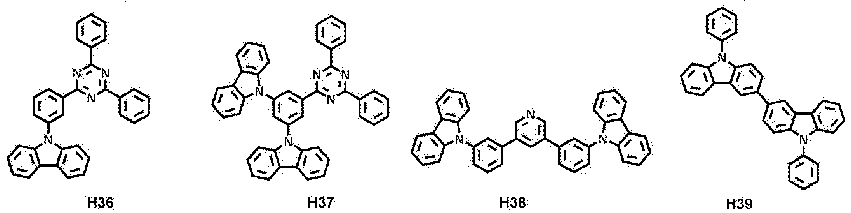

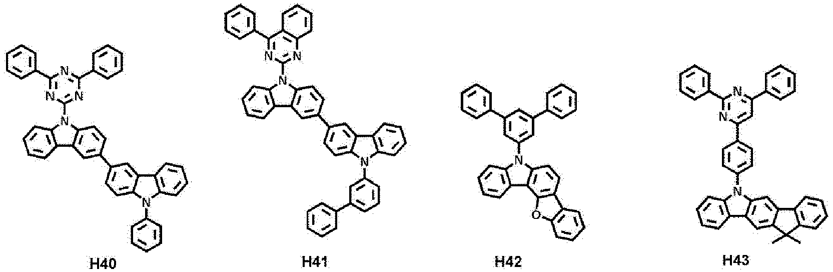

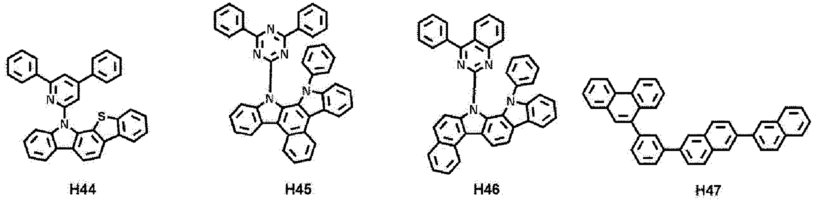

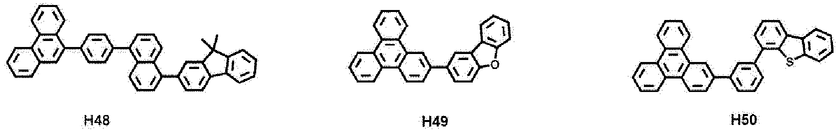

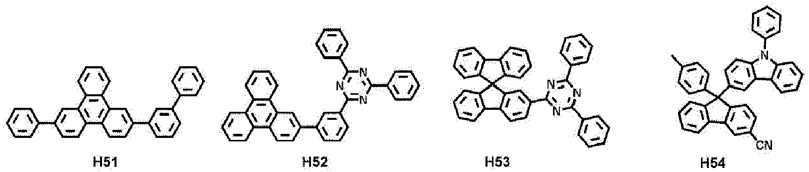

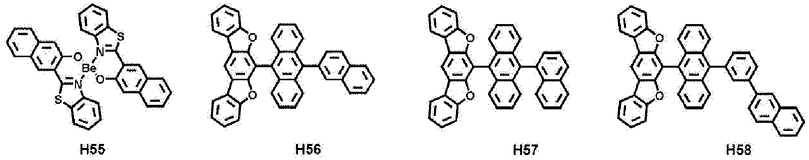

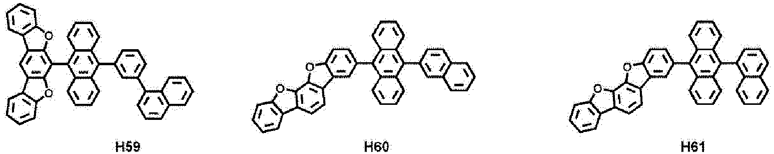

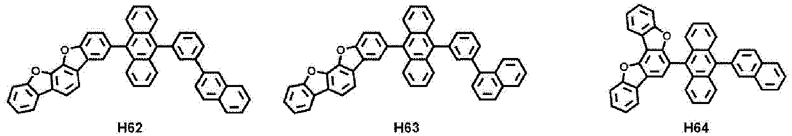

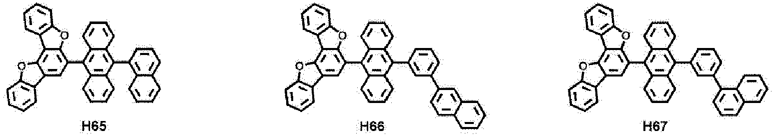

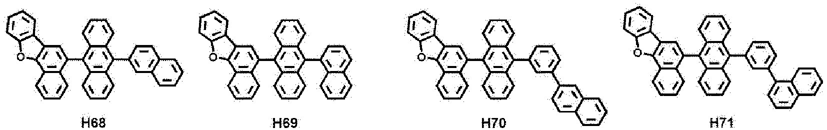

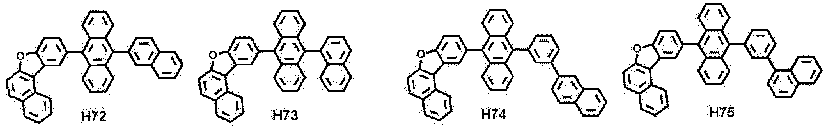

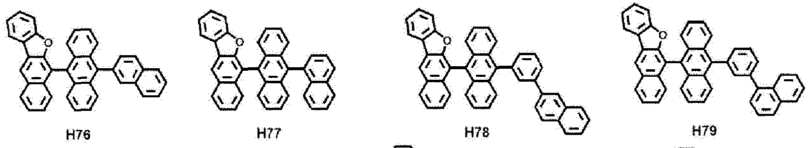

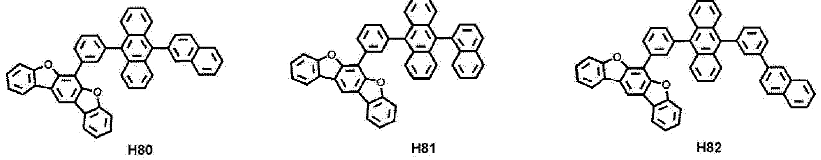

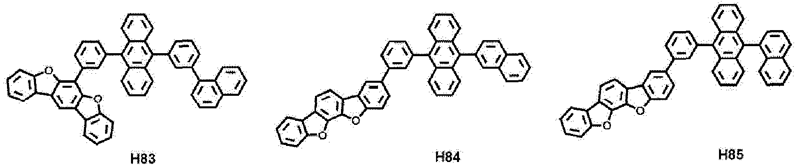

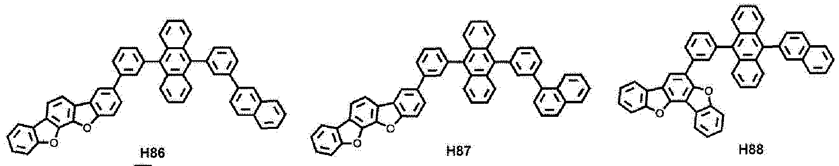

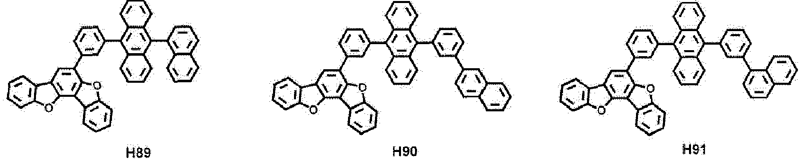

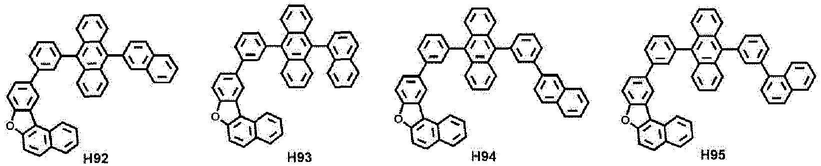

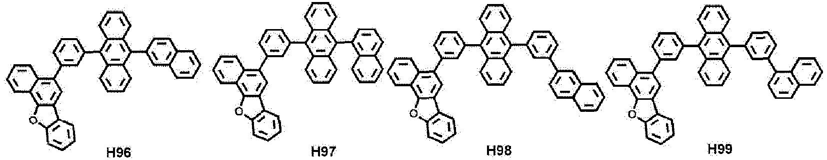

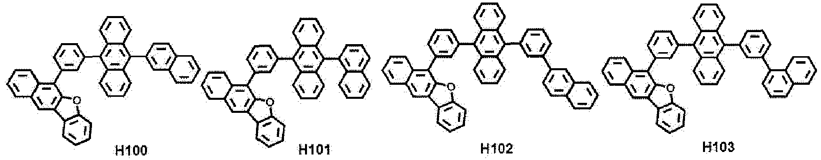

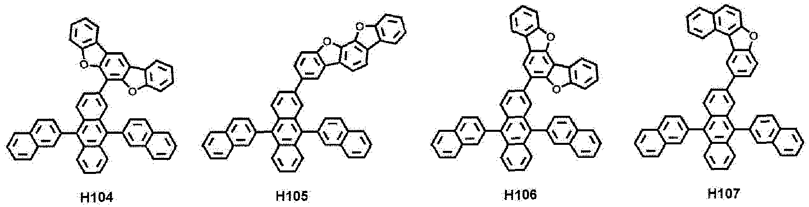

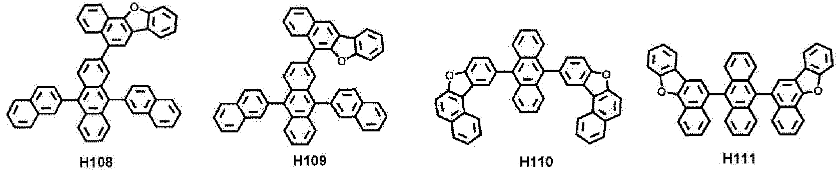

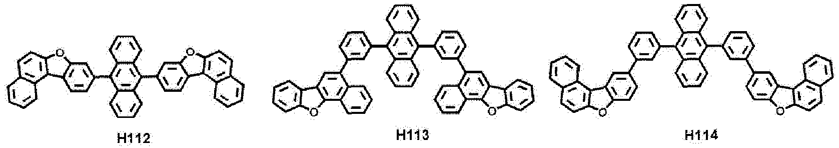

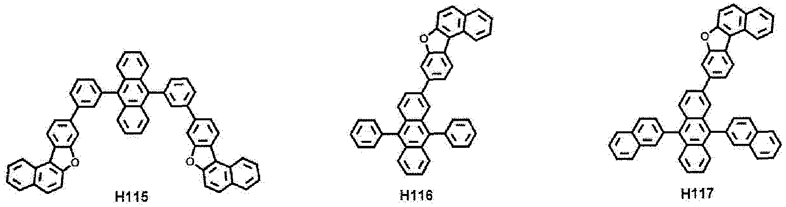

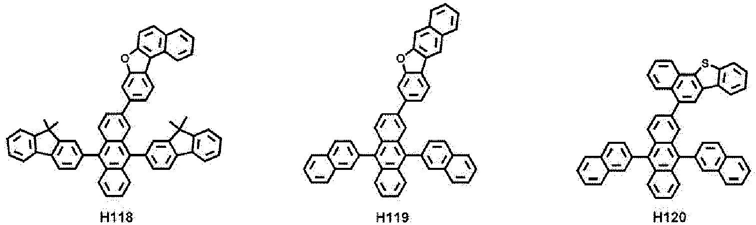

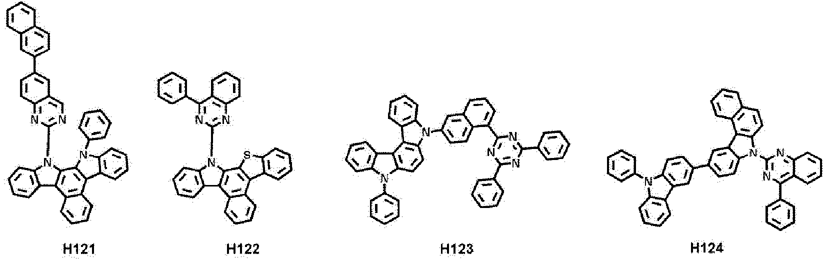

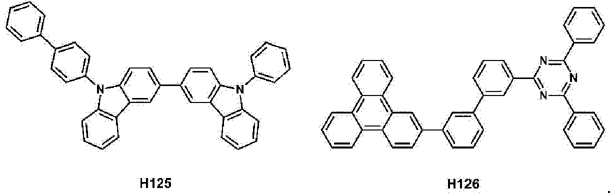

### Phosphorescent dopant

The phosphorescent dopant may include at least one transition metal as a central metal.

The phosphorescent dopant may include a monodentate ligand, a bidentate ligand, a tridentate ligand, a tetradentate ligand, a pentadentate ligand, a hexadentate ligand, or any combination thereof.

The phosphorescent dopant may be electrically neutral.

For example, the phosphorescent dopant may include an organometallic compound represented by Formula 401:

Formula 401 M(L₄₀₁)_{xc1}(L₄₀₂)_{xc2}

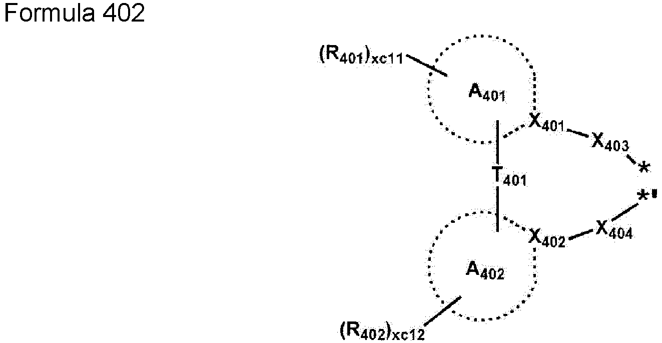

wherein, in Formulae 401 and 402,
M may be transition metal (for example, iridium (Ir), platinum (Pt), palladium (Pd), osmium (Os), titanium (Ti), gold (Au), hafnium (Hf), europium (Eu), terbium (Tb), rhodium (Rh), rhenium (Re), or thulium (Tm)),
L₄₀₁ may be a ligand represented by Formula 402, and xc1 is 1, 2, or 3, wherein, when xc1 is 2 or more, two or more of L₄₀₁ may be identical to or different from each other,
L₄₀₂ may be an organic ligand, and xc2 may be 0, 1, 2, 3, or 4, wherein, when xc2 is 2 or more, two or more of L₄₀₂ may be identical to or different from each other,
X₄₀₁ and X₄₀₂ may each independently be N or C,
ring A₄₀₁ and ring A₄₀₂ may each independently be a C₃-C₆₀ (*e.g.* C₃-C₃₀) carbocyclic group or a C₁-C₆₀ (*e.g.* C₁-C₂₀) heterocyclic group,
T₄₀₁ may be a single bond, -O-, -S-, -C(=O)-, -N(Q₄₁₁)-, -C(Q₄₁₁)(Q₄₁₂)-,-C(Q₄₁₁)=C(Q₄₁₂)-, -C(Q₄₁₁)=, or =C=,
X₄₀₃ and X₄₀₄ may each independently be a chemical bond (for example, a covalent bond or a coordination bond (which may also be referred to as a coordinate covalent bond or a dative bond)), O, S, N(Q₄₁₃), B(Q₄₁₃), P(Q₄₁₃), C(Q₄₁₃)(Q₄₁₄), or Si(Q₄₁₃)(Q₄₁₄),
Q₄₁₁ to Q₄₁₄ may each independently be as defined with respect to Q₁₁,
R₄₀₁ and R₄₀₂ may each independently be hydrogen, deuterium, -F, -Cl, -Br,-I, a hydroxyl group, a cyano group, a nitro group, a C₁-C₂₀ alkyl group unsubstituted or substituted with at least one R₁₀ₐ, a C₁-C₂₀ alkoxy group unsubstituted or substituted with at least one R₁₀ₐ, a C₃-C₆₀ (*e.g.* C₃-C₃₀) carbocyclic group unsubstituted or substituted with at least one R₁₀ₐ, a C₁-C₆₀ (*e.g.* C₁-C₂₀) heterocyclic group unsubstituted or substituted with at least one R₁₀ₐ, -Si(Q₄₀₁)(Q₄₀₂)(Q₄₀₃), -N(Q₄₀₁)(Q₄₀₂), -B(Q₄₀₁)(Q₄₀₂), -C(=O)(Q₄₀₁), -S(=O)₂(Q₄₀₁), or -P(=O)(Q₄₀₁)(Q₄₀₂),
Q₄₀₁ to Q₄₀₃ may each independently be as defined with respect to Q₁₁,
xc11 and xc12 may each independently be an integer from 0 to 10, and
* and *' in Formula 402 each indicate a binding site to M in Formula 401.

For example, in Formula 402, i) X₄₀₁ may be nitrogen and X₄₀₂ may be carbon, or ii) each of X₄₀₁ and X₄₀₂ may be nitrogen.

When xc1 in Formula 401 is 2 or more, in two or more of L₄₀₂, two ring A₄₀₁(s) may optionally be linked to each other via T₄₀₂, which is a linking group, and two ring A₄₀₂(s) may optionally be linked to each other via T₄₀₃, which is a linking group. T₄₀₂ and T₄₀₃ may each independently be as defined with respect to T₄₀₁.

In Formula 401, L₄₀₂ may be an organic ligand. For example, L₄₀₂ may include a halogen group, a diketone group (for example, an acetylacetonate group), a carboxylic acid group (for example, a picolinate group), -C(=O), an isonitrile group, a - CN group, a phosphorus group (for example, a phosphine group, a phosphite group, etc.), or any combination thereof.

The phosphorescent dopant may include, for example, one selected from Compounds PD1 to PD40 or any combination thereof:

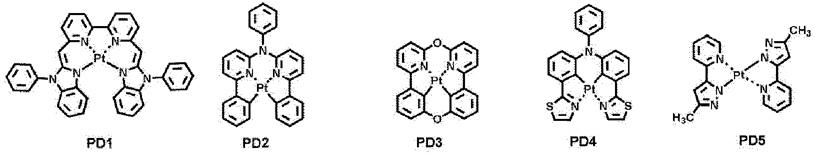

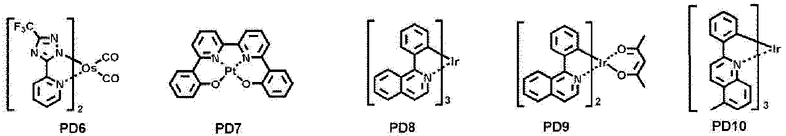

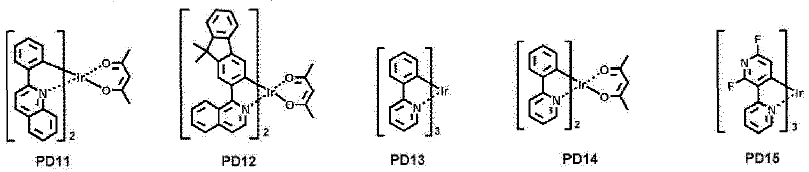

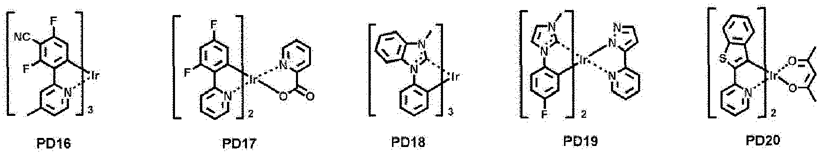

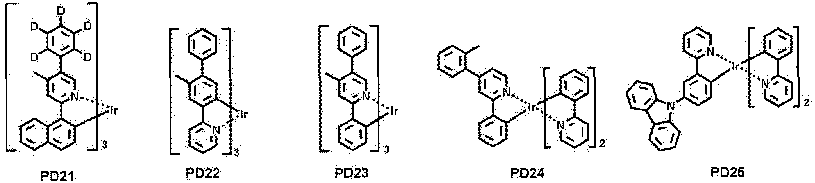

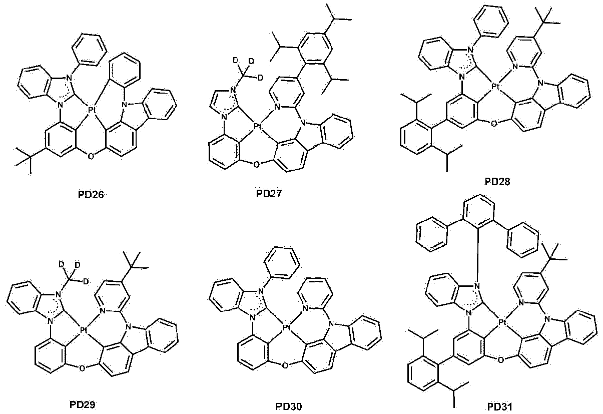

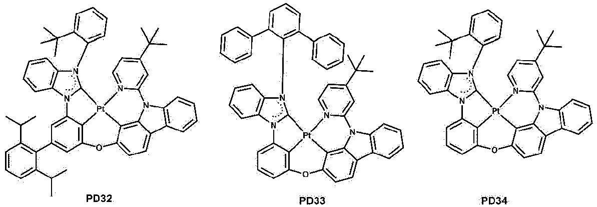

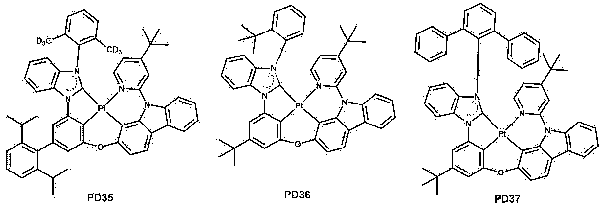

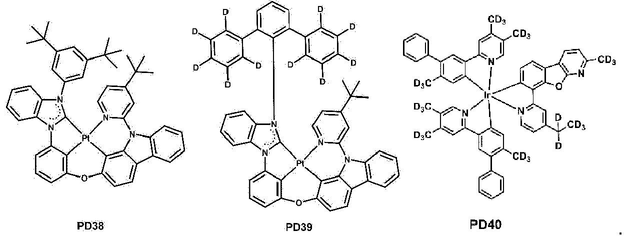

### Fluorescent dopant

The fluorescent dopant may include an amine group-containing compound, a styryl group-containing compound, or any combination thereof.

For example, the fluorescent dopant may include a compound represented by Formula 501: 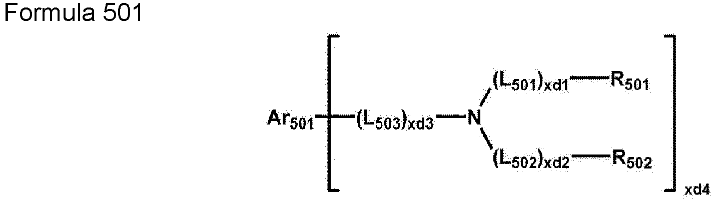 wherein, in Formula 501,
Ar₅₀₁, L₅₀₁ to L₅₀₃, R₅₀₁, and R₅₀₂ may each independently be a C₃-C₆₀ (*e.g.* C₃-C₃₀) carbocyclic group unsubstituted or substituted with at least one R₁₀ₐ or a C₁-C₆₀ (*e.g.* C₁-C₂₀) heterocyclic group unsubstituted or substituted with at least one R₁₀ₐ,
xd1 to xd3 may each independently be 0, 1, 2, or 3, and
xd4 may be 1, 2, 3, 4, 5, or 6.

In an embodiment, Ar₅₀₁ in Formula 501 may be a condensed cyclic group (for example, an anthracene group, a chrysene group, a pyrene group, etc.) in which three or more monocyclic groups are condensed together.

In an embodiment, xd4 in Formula 501 may be 2.

For example, the fluorescent dopant may include: one selected from Compounds FD1 to FD36; DPVBi; DPAVBi; or any combination thereof:

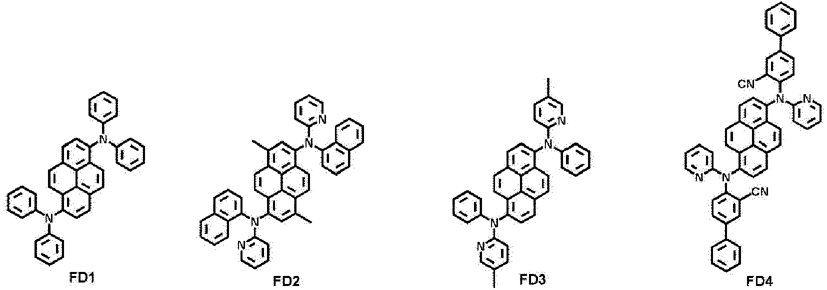

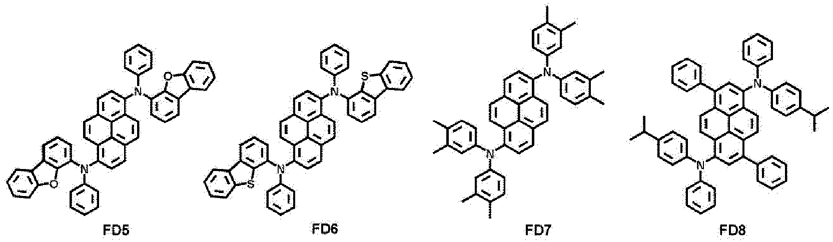

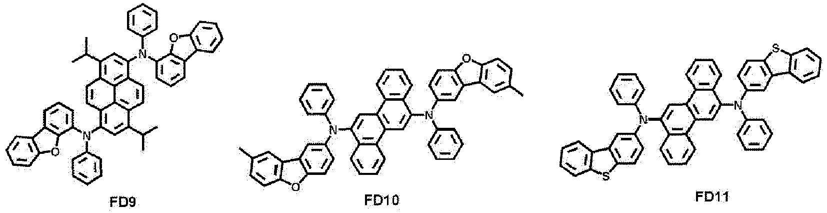

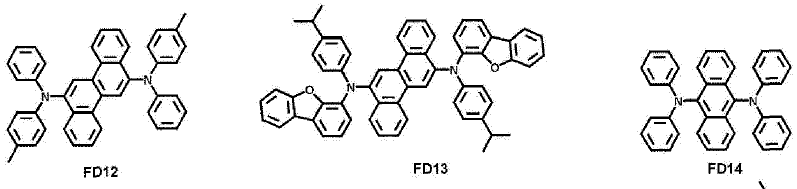

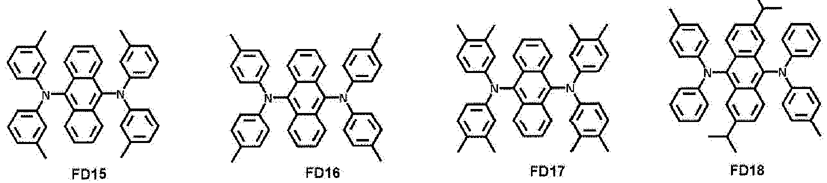

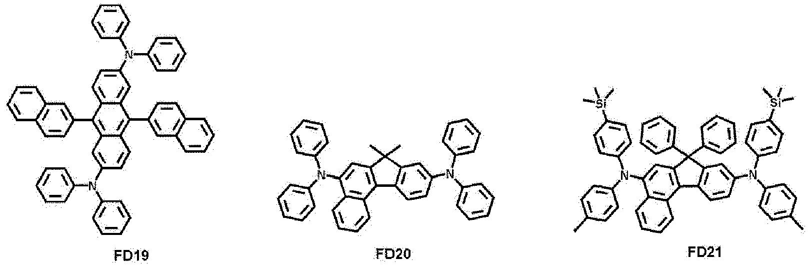

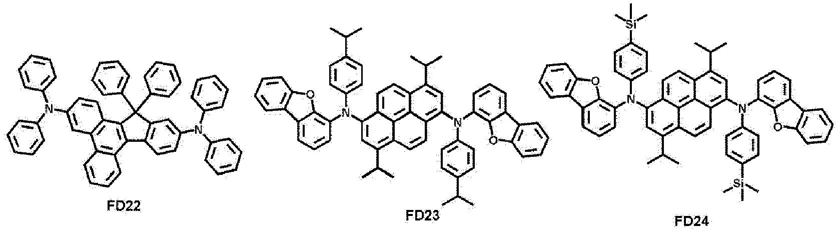

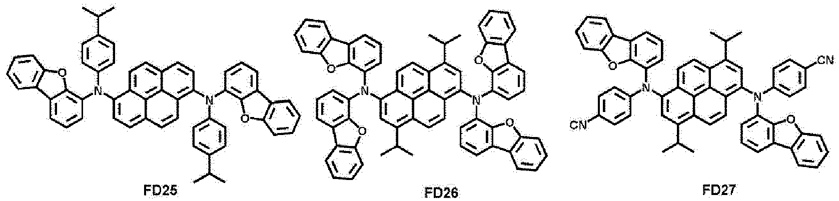

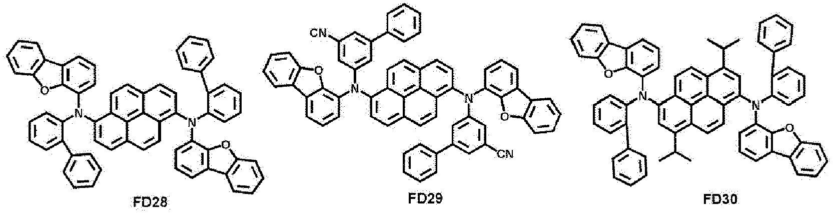

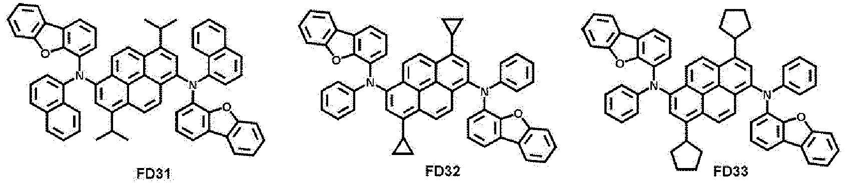

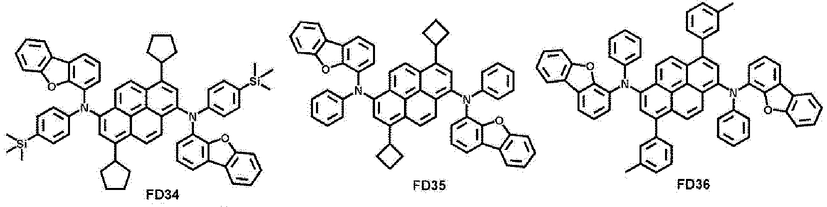

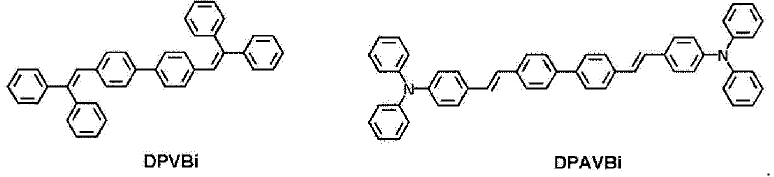

### Delayed fluorescence material

The emission layer may include a delayed fluorescence material.

In the specification, the delayed fluorescence material may be selected from compounds capable of emitting delayed fluorescence based on a delayed fluorescence emission mechanism.

The delayed fluorescence material included in the emission layer may act as a host or a dopant depending on the type (or kind) of other materials included in the emission layer.

In an embodiment, a difference between a triplet energy level (eV) of the delayed fluorescence material and the singlet energy level (eV) of the delayed fluorescence material may be greater than or equal to 0 eV and less than or equal to 0.5 eV. When the difference between the triplet energy level (eV) of the delayed fluorescence material and the singlet energy level (eV) of the delayed fluorescence material is satisfied within the range above, up-conversion from the triplet state to the singlet state of the delayed fluorescence materials may effectively occur, and thus, the light-emitting device 10 may have improved luminescence efficiency.

For example, the delayed fluorescence material may include: i) a material including at least one electron donor (for example, a π electron-rich C₃-C₆₀ (*e.g.* C₃-C₃₀) cyclic group and/or the like, such as a carbazole group) and at least one electron acceptor (for example, a sulfoxide group, a cyano group, a π electron-deficient nitrogen-containing C₁-C₆₀ (*e.g.* C₁-C₃₀) cyclic group, and/or the like), ii) a material including a C₈-C₆₀ (*e.g.* C₃-C₃₀) polycyclic group including at least two cyclic groups condensed to each other while sharing boron (B), and/or the like.

Examples of the delayed fluorescence material may include at least one selected from Compounds DF1 to DF9:

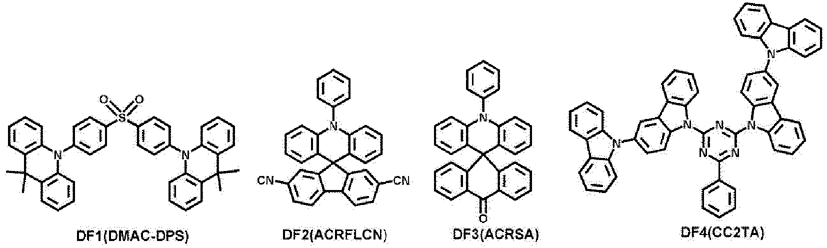

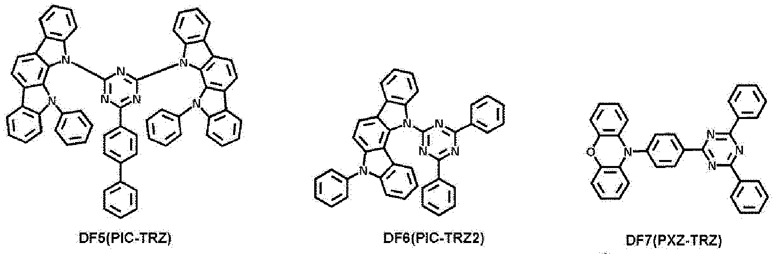

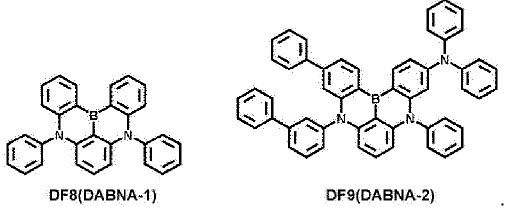

### Quantum dot

The emission layer may include a quantum dot.

The term "quantum dot" as used herein refers to a crystal of a semiconductor compound, and may include any suitable material capable of emitting light of various suitable emission wavelengths according to the size of the crystal.

A diameter of the quantum dot may be, for example, in a range of about 1 nm to about 10 nm.

The quantum dot may be synthesized by a wet chemical process, a metal organic chemical vapor deposition process, a molecular beam epitaxy process, and/or any suitable process similar thereto.

The wet chemical process is a method including mixing a precursor material together with an organic solvent and then growing quantum dot particle crystals. When the crystal grows, the organic solvent naturally acts as a dispersant coordinated on the surface of the quantum dot crystal and controls the growth of the crystal so that the growth of quantum dot particles can be controlled through a process which costs lower, and is easier than vapor deposition methods, such as metal organic chemical vapor deposition (MOCVD) or molecular beam epitaxy (MBE).

The quantum dot may include: a Group II-VI semiconductor compound; a Group III-V semiconductor compound; a Group III-VI semiconductor compound; a Group I-III-VI semiconductor compound; a Group IV-VI semiconductor compound; a Group IV element or compound; or any combination thereof.

Examples of the Group II-VI semiconductor compound are: a binary compound, such as CdS, CdSe, CdTe, ZnS, ZnSe, ZnTe, ZnO, HgS, HgSe, HgTe, MgSe, MgS, and the like; a ternary compound, such as CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and the like; a quaternary compound, such as CdZnSeS, CdZnSeTe, CdZnSTe, CdHgSeS, CdHgSeTe, CdHgSTe, HgZnSeS, HgZnSeTe, HgZnSTe, and the like; or any combination thereof.

Examples of the Group III-V semiconductor compound are: a binary compound, such as GaN, GaP, GaAs, GaSb, AIN, AlP, AlAs, AlSb, InN, InP, InAs, InSb, and/or the like; a ternary compound, such as GaNP, GaNAs, GaNSb, GaPAs, GaPSb, AINP, AlNAs, AlNSb, AlPAs, AlPSb, InGaP, InNP, InAlP, InNAs, InNSb, InPAs, InPSb, and/or the like; a quaternary compound, such as GaAlNP, GaAINAs, GaAINSb, GaAlPAs, GaAlPSb, GaInNP, GaInNAs, GaInNSb, GaInPAs, GaInPSb, InAlNP, InAlNAs, InAlNSb, InAlPAs, InAlPSb, and/or the like; or any combination thereof. In an embodiment, the Group III-V semiconductor compound may further include a Group II element. Examples of the Group III-V semiconductor compound further including the Group II element are InZnP, InGaZnP, InAlZnP, and the like.

Examples of the Group III-VI semiconductor compound are: a binary compound, such as GaS, GaSe, Ga₂Se₃, GaTe, InS, InSe, In₂S₃, In₂Se₃, InTe, and the like; a ternary compound, such as InGaS₃, InGaSe₃, and the like; or any combination thereof.

Examples of the Group I-III-VI semiconductor compound are: a ternary compound, such as AgInS, AgInS₂, CuInS, CuInS₂, CuGaO₂, AgGaO₂, AgAlO₂, and the like; or any combination thereof.

Examples of the Group IV-VI semiconductor compound are: a binary compound, such as SnS, SnSe, SnTe, PbS, PbSe, PbTe, and the like; a ternary compound, such as SnSeS, SnSeTe, SnSTe, PbSeS, PbSeTe, PbSTe, SnPbS, SnPbSe, SnPbTe, and the like; a quaternary compound, such as SnPbSSe, SnPbSeTe, SnPbSTe, and the like; or any combination thereof.

Examples of the Group IV element or compound are: a single element compound, such as Si, Ge, and the like; a binary compound, such as SiC, SiGe, and the like; or any combination thereof.

Each element included in a multi-element compound, such as the binary compound, the ternary compound, and the quaternary compound, may be present at a uniform concentration or non-uniform concentration in a particle.

In an embodiment, the quantum dot may have a single structure in which the concentration of each element in the quantum dot is uniform (e.g., substantially uniform), or may have a core-shell dual structure. For example, a material included in the core and a material included in the shell may be different from each other.

The shell of the quantum dot may act as a protective layer which prevents or reduces chemical denaturation of the core to maintain semiconductor characteristics, and/or as a charging layer which impart electrophoretic characteristics to the quantum dot. The shell may be single-layered or multi-layered. The interface between the core and the shell may have a concentration gradient in which the concentration of an element existing in the shell decreases along a direction toward the center of the core.

Examples of the shell of the quantum dot are an oxide of metal, metalloid, and/or non-metal, a semiconductor compound, or a combination thereof. Examples of the oxide of metal, metalloid, and/or non-metal are: a binary compound, such as SiO₂, Al₂O₃, TiO₂, ZnO, MnO, Mn₂O₃, Mn₃O₄, CuO, FeO, Fe₂O₃, Fe₃O₄, CoO, Co₃O₄, NiO, and the like; a ternary compound, such as MgAl₂O₄, CoFe₂O₄, NiFe₂O₄, CoMn₂O₄, and the like; or any combination thereof. Examples of the semiconductor compound are: as described herein, a Group II-VI semiconductor compound; a Group III-V semiconductor compound; a Group III-VI semiconductor compound; a Group I-III-VI semiconductor compound; a Group IV-VI semiconductor compound; or any combination thereof. Examples of the semiconductor compound are CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, or any combination thereof.

The quantum dot may have an FWHM of the emission wavelength spectrum of less than or equal to about 45 nm, less than or equal to about 40 nm, or for example, less than or equal to about 30 nm. When the FWHM of the quantum dot is within these ranges, the quantum dot may have improved color purity and/or improved color reproducibility. In addition, because light emitted through the quantum dot is emitted in all (e.g., substantially all) directions, the wide viewing angle may be improved.

In addition, the quantum dot may be in the form of spherical, pyramidal, multi-arm, and/or cubic nanoparticles, nanotubes, nanowires, nanofibers, and/or nanoplate particles.

Because the energy band gap may be adjusted by controlling the size of the quantum dot, light having various suitable wavelength bands may be obtained from the quantum dot emission layer. Accordingly, by using quantum dots of different sizes, a light-emitting device that emits light of various suitable wavelengths may be implemented. In an embodiment, the size of the quantum dots may be selected to emit red light, green light, and/or blue light. In addition, the size of the quantum dots may be configured to emit white light by combination of light of various suitable colors.

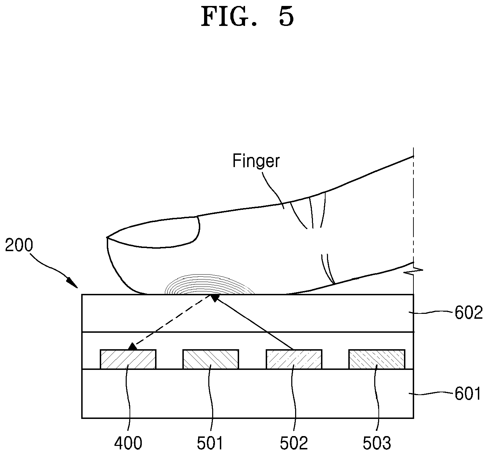

### Descriptions of FIGS. 5-6

FIG. 5 is a view of the electronic apparatus 200 according to an embodiment.

The electronic apparatus 200 of FIG. 5 includes the organic photodiode 400 and the organic light-emitting devices 501, 502, and 503 that are arranged between two substrates 601 and 602.

For example, red light, green light, and blue light may be emitted from the organic light-emitting device 501, the organic light-emitting device 502, and the organic light-emitting device 503, respectively.

The electronic apparatus 200 according to an embodiment may have a function of detecting prints of an object, e.g., a finger, that is in contact with the electronic apparatus. For example, as illustrated in FIG. 5, at least a part of reflective light reflected from the fingerprint of a user among light emitted from the organic light-emitting device 502 may be incident to the organic photodiode 400, and accordingly, the organic photodiode 400 may detect the reflected light. Because ridges of the fingerprint pattern are in close contact to the substrate 602, the organic photodiode 400 may be able to selectively acquire a fingerprint pattern of a user, for example, image information of the ridges. FIG. 5 shows an example of acquiring the information of an object that is in contact with the electronic apparatus 200 by using the light emitted from the organic light-emitting device 502, but the information may be acquired in substantially the same manner by using light emitted from the organic light-emitting device 501 and/or 503.

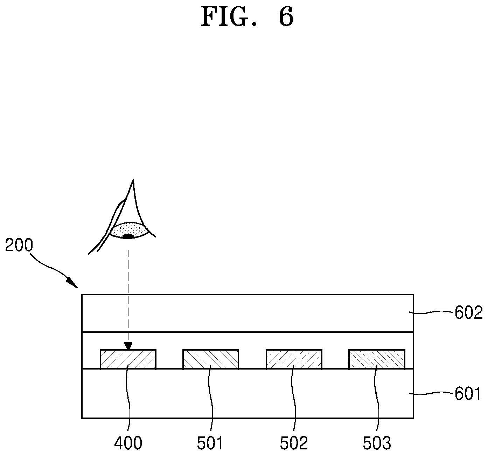

FIG. 6 is a view of the electronic apparatus 200 according to an embodiment.

As shown in FIG. 6, the electronic apparatus 200 according to an embodiment may be able to detect an object that is not contact with the electronic apparatus 200.

### Manufacturing method

The layers included in the hole transport region, the activation layer, and the layers included in the electron transport region may be formed in certain regions by using one or more suitable methods selected from vacuum deposition, spin coating, casting, Langmuir-Blodgett (LB) deposition, ink-jet printing, laser-printing, and laser-induced thermal imaging (LITI).

When the layers of the hole transport region, the activation layer, and the layers of the electron transport region are formed by vacuum deposition, deposition conditions may be selected from within a deposition temperature of about 100 °C to about 500 °C, a vacuum degree of about 10⁻⁸ torr to about 10⁻³ torr, and a deposition rate of about 0.01 Å/sec to about 100 Å/sec, in consideration of the material and structure of a layer to be formed. The deposition rate of the photoactive layer may be from about 1.5 Å/sec to about 3.0 Å/sec.

### Definition of terms

The term "C₃-C₆₀ carbocyclic group" as used herein refers to a cyclic group consisting of carbon only as a ring-forming atom and having three to sixty carbon atoms, and the term "C₁-C₆₀ heterocyclic group" as used herein refers to a cyclic group that has one to sixty carbon atoms and further has, in addition to carbon, a heteroatom as a ring-forming atom. The C₃-C₆₀ carbocyclic group and the C₁-C₆₀ heterocyclic group may each be a monocyclic group consisting of one ring or a polycyclic group in which two or more rings are condensed together with each other. For example, the number of ring-forming atoms of the C₁-C₆₀ heterocyclic group may be from 3 to 61.

The term "cyclic group" as used herein may include both the C₃-C₆₀ carbocyclic group and the C₁-C₆₀ heterocyclic group.

The term "π electron-rich C₃-C₆₀ cyclic group" as used herein refers to a cyclic group that has three to sixty carbon atoms and does not include *-N=*' as a ring-forming moiety, and the term "π electron-deficient nitrogen-containing C₁-C₆₀ cyclic group" as used herein refers to a heterocyclic group that has one to sixty carbon atoms and includes *-N=*' as a ring-forming moiety.

For example, the C₃-C₆₀ carbocyclic group may be i) a T1 group or ii) a condensed cyclic group in which two or more T1 groups are condensed together with each other (for example, a cyclopentadiene group, an adamantane group, a norbornane group, a benzene group, a pentalene group, a naphthalene group, an azulene group, an indacene group, an acenaphthylene group, a phenalene group, a phenanthrene group, an anthracene group, a fluoranthene group, a triphenylene group, a pyrene group, a chrysene group, a perylene group, a pentaphene group, a heptalene group, a naphthacene group, a picene group, a hexacene group, a pentacene group, a rubicene group, a coronene group, an ovalene group, an indene group, a fluorene group, a spiro-bifluorene group, a benzofluorene group, an indenophenanthrene group, or an indenoanthracene group),
the C₁-C₆₀ heterocyclic group may be i) a T2 group, ii) a condensed cyclic group in which at least two T2 groups are condensed together with each other, or iii) a condensed cyclic group in which at least one T2 group and at least one T1 group are condensed together with each other (for example, a pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, or the like),
the π electron-rich C₃-C₆₀ cyclic group may be i) a T1 group, ii) a condensed cyclic group in which at least two T1 groups are condensed together with each other, iii) a T3 group, iv) a condensed cyclic group in which at least two T3 groups are condensed together with each other, or v) a condensed cyclic group in which at least one T3 group and at least one T1 group are condensed together with each other (for example, the C₃-C₆₀ carbocyclic group, a 1H-pyrrole group, a silole group, a borole group, a 2H-pyrrole group, a 3H-pyrrole group, a thiophene group, a furan group, an indole group, a benzoindole group, a naphthoindole group, an isoindole group, a benzoisoindole group, a naphthoisoindole group, a benzosilole group, a benzothiophene group, a benzofuran group, a carbazole group, a dibenzosilole group, a dibenzothiophene group, a dibenzofuran group, an indenocarbazole group, an indolocarbazole group, a benzofurocarbazole group, a benzothienocarbazole group, a benzosilolocarbazole group, a benzoindolocarbazole group, a benzocarbazole group, a benzonaphthofuran group, a benzonaphthothiophene group, a benzonaphthosilole group, a benzofurodibenzofuran group, a benzofurodibenzothiophene group, a benzothienodibenzothiophene group, or the like), and
the π electron-deficient nitrogen-containing C₁-C₆₀ cyclic group may be i) a T4 group, ii) a condensed cyclic group in which at least two T4 groups are condensed together with each other, iii) a condensed cyclic group in which at least one T4 group and at least one T1 group are condensed together with each other, iv) a condensed cyclic group in which at least one T4 group and at least one T3 group are condensed together with each other, or v) a condensed cyclic group in which at least one T4 group, at least one T1 group, and at least one T3 group are condensed together with one another (for example, a pyrazole group, an imidazole group, a triazole group, an oxazole group, an isoxazole group, an oxadiazole group, a thiazole group, an isothiazole group, a thiadiazole group, a benzopyrazole group, a benzimidazole group, a benzoxazole group, a benzoisoxazole group, a benzothiazole group, a benzoisothiazole group, a pyridine group, a pyrimidine group, a pyrazine group, a pyridazine group, a triazine group, a quinoline group, an isoquinoline group, a benzoquinoline group, a benzoisoquinoline group, a quinoxaline group, a benzoquinoxaline group, a quinazoline group, a benzoquinazoline group, a phenanthroline group, a cinnoline group, a phthalazine group, a naphthyridine group, an imidazopyridine group, an imidazopyrimidine group, an imidazotriazine group, an imidazopyrazine group, an imidazopyridazine group, an azacarbazole group, an azafluorene group, an azadibenzosilole group, an azadibenzothiophene group, an azadibenzofuran group, and the like),
the T1 group may be a cyclopropane group, a cyclobutane group, a cyclopentane group, a cyclohexane group, a cycloheptane group, a cyclooctane group, a cyclobutene group, a cyclopentene group, a cyclopentadiene group, a cyclohexene group, a cyclohexadiene group, a cycloheptene group, an adamantane group, a norbornane (or bicyclo[2.2.1]heptane) group, a norbornene group, a bicyclo[1.1.1]pentane group, a bicyclo[2.1.1]hexane group, a bicyclo[2.2.2]octane group, or a benzene group,
the T2 group may be a furan group, a thiophene group, a 1H-pyrrole group, a silole group, a borole group, a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, a tetrazine group, a pyrrolidine group, an imidazolidine group, a dihydropyrrole group, a piperidine group, a tetrahydropyridine group, a dihydropyridine group, a hexahydropyrimidine group, a tetrahydropyrimidine group, a dihydropyrimidine group, a piperazine group, a tetrahydropyrazine group, a dihydropyrazine group, a tetrahydropyridazine group, or a dihydropyridazine group,
the T3 group may be a furan group, a thiophene group, a 1H-pyrrole group, a silole group, or a borole group, and

The T4 group may be a 2H-pyrrole group, a 3H-pyrrole group, an imidazole group, a pyrazole group, a triazole group, a tetrazole group, an oxazole group, an isoxazole group, an oxadiazole group, a thiazole group, an isothiazole group, a thiadiazole group, an azasilole group, an azaborole group, a pyridine group, a pyrimidine group, a pyrazine group, a pyridazine group, a triazine group, or a tetrazine group.

The terms "the cyclic group, the C₃-C₆₀ carbocyclic group, the C₁-C₆₀ heterocyclic group, the π electron-rich C₃-C₆₀ cyclic group, or the π electron-deficient nitrogen-containing C₁-C₆₀ cyclic group" as used herein refer to a group condensed to any cyclic group, a monovalent group, or a polyvalent group (for example, a divalent group, a trivalent group, a tetravalent group, etc.) according to the structure of a formula for which the corresponding term is used. In an embodiment, "a benzene group" may be a benzo group, a phenyl group, a phenylene group, or the like, which may be easily understand by one of ordinary skill in the art according to the structure of a formula including the "benzene group."

Examples of the monovalent C₃-C₆₀ carbocyclic group and the monovalent C₁-C₆₀ heterocyclic group are a C₃-C₁₀ cycloalkyl group, a C₁-C₁₀ heterocycloalkyl group, a C₃-C₁₀ cycloalkenyl group, a C₁-C₁₀ heterocycloalkenyl group, a C₆-C₆₀ aryl group, a C₁-C₆₀ heteroaryl group, a monovalent non-aromatic condensed polycyclic group, and a monovalent non-aromatic condensed heteropolycyclic group. Examples of the divalent C₃-C₆₀ carbocyclic group and the monovalent C₁-C₆₀ heterocyclic group are a C₃-C₁₀ cycloalkylene group, a C₁-C₁₀ heterocycloalkylene group, a C₃-C₁₀ cycloalkenylene group, a C₁-C₁₀ heterocycloalkenylene group, a C₆-C₆₀ arylene group, a C₁-C₆₀ heteroarylene group, a divalent non-aromatic condensed polycyclic group, and a substituted or unsubstituted divalent non-aromatic condensed heteropolycyclic group.

The term "C₁-C₆₀ alkyl group" as used herein refers to a linear or branched aliphatic hydrocarbon monovalent group that has 1 to 60 carbon atoms, and examples thereof are a methyl group, an ethyl group, an n-propyl group, an isopropyl group, an n-butyl group, a sec-butyl group, an isobutyl group, a tert-butyl group, an n-pentyl group, a tert-pentyl group, a neopentyl group, an isopentyl group, a sec-pentyl group, a 3-pentyl group, a sec-isopentyl group, an n-hexyl group, an isohexyl group, a sec-hexyl group, a tert-hexyl group, an n-heptyl group, an isoheptyl group, a sec-heptyl group, a tert-heptyl group, an n-octyl group, an isooctyl group, a sec-octyl group, a tert-octyl group, an n-nonyl group, an isononyl group, a sec-nonyl group, a tert-nonyl group, an n-decyl group, an isodecyl group, a sec-decyl group, and a tert-decyl group. The term "C₁-C₆₀ alkylene group" as used herein refers to a divalent group having substantially the same structure as the C₁-C₆₀ alkyl group. Corresponding definitions apply to other ranges given for the number of carbon atoms in an alkyl/alkylene group.

The term "C₂-C₆₀ alkenyl group" as used herein refers to a monovalent hydrocarbon group having at least one carbon-carbon double bond in the middle or at the terminus of the C₂-C₆₀ alkyl group, and examples thereof are an ethenyl group, a propenyl group, a butenyl group, and the like. The term "C₂-C₆₀ alkenylene group" as used herein refers to a divalent group having substantially the same structure as the C₂-C₆₀ alkenyl group. Corresponding definitions apply to other ranges given for the number of carbon atoms in an alkenyl/alkenylene group.

The term "C₂-C₆₀ alkynyl group" as used herein refers to a monovalent hydrocarbon group having at least one carbon-carbon triple bond in the middle or at the terminus of the C₂-C₆₀ alkyl group, and examples thereof are an ethynyl group, a propynyl group, and the like. The term "C₂-C₆₀ alkynylene group" as used herein refers to a divalent group having substantially the same structure as the C₂-C₆₀ alkynyl group. Corresponding definitions apply to other ranges given for the number of carbon atoms in an alkynyl/alkynylene group.

The term "C₁-C₆₀ alkoxy group" as used herein refers to a monovalent group represented by -OA₁₀₁ (wherein A₁₀₁ is the C₁-C₆₀ alkyl group), and examples thereof are a methoxy group, an ethoxy group, an isopropyloxy group, and the like. Corresponding definitions apply to other ranges given for the number of carbon atoms in an alkoxy group.

The term "C₃-C₁₀ cycloalkyl group" as used herein refers to a monovalent saturated hydrocarbon cyclic group having 3 to 10 carbon atoms, and examples thereof are a cyclopropyl group, a cyclobutyl group, a cyclopentyl group, a cyclohexyl group, a cycloheptyl group, a cyclooctyl group, an adamantanyl group, a norbornanyl group (or bicyclo[2.2.1]heptyl group), a bicyclo[1.1.1]pentyl group, a bicyclo[2.1.1]hexyl group, a bicyclo[2.2.2]octyl group, and the like. The term "C₃-C₁₀ cycloalkylene group" as used herein refers to a divalent group having substantially the same structure as the C₃-C₁₀ cycloalkyl group.

The term "C₁-C₁₀ heterocycloalkyl group" as used herein refers to a monovalent cyclic group of 1 to 10 carbon atoms, further including, in addition to carbon atoms, at least one heteroatom, as ring-forming atoms, and examples thereof are a 1,2,3,4-oxatriazolidinyl group, a tetrahydrofuranyl group, a tetrahydrothiophenyl group, and the like. The term "C₁-C₁₀ heterocycloalkylene group" as used herein refers to a divalent group having substantially the same structure as the C₁-C₁₀ heterocycloalkyl group.

The term "C₃-C₁₀ cycloalkenyl group" as used herein refers to a monovalent cyclic group that has three to ten carbon atoms and at least one carbon-carbon double bond in the ring thereof and no aromaticity (e.g., is not aromatic), and examples thereof are a cyclopentenyl group, a cyclohexenyl group, a cycloheptenyl group, and the like. The term "C₃-C₁₀ cycloalkenylene group" as used herein refers to a divalent group having substantially the same structure as the C₃-C₁₀ cycloalkenyl group.

The term "C₁-C₁₀ heterocycloalkenyl group" as used herein refers to a monovalent cyclic group of 1 to 10 carbon atoms, further including, in addition to carbon atoms, at least one heteroatom, as ring-forming atoms, and having at least one carbon-carbon double bond in the cyclic structure thereof. Examples of the C₁-C₁₀ heterocycloalkenyl group are a 4,5-dihydro-1,2,3,4-oxatriazolyl group, a 2,3-dihydrofuranyl group, a 2,3-dihydrothiophenyl group, and the like. The term "C₁-C₁₀ heterocycloalkenylene group" as used herein refers to a divalent group having substantially the same structure as the C₁-C₁₀ heterocycloalkenyl group.

The term "C₆-C₆₀ aryl group" as used herein refers to a monovalent group having a carbocyclic aromatic system of 6 to 60 carbon atoms, and the term "C₆-C₆₀ arylene group" as used herein refers to a divalent group having a carbocyclic aromatic system of 6 to 60 carbon atoms. Examples of the C₆-C₆₀ aryl group are a phenyl group, a pentalenyl group, a naphthyl group, an azulenyl group, an indacenyl group, an acenaphthyl group, a phenalenyl group, a phenanthrenyl group, an anthracenyl group, a fluoranthenyl group, a triphenylenyl group, a pyrenyl group, a chrysenyl group, a perylenyl group, a pentaphenyl group, a heptalenyl group, a naphthacenyl group, a picenyl group, a hexacenyl group, a pentacenyl group, a rubicenyl group, a coronenyl group, an ovalenyl group, and the like. When the C₆-C₆₀ aryl group and the C₆-C₆₀ arylene group each include two or more rings, the rings may be condensed together with each other. Corresponding definitions apply to other ranges given for the number of carbon atoms in an aryl/arylene group.

The term "C₁-C₆₀ heteroaryl group" as used herein refers to a monovalent group having a heterocyclic aromatic system of 1 to 60 carbon atoms, further including, in addition to carbon atoms, at least one heteroatom, as ring-forming atoms. The term "C₁-C₆₀ heteroarylene group" as used herein refers to a divalent group having a heterocyclic aromatic system of 1 to 60 carbon atoms, further including, in addition to carbon atoms, at least one heteroatom, as ring-forming atoms. Examples of the C₁-C₆₀ heteroaryl group are a pyridinyl group, a pyrimidinyl group, a pyrazinyl group, a pyridazinyl group, a triazinyl group, a quinolinyl group, a benzoquinolinyl group, an isoquinolinyl group, a benzoisoquinolinyl group, a quinoxalinyl group, a benzoquinoxalinyl group, a quinazolinyl group, a benzoquinazolinyl group, a cinnolinyl group, a phenanthrolinyl group, a phthalazinyl group, and a naphthyridinyl group. When the C₁-C₆₀ heteroaryl group and the C₁-C₆₀ heteroarylene group each include two or more rings, the rings may be condensed together with each other. Corresponding definitions apply to other ranges given for the number of carbon atoms in a heteroaryl/heteroarylene group.

The term "monovalent non-aromatic condensed polycyclic group" as used herein refers to a monovalent group (for example, having 8 to 60 carbon atoms) having two or more rings condensed to each other, only carbon atoms as ring-forming atoms, and no aromaticity in its entire molecular structure (e.g., is not aromatic when considered as a whole). Examples of the monovalent non-aromatic condensed polycyclic group are an indenyl group, a fluorenyl group, a spiro-bifluorenyl group, a benzofluorenyl group, an indenophenanthrenyl group, an indeno anthracenyl group, and the like. The term "divalent non-aromatic condensed polycyclic group" as used herein refers to a divalent group having substantially the same structure as the monovalent non-aromatic condensed polycyclic group described above.

The term "monovalent non-aromatic condensed heteropolycyclic group" as used herein refers to a monovalent group (for example, having 1 to 60 carbon atoms) having two or more rings condensed to each other, further including, in addition to carbon atoms, at least one heteroatom, as ring-forming atoms, and having non-aromaticity in its entire molecular structure (e.g., is not aromatic when considered as a whole). Examples of the monovalent non-aromatic condensed heteropolycyclic group are a pyrrolyl group, a thiophenyl group, a furanyl group, an indolyl group, a benzoindolyl group, a naphthoindolyl group, an isoindolyl group, a benzoisoindolyl group, a naphthoisoindolyl group, a benzosilolyl group, a benzothiophenyl group, a benzofuranyl group, a carbazolyl group, a dibenzosilolyl group, a dibenzothiophenyl group, a dibenzofuranyl group, an azacarbazolyl group, an azafluorenyl group, an azadibenzosilolyl group, an azadibenzothiophenyl group, an azadibenzofuranyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, a thiadiazolyl group, a benzopyrazolyl group, a benzimidazolyl group, a benzoxazolyl group, a benzothiazolyl group, a benzoxadiazolyl group, a benzothiadiazolyl group, an imidazopyridinyl group, an imidazopyrimidinyl group, an imidazotriazinyl group, an imidazopyrazinyl group, an imidazopyridazinyl group, an indeno carbazolyl group, an indolocarbazolyl group, a benzofurocarbazolyl group, a benzothienocarbazolyl group, a benzosilolocarbazolyl group, a benzoindolocarbazolyl group, a benzocarbazolyl group, a benzonaphthofuranyl group, a benzonaphthothiophenyl group, a benzonaphthosilolyl group, a benzofurodibenzofuranyl group, a benzofurodibenzothiophenyl group, and a benzothienodibenzothiophenyl group. The term "divalent non-aromatic condensed heteropolycyclic group" as used herein refers to a divalent group having substantially the same structure as the monovalent non-aromatic condensed heteropolycyclic group described above.

The term "C₆-C₆₀ aryloxy group" as used herein indicates -OA₁₀₂ (wherein A₁₀₂ is the C₆-C₆₀ aryl group), and the term "C₆-C₆₀ arylthio group" as used herein indicates -SA₁₀₃ (wherein A₁₀₃ is the C₆-C₆₀ aryl group). Corresponding definitions apply to other ranges given for the number of carbon atoms in an aryloxy group and an arylthio group.

The term "C₇-C₆₀ arylalkyl group" as used herein refers to -A₁₀₄A₁₀₅ (wherein A₁₀₄ is a C₁-C₅₄ alkylene group, and A₁₀₅ is a C₆-C₅₉ aryl group), and the term "C₂-C₆₀ heteroarylalkyl group" as used herein refers to -A₁₀₆A₁₀₇ (wherein A₁₀₆ is a C₁-C₅₉ alkylene group, and A₁₀₇ is a C₁-C₅₉ heteroaryl group).

The term "R₁₀ₐ" as used herein may be:
deuterium, -F, -Cl, -Br, -I, a hydroxyl group, a cyano group, or a nitro group;
a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, or a C₁-C₆₀ (*e.g.* C₁-C₂₀) alkoxy group, each unsubstituted or substituted with deuterium, -F, -Cl, -Br, -I, a hydroxyl group, a cyano group, a nitro group, a C₃-C₆₀ (*e.g.* C₃-C₃₀) carbocyclic group, a C₁-C₆₀ (*e.g.* C₁-C₂₀) heterocyclic group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryloxy group, a C₁-C₆₀ (*e.g.* C₁-C₂₀) heteroarylthio group, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁),-P(=O)(Q₁₁)(Q₁₂), or any combination thereof;
a C₃-C₆₀ (*e.g.* C₃-C₃₀) carbocyclic group, a C₁-C₆₀ (*e.g.* C₁-C₂₀) heterocyclic group, a C₆-C₆₀ (e*.g.* C₆-C₃₀) aryloxy group, a C₆-C₆₀ (*e.g.* C₆-C₃₀) arylthio group, a C₁-C₆₀ (*e.g.* C₁-C₂₀) heteroaryloxy group, or a C₁-C₆₀ (*e.g.* C₁-C₂₀) heteroarylthio group, each unsubstituted or substituted with deuterium, -F, -Cl, -Br, -I, a hydroxyl group, a cyano group, a nitro group, a C₁-C₆₀ (*e.g.* C₁-C₂₀) alkyl group, a C₂-C₆₀ (*e.g.* C₂-C₂₀) alkenyl group, a C₂-C₆₀ (*e.g.* C₂-C₂₀) alkynyl group, a C₁-C₆₀ (*e.g.* C₁-C₂₀) alkoxy group, a C₃-C₆₀ (*e.g.* C₃-C₃₀) carbocyclic group, a C₁-C₆₀ (*e.g.* C₁-C₂₀) heterocyclic group, a C₆-C₆₀ (*e.g.* C₆-C₃₀) aryloxy group, a C₆-C₆₀ (*e.g.* C₆-C₃₀) arylthio group, a C₁-C₆₀ (*e.g.* C₁-C₂₀) heteroaryloxy group, a C₁-C₆₀ (*e.g.* C₁-C₂₀) heteroarylthio group, - Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁),-P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or-P(=O)(Q₃₁)(Q₃₂).

In the specification, Q₁₁ to Q₁₃, Q₂₁ to Q₂₃, and Q₃₁ to Q₃₃ may each independently be: hydrogen; deuterium; -F; -Cl; -Br; -I; a hydroxyl group; a cyano group; a nitro group; a C₁-C₆₀ (*e.g.* C₁-C₂₀) alkyl group; a C₂-C₆₀ (*e.g.* C₂-C₂₀) alkenyl group; a C₂-C₆₀ (*e.g.* C₂-C₂₀) alkynyl group; a C₁-C₆₀ (*e.g.* C₁-C₂₀) alkoxy group; a C₃-C₆₀ (*e.g.* C₃-C₃₀) carbocyclic group or a C₁-C₆₀ (*e.g.* C₁-C₂₀) heterocyclic group, each unsubstituted or substituted with deuterium, -F, a cyano group, a C₁-C₆₀ (*e.g.* C₁-C₂₀) alkyl group, a C₁-C₆₀ (*e.g.* C₁-C₂₀) alkoxy group, a phenyl group, a biphenyl group, or any combination thereof; a C₁-C₆₀ (*e.g.* C₁-C₂₀) heteroaryloxy group; or a C₁-C₆₀ (*e.g.* C₁-C₂₀) heteroarylthio group.

The term "heteroatom" as used herein refers to any atom other than a carbon atom. Examples of the heteroatom are O, S, N, P, Si, B, Ge, Se, and any combination thereof.

In the present specification, the third-row transition metal may include hafnium (Hf), tantalum (Ta), tungsten (W), rhenium (Re), osmium (Os), iridium (Ir), platinum (Pt), gold (Au), and/or the like.

The term "Ph" as used herein refers to a phenyl group, the term "Me" as used herein refers to a methyl group, the term "Et" as used herein refers to an ethyl group, the term "tert-Bu" or "Bu^{t}" as used herein refers to a tert-butyl group, and the term "OMe" as used herein refers to a methoxy group.

The term "biphenyl group" as used herein refers to "a phenyl group substituted with a phenyl group." In other words, the "biphenyl group" is a substituted phenyl group having a C₆-C₆₀ aryl group as a substituent.

The term "terphenyl group" as used herein refers to "a phenyl group substituted with a biphenyl group". In other words, the "terphenyl group" is a substituted phenyl group having, as a substituent, a C₆-C₆₀ aryl group substituted with a C₆-C₆₀ aryl group.
* and *' as used herein, unless defined otherwise, each refer to a binding site to a neighboring atom in a corresponding formula or moiety.

Hereinafter, manufacture of electronic apparatuses according to embodiments of the disclosure and evaluation results thereof will be described with reference to examples.

### Example 1

A display device in which an organic photodiode and red, green, and blue organic light-emitting devices were included using common layers in one pixel was manufactured.

An ITO glass substrate (anode) was cut to a size of 50 mm x 50 mm x 0.5 mm, ultrasonically cleaned with isopropyl alcohol and pure water each for 15 minutes, and then cleaned by irradiation of ultraviolet rays and exposure to ozone for 10 minutes. Then, the ITO substrate was loaded into a vacuum deposition apparatus. Compound HAT-CN was vacuum-deposited on the anode to form a hole injection layer of a thickness of 100 Å, and Compound HT3 was vacuum-deposited on the hole injection layer to form a hole transport layer of a thickness of 1,250 Å.

Compound HT38 was vacuum-deposited on the hole transport layer to form an auxiliary layer for each device. Here, auxiliary layers for a red light-emitting device and an organic photodiode were simultaneously formed to a thickness of 600 Å, an auxiliary layer for a green light-emitting device was formed to a thickness of 350 Å, and an auxiliary layer for a blue light-emitting device was formed to a thickness of 50 Å.

Donor Compound 1 and NTCDA were sequentially deposited at a deposition rate of 1.6 Å/sec on the auxiliary layer of the organic photodiode to a thickness of 50 Å and a thickness of 150 Å, respectively, so as to form a photoactive layer having a total thickness of 200 Å.

Compound CBP and Compound Ir(pq)₂(acac) were co-deposited (at a weight ratio of 90:10) on the auxiliary layer of the red organic light-emitting device to form a red emission layer having a thickness of 450 Å, Compound CBP and Compound Ir(ppy)₃ were co-deposited (at a weight ratio of 90:10) on the auxiliary layer of the green organic light-emitting device to form a green emission layer having a thickness of 400 Å, and Compound CBP and Compound Ir(pmp)₃ were co-deposited (at a weight ratio of 90:10) on the auxiliary layer of the blue organic light-emitting device to form a blue emission layer having a thickness of 200 Å.

Subsequently, BAlq was vacuum-deposited to form a hole blocking layer having a thickness of 50 Å, and ET1 was vacuum-deposited on the hole blocking layer to form an electron transport layer having a thickness of 300 Å.

LiQ and MgAg were sequentially deposited on the electron transport layer to a thickness of 10 Å and a thickness of 100 Å, respectively, so as to manufacture a display device including three organic light-emitting devices and one organic photodiode.

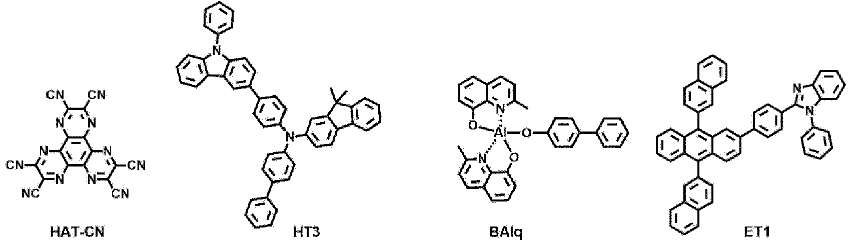

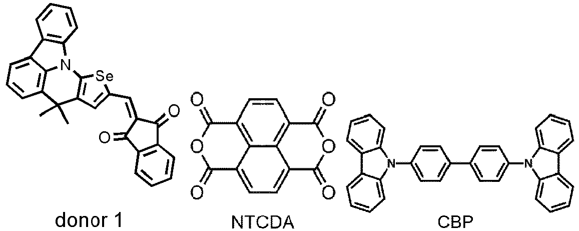

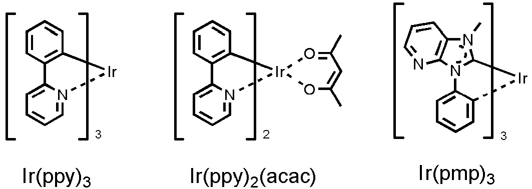

### Example 2

A display device was manufactured in substantially the same manner as in Example 1, except that a photoactive layer of an organic photodiode was formed to a total thickness of 250 Å by sequentially depositing Donor Compound 1 and NTCDA at a deposition rate of 2.0 Å/sec to a thickness of 50 Å and a thickness of 200 Å, respectively.

### Example 3

A display device was manufactured in substantially the same manner as in Example 1, except that a photoactive layer of an organic photodiode was formed to a total thickness of 300 Å by sequentially depositing Donor Compound 1 and NTCDA at a deposition rate of 2.4 Å/sec to a thickness of 50 Å and a thickness of 250 Å, respectively.

### Comparative Example 1

A display device was manufactured in substantially the same manner as in Example 1, except that a photoactive layer of an organic photodiode was formed to a total thickness of 100 Å by sequentially depositing Donor Compound 1 and NTCDA at a deposition rate of 0.8 Å/sec to a thickness of 50 Å and a thickness of 50 Å, respectively.

### Comparative Example 2

A display device was manufactured in substantially the same manner as in Example 1, except that a photoactive layer of an organic photodiode was formed to a total thickness of 150 Å by sequentially depositing Donor Compound 1 and NTCDA at a deposition rate of 1.2 Å/sec to a thickness of 50 Å and a thickness of 100 Å, respectively.

### Comparative Example 3

A display device was manufactured in substantially the same manner as in Example 1, except that a photoactive layer of an organic photodiode was formed to a total thickness of 350 Å by sequentially depositing Donor Compound 1 and NTCDA at a deposition rate of 2.8 Å/sec to a thickness of 50 Å and a thickness of 300 Å, respectively.

### Comparative Example 4

A display device was manufactured in substantially the same manner as in Example 1, except that a photoactive layer of an organic photodiode was formed to a total thickness of 450 Å by sequentially depositing Donor Compound 1 and NTCDA at a deposition rate of 3.6 Å/sec to a thickness of 50 Å and a thickness of 400 Å, respectively.

### Comparative Example 5

A display device was manufactured in substantially the same manner as in Example 1, except that an auxiliary layer for an organic photodiode and an auxiliary layer for a green organic light-emitting device were simultaneously formed to a thickness of 300 Å and that a photoactive layer of the organic photodiode was formed to a total thickness of 500 Å by sequentially depositing Donor Compound 1 and NTCDA at a deposition rate of 4.0 Å/sec to a thickness of 50 Å and a thickness of 450 Å, respectively.

### Evaluation Example

For the organic photodiodes of the display devices of Examples 1 to 3 and Comparative Examples 1 to 5, the light (at a wavelength of 530 nm) was irradiated using an external quantum efficiency (EQE) measurement device (K3100EQX, McScience, Korea), and the current generated by the light irradiation were measured using a current meter (Keithley, Tektronix, USA), and the measured values were calculated as EQE values and listed in Table 1.

**Table 1**

| | Thickness of photoactive layer | Deposition rate of photoactive layer | EQE@530 nm |
|---|---|---|---|
| Example 1 | 200 Å | 1.6 Å/sec | 24.3 % |
| Example 2 | 250 Å | 2.0 Å/sec | 30.5 % |
| Example 3 | 300 Å | 2.4 Å/sec | 21.1 % |
| Comparative Example 1 | 100 Å | 0.8 Å/sec | 1.5 % |
| Comparative Example 2 | 150 Å | 1.2 Å/sec | 8.9 % |
| Comparative Example 3 | 350 Å | 2.8 Å/sec | 8.4 % |
| Comparative Example 4 | 450 Å | 3.6 Å/sec | 0.5 % |
| Comparative Example 5 | 500 Å | 4.0 Å/sec | 7.8 % |

Referring to Table 1, it can be seen that the EQE of the organic photodiodes of Examples 1 to 3 was significantly excellent as compared with the EQE of the organic photodiodes of Comparative Examples 1 to 5.

It was determined that the EQE was reduced in Comparative Examples 1 and 2 because the light absorption was insufficient and the resonance distance was shortened due to the decreased thickness of the photoactive layer, and that the EQE was reduced in Comparative Examples 3 and 4 because the resonance distance was lengthened due to the increased thickness of the photoactive layer and also because the crystallinity was reduced due to the increased deposition rate of the photoactive layer. It was determined that that the EQE was reduced in Comparative Example 5 because, although the resonance distance of the organic photodiode was suitable, the crystallinity of the photoactive layer was reduced due to the increased deposition rate of the photoactive layer and because the charge mobility was decreased.

It was determined that the EQE was improved in Examples 1 to 3 because the resonance distance of the organic photodiode was suitable with respect to the incident light and the photoactive layer had good crystallinity upon deposition, but the present disclosure is not limited by any particular mechanism or theory.

An electronic apparatus according to an aspect of embodiments may improve the quality of a photoactive layer of an organic photodiode by forming a thin film, and may increase an external quantum efficiency of an organic photodiode as well as secure a resonance distance of an organic photodiode by forming an auxiliary layer to the same thickness as an auxiliary layer for an organic light-emitting device.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the scope as defined by the following claims.

## Claims

1. An electronic apparatus (200) comprising:
a substrate (601);
a plurality of organic light-emitting devices (501, 502, 503) and an organic photodiode (400) that are arranged on the substrate (601), wherein:
the plurality of organic light-emitting devices (501, 502, 503) comprise a first light-emitting device (501), a second light-emitting device (502), and a third light-emitting device (503),
the organic photodiode (400) sequentially comprises a first electrode (410), a first auxiliary layer (427), a photoactive layer (430), and a common electrode (450),
the first light-emitting device (501) sequentially comprises a second electrode (511), a second auxiliary layer (521), a first emission layer (531), and a common electrode (450),
the second light-emitting device (502) sequentially comprises a third electrode 512), a third auxiliary layer (522), a second emission layer (532), and a common electrode (450),
the third light-emitting device (503) sequentially comprises a fourth electrode (513), a fourth auxiliary layer (523), a third emission layer (533), and a common electrode (450),
the photoactive layer (430) has a thickness in a range of about 200 Å to about 300 Å,
the second auxiliary layer (521), the third auxiliary layer (522), and the fourth auxiliary layer (523) have different thicknesses from each other, and
a thickness of the first auxiliary layer (427) is identical to a thickness of one selected from the second auxiliary layer (521), the third auxiliary layer (522), and the fourth auxiliary layer (523).

2. The electronic apparatus (200) of claim 1, wherein the first emission layer (531) to third emission layer (533) each have a thickness in a range of about 150 Å to about 500 Å.

3. The electronic apparatus (200) of claim 1 or claim 2, wherein thicknesses of the second auxiliary layer (521), the third auxiliary layer (522), and the fourth auxiliary layer (523) are each proportional to the wavelength of light emitted from each of the first light-emitting device (501), the second light-emitting device (502), and the third light-emitting device (503).

4. The electronic apparatus (200) of any one of claims 1 to 3, wherein:
the first light-emitting device (501) emits red light,
the second light-emitting device (502) emits green light, and
the third light-emitting device (503) emits blue light.

5. The electronic apparatus (200) of claim 4, wherein a resonance distance of the organic photodiode (400) is identical to that of the second light-emitting device (502).

6. The electronic apparatus (200) of claim 4 or claim 5, wherein:
the first auxiliary layer (427) has an identical thickness to a thickness of the second auxiliary layer (521).

7. The electronic apparatus (200) of claim 4 or claim 5, wherein the organic photodiode (400) detects light emitted from the second light-emitting device (502) and reflected from a subject.

8. The electronic apparatus (200) of any one of claims 1 to 7, wherein the photoactive layer (430) comprises a p-type semiconductor and an n-type semiconductor.

9. The electronic apparatus (200) of claim 8, wherein:
(i) the photoactive layer (430) has a planar heterojunction structure of the p-type semiconductor and the n-type semiconductor; and/or
(ii) the photoactive layer (430) has a bulk heterojunction structure of the p-type semiconductor and the n-type semiconductor.

10. The electronic apparatus (200) of claim 8 or claim 9, wherein the p-type semiconductor comprises a donor compound represented by Formula 110: 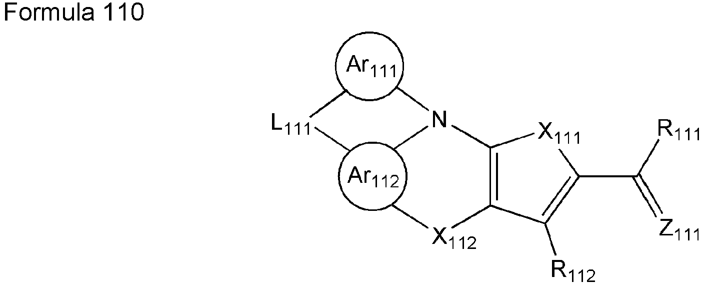 wherein, in Formula 110,
Ar₁₁₁ and Ar₁₁₂ are each independently a C₆-C₃₀ arylene group that is unsubstituted or substituted with at least one R₁₀ₐ or a C₃-C₃₀ heteroarylene group that is unsubstituted or substituted with at least one R₁₀ₐ,
X₁₁₁ is selected from -Se-, -Te-, -S(=O)-, -S(=O)₂-, -N(Q₁₁₁)-, -B(Q₁₁₁)-,-C(Q₁₁₁)(Q₁₁₂)-, -Si(Q₁₁₁)(Q₁₁₂)-, and -Ge(Q₁₁₁)(Q₁₁₂)-,
X₁₁₂ and L₁₁₁ are each independently selected from -O-, -S-, -Se-, -Te-, -S(=O)-, -S(=O)₂-, -N(Q₁₁₁)-, -B(Q₁₁₁)-, -C(Q₁₁₁)(Q₁₁₂)-, -Si(Q₁₁₁)(Q₁₁₂)-, -Ge(Q₁₁₁)(Q₁₁₂)-,-(C(Q₁₁₁)=C(Q₁₁₂))-, and -(C(Q₁₁₁)=N))-,
when L₁₁₁ is selected from -N(Q₁₁₁)-, -B(Q₁₁₁)-, -C(Q₁₁₁)(Q₁₁₂)-, -Si(Q₁₁₁)(Q₁₁₂)-,-Ge(Q₁₁₁)(Q₁₁₂)-, -(C(Q₁₁₁)=C(Q₁₁₂))-, and -(C(Q₁₁₁)=N))-, L₁₁₁ is optionally linked to Ar₁₁₁ or Ar₁₁₂ to form a condensed ring,
Z₁₁₁ is a C₆-C₃₀ carbocyclic group that is unsubstituted or substituted with at least one R₁₀ₐ and has at least one functional group selected from C=O, C=S, C=Se, and C=Te, or a C₁-C₃₀ heterocyclic group that is unsubstituted or substituted with at least one R₁₀ₐ and has at least one functional group selected from C=O, C=S, C=Se, and C=Te,
R₁₁₁ to R₁₁₂ are each independently hydrogen, deuterium, a halogen, a cyano group, a nitro group, a hydroxy group, a C₁-C₃₀ alkyl group unsubstituted or substituted with at least one R₁₀ₐ, a C₁-C₃₀ alkoxy group unsubstituted or substituted with at least one R₁₀ₐ, a C₆-C₃₀ aryl group unsubstituted or substituted with at least one R₁₀ₐ, a C₃-C₃₀ heteroaryl group unsubstituted or substituted with at least one R₁₀ₐ, a C₂-C₃₀ acyl group unsubstituted or substituted with at least one R₁₀ₐ, or any combination thereof,
R₁₀ₐ is:
deuterium, -F, -Cl, -Br, -I, a hydroxyl group, a cyano group, or a nitro group; a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, C₆-C₆₀ aryloxy group, a C₆-C₆₀ arylthio group, a C₁-C₆₀ heteroaryloxy group, a C₁-C₆₀ heteroarylthio group, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₁-C₆₀ heteroaryloxy group, or a C₁-C₆₀ heteroarylthio group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₁-C₆₀ heteroaryloxy group, a C₁-C₆₀ heteroarylthio group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or-P(=O)(Q₃₁)(Q₃₂), and
Q₁₁ to Q₁₃, Q₂₁ to Q₂₃, Q₃₁ to Q₃₃, Q₁₁₁, and Q₁₁₂ are each independently: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

11. The electronic apparatus (200) of any one of claims 8 to 10, wherein the n-type semiconductor comprises a compound represented by Formula 120: 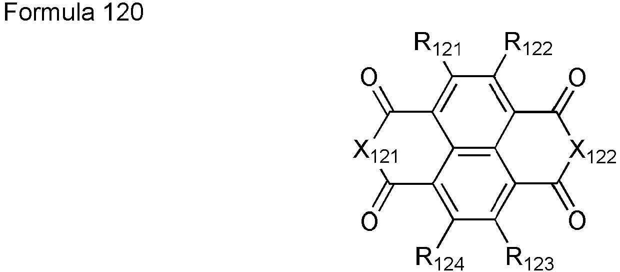 wherein, in Formula 120,
X₁₂₁ and X₁₂₂ are each independently O or NR₁₂₅,
R₁₂₁ to R₁₂₄ and R₁₂₅ are each independently hydrogen, deuterium, a halogen, a cyano group, a nitro group, a hydroxy group, a C₁-C₃₀ alkyl group substituted or unsubstituted with at least one R₁₀ₐ, a C₆-C₃₀ aryl group substituted or unsubstituted with at least one R₁₀ₐ, a C₃-C₃₀ heteroaryl group substituted or unsubstituted with at least one R₁₀ₐ, or any combination thereof, and
R₁₀ₐ is:
deuterium, -F, -Cl, -Br, -I, a hydroxyl group, a cyano group, or a nitro group; a C₁-C₆₀ alkyl group, a C₂-C₆₀ alkenyl group, a C₂-C₆₀ alkynyl group, or a C₁-C₆₀ alkoxy group, each unsubstituted or substituted with deuterium, -F, -Cl, -Br, -I, a hydroxyl group, a cyano group, a nitro group, a C₃-C₆₀ carbocyclic group, a C₁-C₆₀ heterocyclic group, C₆-C₆₀ aryloxy group, a C₆-C₆₀ arylthio group, a C₁-C₆₀ heteroaryloxy group, a C₁-C₆₀ heteroarylthio group, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), -P(=O)(Q₁₁)(Q₁₂), or any combination thereof;
a C₃-C₆₀ carbocyclic group, a C₁-C₆₀ heterocyclic group, a C₆-C₆₀ aryloxy group, a C₆-C₆₀ arylthio group, a C₁-C₆₀ heteroaryloxy group, or a C₁-C₆₀ heteroarylthio group, each unsubstituted or substituted with deuterium, -F, -Cl, -Br, -I, a hydroxyl group, a cyano group, a nitro group, a C₁-C₆₀ alkyl group, a C₂-C₆₀ alkenyl group, a C₂-C₆₀ alkynyl group, a C₁-C₆₀ alkoxy group, a C₃-C₆₀ carbocyclic group, a C₁-C₆₀ heterocyclic group, a C₆-C₆₀ aryloxy group, a C₆-C₆₀ arylthio group, a C₁-C₆₀ heteroaryloxy group, a C₁-C₆₀ heteroarylthio group, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂), or any combination thereof; or
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), or-P(=O)(Q₃₁)(Q₃₂), and
Q₁₁ to Q₁₃, Q₂₁ to Q₂₃, and Q₃₁, Q₃₂ are each independently: hydrogen; deuterium; -F; -Cl; -Br; -I; a hydroxyl group; a cyano group; a nitro group; a C₁-C₆₀ alkyl group; a C₂-C₆₀ alkenyl group; a C₂-C₆₀ alkynyl group; a C₁-C₆₀ alkoxy group; or a C₃-C₆₀ carbocyclic group or a C₁-C₆₀ heterocyclic group, each unsubstituted or substituted with deuterium, -F, a cyano group, a C₁-C₆₀ alkyl group, a C₁-C₆₀ alkoxy group, a phenyl group, a biphenyl group, or any combination thereof.

12. The electronic apparatus (200) of any one of claims 1 to 11, wherein:
(i) a first common layer (420) is included between the first electrode (410) and the first auxiliary layer (427), between the second electrode (511) and the second auxiliary layer (521), between the third electrode (512) and the third auxiliary layer (522), and between the fourth electrode (513) and the fourth auxiliary layer (523); and/or
(ii) a second common layer (440) is included between the photoactive layer (430) and the common electrode (450), between the first emission layer (531) and the common electrode (450), between the second emission layer (532) and the common electrode (450), and between the third emission layer (533) and the common electrode (450).

13. The electronic apparatus (200) of any one of claims 10 to 12, wherein the first common layer (420) comprises at least one selected from a hole injection layer, a hole transport layer, and an electron blocking layer.

14. The electronic apparatus of any one of claims 11 to 13, wherein the second common layer (440) comprises at least one selected from a buffer layer, a hole blocking layer, an electron transport layer, and an electron injection layer.

15. The electronic apparatus (200) of any one of claims 1 to 14, wherein:
(i) the first auxiliary layer (427) is in direct contact with the photoactive layer (430),
the second auxiliary layer (521) is in direct contact with the first emission layer (531),
the third auxiliary layer (522) is in direct contact with the second emission layer (532), and
the fourth auxiliary layer (523) is in direct contact with the third emission layer (533); and/or
(ii) a material for forming the first auxiliary layer (427), the second auxiliary layer (521), the third auxiliary layer (522), and the fourth auxiliary layer (523) comprises a fluorene-based compound, a carbazole-based compound, an arylamine-based compound, a dibenzofuran-based compound, a dibenzothiophene-based compound, and/or a dibenzosilole-based compound; and/or
(iii) the plurality of the organic light-emitting devices (501, 502, 503)) and the organic photodiode (400) form a pixel, and the electronic apparatus (200) comprises a plurality of the pixels.

## Patentansprüche

1. Elektronische Einrichtung (200), umfassend:
ein Substrat (601);
eine Vielzahl organischer lichtemittierender Vorrichtungen (501, 502, 503) **und** eine organische Photodiode (400), die auf dem Substrat (601) angeordnet sind, wobei:
die Vielzahl organischer lichtemittierender Vorrichtungen (501, 502, 503) eine erste lichtemittierende Vorrichtung (501), eine zweite lichtemittierende Vorrichtung (502) **und** eine dritte lichtemittierende Vorrichtung (503) umfasst,
die organische Photodiode (400) nacheinander eine erste Elektrode (410), eine erste Hilfsschicht (427), eine photoaktive Schicht (430) **und** eine gemeinsame Elektrode (450) umfasst,
die erste lichtemittierende Vorrichtung (501) nacheinander eine zweite Elektrode (511), eine zweite Hilfsschicht (521), eine erste Emissionsschicht (531) **und** eine gemeinsame Elektrode (450) umfasst,
die zweite lichtemittierende Vorrichtung (502) nacheinander eine dritte Elektrode (512), eine dritte Hilfsschicht (522), eine zweite Emissionsschicht (532) **und** eine gemeinsame Elektrode (450) umfasst,
die dritte lichtemittierende Vorrichtung (503) nacheinander eine vierte Elektrode (513), eine vierte Hilfsschicht (523), eine dritte Emissionsschicht (533) **und** eine gemeinsame Elektrode (450) umfasst,
die photoaktive Schicht (430) eine Dicke im Bereich von etwa 200 Å bis etwa 300 Å aufweist,
die zweite Hilfsschicht (521), die dritte Hilfsschicht (522) und die vierte Hilfsschicht (523) jeweils unterschiedliche Dicken aufweisen, und
eine Dicke der ersten Hilfsschicht (427) identisch mit einer Dicke einer Schicht ist, die aus der zweiten Hilfsschicht (521), der dritten Hilfsschicht (522) und der vierten Hilfsschicht (523) ausgewählt ist.

2. Elektronische Einrichtung (200) nach Anspruch 1, wobei die erste Emissionsschicht (531) bis zur dritten Emissionsschicht (533) jeweils eine Dicke im Bereich von etwa 150 Å bis etwa 500 Å aufweisen.

3. Elektronische Einrichtung (200) nach Anspruch 1 oder Anspruch 2, wobei Dicken der zweiten Hilfsschicht (521), der dritten Hilfsschicht (522) und der vierten Hilfsschicht (523) jeweils proportional zur Wellenlänge des von der ersten lichtemittierenden Vorrichtung (501), der zweiten lichtemittierenden Vorrichtung (502) und der dritten lichtemittierenden Vorrichtung (503) emittierten Lichts ist.

4. Elektronische Einrichtung (200) nach einem der Ansprüche 1 bis 3, wobei:
die erste lichtemittierende Vorrichtung (501) rotes Licht emittiert,
die zweite lichtemittierende Vorrichtung (502) grünes Licht emittiert, und
die dritte lichtemittierende Vorrichtung (503) blaues Licht emittiert.

5. Elektronische Einrichtung (200) nach Anspruch 4, wobei ein Resonanzabstand der organischen Photodiode (400) mit dem der zweiten lichtemittierenden Vorrichtung (502) identisch ist.

6. Elektronische Einrichtung (200) nach Anspruch 4 oder 5, wobei:
die erste Hilfsschicht (427) eine gleiche Dicke wie eine Dicke der zweite Hilfsschicht (521) aufweist.

7. Elektronische Einrichtung (200) nach Anspruch 4 oder Anspruch 5, wobei die organische Photodiode (400) Licht detektiert, das von der zweiten lichtemittierenden Vorrichtung (502) emittiert und von einem Subjekt reflektiert wird.

8. Elektronische Einrichtung (200) nach einem der Ansprüche 1 bis 7, wobei die photoaktive Schicht (430) einen p-Typ-Halbleiter und einen n-Typ-Halbleiter umfasst.

9. Elektronische Einrichtung (200) nach Anspruch 8, wobei:
(i) die photoaktive Schicht (430) eine planare Heter-Übergangsstruktur des p-Typ-Halbleiters und n-Typ-Halbleiters aufweist; und/oder
(ii) die photoaktive Schicht (430) eine Bulk-Hetero-Übergangsstruktur des p-Typ-Halbleiters und n-Typ-Halbleiters aufweist.

10. Elektronische Einrichtung (200) nach Anspruch 8 oder 9, wobei der p-Typ-Halbleiter eine Donorverbindung umfasst, die durch Formel 110 dargestellt ist: 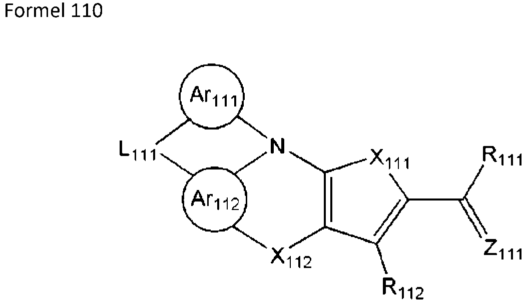 wobei in Formel 110,
Ar₁₁₁ und Ar₁₁₂ jeweils unabhängig voneinander eine C₆-C₃₀ Arylengruppe sind, die unsubstituiert oder mit mindestens einem R₁₀ₐ substituiert ist, oder eine C₃-C₃₀ Heteroarylengruppe, die unsubstituiert oder mit mindestens einem R₁₀ₐsubstituiert ist,
X₁₁₁ aus -Se-, -Te-, -S(=O)-, -S(=O)₂-, -N(Q₁₁₁)-, -B(Q₁₁₁)-, -C(Q₁₁₁)(Q₁₁₂)-, - Si(O₁₁₁)(O₁₁₂)-, und -Ge(Q₁₁₁)(Q₁₁₂)-, ausgewählt ist,
X₁₁₂ und L₁₁₁ jeweils unabhängig voneinander aus -O-, -S-, -Se-, -Te-, -S(=O)-, -S(=O)₂- , -N(Q₁₁₁)-, -B(Q₁₁₂)-, -C(Q₁₁₁)(Q₁₁₂)-, -Si(Q₁₁₁)(Q₁₁₂)-, -Ge(Q₁₁₁)(Q₁₁₂)-, -(C(Q₁₁₁)=C(₁₁₂))-, und -(C(Q₁₁₁)=N))-, ausgewählt sind,
wenn L₁₁₁ aus -N(Q₁₁₁)-, -B(Q₁₁₁)-, -C(Q₁₁₁)(Q₁₁₂)-, -Si(Q₁₁₁)(O₁₁₂)-, -Ge(Q₁₁₁)(Q₁₁₂)-, - (C(Q₁₁₁)=C(Q₁₁₂))-, und -(C(Q₁₁₁)=N))-, ausgewählt ist, ist L₁₁₁ optional mit Ar₁₁₁ oder A₁₁₂ verknüpft, um einen kondensierten Ring zu bilden,
Z₁₁₁ eine C₆-C₃₀ carbocyclische Gruppe ist, die unsubstituiert ist oder mit mindestens einem R₁₀ₐ substituiert ist, und mindestens eine funktionelle Gruppe aufweist, die ausgewählt ist aus C=O, C=S, C=Se, und C=Te, oder eine_{C1-C30-} heterocyclische Gruppe ist, die unsubstituiert ist oder mit mindestens einem R₁₀ₐ substituiert ist und mindestens eine funktionelle Gruppe aufweist, die ausgewählt ist aus C=O, C=S, C=Se, und C=Te,
R₁₁₁ bis R₁₁₂ jeweils unabhängig voneinander Wasserstoff, Deuterium, ein Halogen, eine Cyanogruppe, eine Nitrogruppe, eine Hydroxygruppe, eine C₁-C₃₀ Alkylgruppe, die unsubstituiert ist oder mit mindestens einem R₁₀ₐsubstituiert ist, eine C₁-C₃₀ Alkoxygruppe, die unsubstituiert ist oder mit mindestens einem R₁₀ₐsubstituiert ist, eine C₆-C₃₀ Arylgruppe, die unsubstituiert ist oder mit mindestens einem R₁₀ₐsubstituiert ist, eine C₃-C₃₀ Heteroarylgruppe, die unsubstituiert oder mit mindestens einem R₁₀ₐsubstituiert ist, eine C₂-C₃₀ Acylgruppe, die unsubstituiert ist oder mit mindestens einem R₁₀ₐsubstituiert ist, oder eine beliebige Kombination davon sind,
R₁₀ₐ ist:
Deuterium, -F, -Cl, -Br, -I, eine Hydroxylgruppe, eine Cyanogruppe oder eine Nitrogruppe; eine C₁-C₆₀ Alkylgruppe, eine C₂-C₆₀ Alkenylgruppe, eine C₂-C₆₀ Alkinylgruppe oder eine C₁-C₆₀ Alkoxygruppe, jeweils unsubstituiert oder substituiert mit Deuterium, -F, -Cl, -Br, -I, einer Hydroxylgruppe, einer Cyanogruppe, einer Nitrogruppe, eine C₃-C₆₀ carbocyclischen Gruppe, eine C₁-C₆₀ heterocyclische Gruppe, eine C₆-C₆₀ Aryloxygruppe, eine C₆-C₆₀ Arylthiogruppe, eine C₁-C₆₀ Heteroaryloxygruppe, eine C₁-C₆₀ Heteroarylthiogruppe, -Si(Q₁₁)(Q₁₂)(Q₁₃), -N(Q_{11 )(Q 12)}, -B(Q₁₁)(Q₁₂), -C(=O)(Q₁₁), - S(=O)₂(O₁₁), -P(=O)(Q₁₁)(Q₁₂), oder eine beliebige Kombination davon;
eine C₃-C₆₀ carbocyclische Gruppe, eine C₁-C₆₀- heterocyclische Gruppe, eine C₆-_{C}60 Aryloxygruppe, eine C₆-C₆₀₋ Arylthiogruppe, eine C₁-C₆₀₋ Heteroaryloxygruppe oder eine C₁-C₆₀₋ Heteroarylthiogruppe, jeweils unsubstituiert oder substituiert mit Deuterium, -F, -Cl, -Br, -I, eine Hydroxylgruppe, eine Cyanogruppe, eine Nitrogruppe, eine C₁-C₆₀ Alkylgruppe, eine C₂-C₆₀ Alkenylgruppe, eine C₂-C₆₀ Alkinylgruppe, eine C₁-C₆₀ Alkoxygruppe, eine C₃-C₆₀ carbocyclische Gruppe, eine C₁-C₆₀ heterocyclische Gruppe, eine C₆-C₆₀ Aryloxygruppe, eine C₆-C₆₀ Arylthiogruppe, eine C₁-C₆₀ Heteroaryloxygruppe, eine C₁-C₆₀ Heteroarylthiogruppe, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂) oder eine beliebige Kombination davon; oder
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), oder - P(=O)(Q₃₁)(Q₃₂), und
Q₁₁ bis Q₁₃, Q₂₁ bis Q₂₃, Q₃, bis Q₃₃, Q₁₁₁, und Q₁₁₂ jeweils unabhängig voneinander sind: Wasserstoff; Deuterium; -F; -Cl; -Br; -I; eine Hydroxylgruppe; eine Cyanogruppe; eine Nitrogruppe; eine C₁-C₆₀ Alkylgruppe; eine C₂-C₆₀ Alkenylgruppe; eine C₂-C₆₀ Alkinylgruppe; eine C₁-C₆₀ Alkoxygruppe; oder eine C₃-C₆₀ carbocyclische Gruppe oder eine C₁-C₆₀ heterocyclische Gruppe, jeweils unsubstituiert oder substituiert mit Deuterium, -F, eine Cyanogruppe, eine C₁-C₆₀ Alkylgruppe, eine C₁-C₆₀ Alkoxygruppe, eine Phenylgruppe, eine Biphenylgruppe oder eine beliebige Kombination davon.

11. Elektronische Einrichtung (200) nach einem der Ansprüche 8 bis 10, wobei der n-Typ-Halbleiter eine Verbindung umfasst, die durch Formel 120 dargestellt ist: 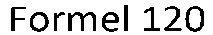 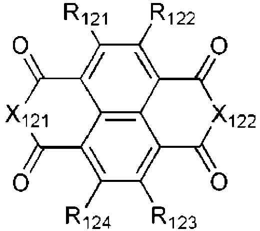 wobei in Formel 120
X₁₂₁ und X₁₂₂ jeweils unabhängig O oder NR₁₂₅ sind,
R₁₂₁ bis R₁₂₄ und R₁₂₅ jeweils unabhängig voneinander Wasserstoff, Deuterium, ein Halogen, eine Cyanogruppe, eine Nitrogruppe, eine Hydroxygruppe, eine C₁-C₃₀ Alkylgruppe, die mit mindestens einem R₁₀ₐsubstituiert oder unsubstituiert ist, eine C₆-C₃₀ Arylgruppe, die mit mindestens einem R₁₀ₐsubstituiert oder unsubstituiert ist, eine C₃-C₃₀ Heteroarylgruppe, die mit mindestens einem R₁₀ₐsubstituiert oder unsubstituiert ist, oder eine beliebige Kombination davon sind, und
R₁₀ₐ ist:
Deuterium, -F, -Cl, -Br, -I, eine Hydroxylgruppe, eine Cyanogruppe oder eine Nitrogruppe; eine C₁-C₆₀ Alkylgruppe, eine C₂-C₆₀ Alkenylgruppe, eine C₂-C₆₀ Alkinylgruppe oder eine C₁-C₆₀ Alkoxygruppe, jeweils unsubstituiert oder substituiert mit Deuterium, -F, -Cl, -Br, -I, einer Hydroxylgruppe, einer Cyanogruppe, einer Nitrogruppe, eine C₃-C₆₀ carbocyclischen Gruppe, eine C₁-C₆₀ heterocyclische Gruppe, eine C₆-C₆₀ Aryloxygruppe, eine C₆-C₆₀ Arylthiogruppe, eine C₁-C₆₀ Heteroaryloxygruppe, eine C₁-C₆₀ Heteroarylthiogruppe, -Si(Q₁₁)(Q₁₂)(Q₁₂), -N(Q_{11 )(Q 12)}, -B(Q₁₁)(Q₁₂), -C(=O)(Q₁₁), - S(=O)₂(Q₁₁), -P(=O)(Q₁₁)(Q₁₂), oder eine beliebige Kombination davon;
eine C₃-C₆₀ carbocyclische Gruppe, eine C₁-C₆₀₋ heterocyclische Gruppe, eine C_{6-C}60 Aryloxygruppe, eine C₆-C₆₀₋ Arylthiogruppe, eine C₁-C₆₀₋ Heteroaryloxygruppe oder eine C₁-C₆₀₋ Heteroarylthiogruppe, jeweils unsubstituiert oder substituiert mit Deuterium, -F, -Cl, -Br, -I, eine Hydroxylgruppe, eine Cyanogruppe, eine Nitrogruppe, eine C₁-C₆₀ Alkylgruppe, eine C₂-C₆₀ Alkenylgruppe, eine C₂-C₆₀ Alkinylgruppe, eine C₁-C₆₀ Alkoxygruppe, eine C₃-C₆₀ carbocyclische Gruppe, eine C₁-C₆₀ heterocyclische Gruppe, eine C₆-C₆₀ Aryloxygruppe, eine C₆-C₆₀ Arylthiogruppe, eine C₁-C₆₀ Heteroaryloxygruppe, eine C₁-C₆₀ Heteroarylthiogruppe, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂) oder eine beliebige Kombination davon; oder
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), oder - P(=O)(Q₃₁)(Q₃₂), und
Q₁₁ bis Q₁₃, Q₂₁ bis Q₂₃, und Q₃₁, Q₃₂ jeweils unabhängig voneinander sind: Wasserstoff; Deuterium; -F; -Cl; -Br; -I; eine Hydroxylgruppe; eine Cyanogruppe; eine Nitrogruppe; eine C₁-C₆₀ Alkylgruppe; eine C₂-C₆₀ Alkenylgruppe; eine C₂-C₆₀ Alkinylgruppe; eine C₁-C₆₀ Alkoxygruppe; oder eine C₃-C₆₀ carbocyclische Gruppe oder eine C₁-C₆₀ heterocyclische Gruppe, jeweils unsubstituiert oder substituiert mit Deuterium, -F, einer Cyanogruppe, eine C₁-C₆₀ Alkylgruppe, eine C₁-C₆₀ Alkoxygruppe, eine Phenylgruppe, einer Biphenylgruppe oder eine beliebigen Kombination davon.

12. Elektronische Einrichtung (200) nach einem der Ansprüche 1 bis 11, wobei:
(i) eine erste gemeinsame Schicht (420) zwischen der ersten Elektrode (410) und der ersten Hilfsschicht (427), zwischen der zweiten Elektrode (511) und der zweiten Hilfsschicht (521), zwischen der dritten Elektrode (512) und der dritten Hilfsschicht (522) sowie zwischen der vierten Elektrode (513) und der vierten Hilfsschicht (523) eingefügt ist; und/oder
(ii) eine zweite gemeinsame Schicht (440) zwischen der photoaktiven Schicht (430) und der gemeinsamen Elektrode (450), zwischen der ersten Emissionsschicht (531) und der gemeinsamen Elektrode (450), zwischen der zweiten Emissionsschicht (532) und der gemeinsamen Elektrode (450) sowie zwischen der dritten Emissionsschicht (533) und der gemeinsamen Elektrode (450) eingefügt ist.

13. Elektronische Einrichtung (200) nach einem der Ansprüche 10 bis 12, wobei die erste gemeinsame Schicht (420) mindestens eine ausgewählt aus einer Lochinjektionsschicht, einer Lochtransportschicht und einer Elektronenblockierschicht umfasst.

14. Elektronische Einrichtung nach einem der Ansprüche 11 bis 13, wobei die zweite gemeinsame Schicht (440) mindestens eine ausgewählt aus einer Pufferschicht, einer Lochblockierschicht, einer Elektronentransportschicht und einer Elektroneninjektionsschicht umfasst.

15. Elektronische Einrichtung (200) nach einem der Ansprüche 1 bis 14, wobei:
( i) die erste Hilfsschicht (427) in direktem Kontakt mit der photoaktiven Schicht (430) steht,
die zweite Hilfsschicht (521) in direktem Kontakt mit der ersten Emissionsschicht (531) steht,
die dritte Hilfsschicht (522) in direktem Kontakt mit der zweiten Emissionsschicht (532) steht, und
die vierte Hilfsschicht (523) in direktem Kontakt mit der dritten Emissionsschicht (533) steht; und/oder
(ii) ein Material zur Bildung der ersten Hilfsschicht (427), der zweiten Hilfsschicht (521), der dritten Hilfsschicht (522) und der vierten Hilfsschicht (523) eine Fluorenbasierte Verbindung, eine Carbazol-basierte Verbindung, eine Arylamin-basierte Verbindung, eine Dibenzofuran-basierte Verbindung, eine Dibenzothiophen-basierte Verbindung, und/oder eine Verbindung auf Dibenzosilolbasis umfasst; und/oder
( iii) die Vielzahl der organischen lichtemittierenden Vorrichtungen (501, 502, 503) und die organische Photodiode (400) ein Pixel bilden, und die elektronische Einrichtung (200) eine Vielzahl von Pixeln umfasst.

## Revendications

1. Appareil électronique (200) comprenant :
un substrat (601) ;
une pluralité de dispositifs électroluminescents organiques (501, 502, 503) et une photodiode organique (400) qui sont agencés sur le substrat (601), dans lequel :
la pluralité de dispositifs électroluminescents organiques (501, 502, 503) comprennent un premier dispositif électroluminescent (501), un deuxième dispositif électroluminescent (502) et un troisième dispositif électroluminescent (503),
la photodiode organique (400) comprend séquentiellement une première électrode (410), une première couche auxiliaire (427), une couche photoactive (430) et une électrode commune (450),
le premier dispositif électroluminescent (501) comprend séquentiellement une deuxième électrode (511), une deuxième couche auxiliaire (521), une première couche d'émission (531) et une électrode commune (450),
le deuxième dispositif électroluminescent (502) comprend séquentiellement une troisième électrode (512), une troisième couche auxiliaire (522), une deuxième couche d'émission (532) et une électrode commune (450),
le troisième dispositif électroluminescent (503) comprend séquentiellement une quatrième électrode (513), une quatrième couche auxiliaire (523), une troisième couche d'émission (533) et une électrode commune (450),
la couche photoactive (430) présente une épaisseur dans une plage d'environ 200 Å à environ 300 Å,
la deuxième couche auxiliaire (521), la troisième couche auxiliaire (522) et la quatrième couche auxiliaire (523) présentent des épaisseurs différentes les unes des autres, et
une épaisseur de la première couche auxiliaire (427) est identique à une épaisseur d'une couche sélectionnée parmi la deuxième couche auxiliaire (521), la troisième couche auxiliaire (522) et la quatrième couche auxiliaire (523).

2. Appareil électronique (200) selon la revendication 1, dans lequel les première (531) à troisième (533) couches d'émission présentent chacune une épaisseur dans une plage d'environ 150 Å à environ 500 Å.

3. Appareil électronique (200) selon la revendication 1 ou la revendication 2, dans lequel les épaisseurs de la deuxième couche auxiliaire (521), de la troisième couche auxiliaire (522) et de la quatrième couche auxiliaire (523) sont chacune proportionnelle à la longueur d'onde de la lumière émise par chacun du premier dispositif électroluminescent (501), du deuxième dispositif électroluminescent (502) et du troisième dispositif électroluminescent (503).

4. Appareil électronique (200) selon l'une quelconque des revendications 1 à 3, dans lequel :
le premier dispositif électroluminescent (501) émet une lumière rouge,
le deuxième dispositif électroluminescent (502) émet une lumière verte, et
le troisième dispositif électroluminescent (503) émet une lumière bleue.

5. Appareil électronique (200) selon la revendication 4, dans lequel une distance de résonance de la photodiode organique (400) est identique à celle du deuxième dispositif électroluminescent (502).

6. Appareil électronique (200) selon la revendication 4 ou la revendication 5, dans lequel :
la première couche auxiliaire (427) présente une épaisseur identique à une épaisseur de la deuxième couche auxiliaire (521).

7. Appareil électronique (200) selon la revendication 4 ou la revendication 5, dans lequel la photodiode organique (400) détecte la lumière émise par le deuxième dispositif électroluminescent (502) et réfléchie par un sujet.

8. Appareil électronique (200) selon l'une quelconque des revendications 1 à 7, dans lequel la couche photoactive (430) comprend un semi-conducteur de type p et un semi-conducteur de type n.

9. Appareil électronique (200) selon la revendication 8, dans lequel :
(i) la couche photoactive (430) présente une structure d'hétérojonction planaire du semi-conducteur de type p et du semi-conducteur de type n ; et/ou
(ii) la couche photoactive (430) présente une structure d'hétérojonction en volume du semi-conducteur de type p et du semi-conducteur de type n.

10. Appareil électronique (200) selon la revendication 8 ou la revendication 9, dans lequel le semi-conducteur de type p comprend un composé donneur représenté par la Formule 110 : 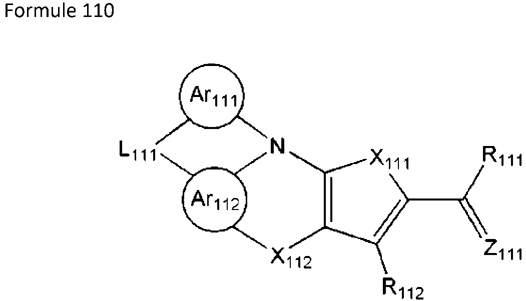 dans lequel, dans la Formule 110,
Ar₁₁₁ et Ar₁₁₂ sont chacun indépendamment un groupe arylène en C₆-C₃₀ qui est non substitué ou substitué par au moins un R₁₀ₐ ou un groupe hétéroarylène en C₃-C₃₀ qui est non substitué ou substitué par au moins un R₁₀ₐ,
X₁₁₁ est sélectionné parmi -Se-, -Te-, -S(=O)-, -S(=O)₂-, -N(Q₁₁₁)-, -B(Q₁₁₁)-, - C(Q₁₁₁)(Q₁₁₂)-, -Si(Q₁₁₁)(Q₁₁₂)-, et -Ge(Q₁₁₁)(Q₁₁₂)-,
X₁₁₂ et L₁₁₁ sont chacun indépendamment sélectionné parmi -O-, -S-, -Se-, -Te-, - S(=O)-, -S(=O)₂-, -N(Q₁₁₁)-, -B(Q₁₁₁)-, -C(Q₁₁₁)(Q₁₁₂)-, -Si(Q₁₁₁)(Q₁₁₂)-, -Ge(Q₁₁₁)(Q₁₁₂)-, - (C(Q₁₁₁)=C(₁₁₂))-, et -(C(Q₁₁₁)=N))-,
lorsque L₁₁₁ est sélectionné parmi -N(Q₁₁₁)-, -B(Q₁₁₁)-, -C(Q₁₁₁)(Q₁₁₂)-, -Si(Q₁₁₁)(Q₁₁₂)-, -Ge(Q₁₁₁)(Q₁₁₂)-, -(C(Q₁₁₁)=C(Q₁₁₂))-, et -(C(Q₁₁₁)=N))-, L₁₁₁ est éventuellement lié à Ar₁₁₁ ou à A₁₁₂ pour former un noyau condensé,
Z₁₁₁ est un groupe carbocyclique en C₆-C₃₀ qui est non substitué ou substitué par au moins un R₁₀ₐ et présente au moins un groupe fonctionnel sélectionné parmi C=O, C=S, C=Se, et C=Te, ou un groupe hétérocyclique en C₁-C₃₀ qui est non substitué ou substitué par au moins un R₁₀ₐ et présente au moins un groupe fonctionnel sélectionné parmi C=O, C=S, C=Se, et C=Te,
R₁₁₁ à R₁₁₂ sont chacun indépendamment hydrogène, deutérium, un halogène, un groupe cyano, un groupe nitro, un groupe hydroxy, un groupe alkyle en C₁-C₃₀ non substitué ou substitué par au moins un R₁₀ₐ, un groupe alcoxy en C₁-C₃₀ non substitué ou substitué par au moins un R₁₀ₐ, un groupe aryle en C₆-C₃₀ non substitué ou substitué par au moins un R₁₀ₐ, un groupe hétéroaryle en C₃-C₃₀ non substitué ou substitué par au moins un R₁₀ₐ, un groupe acyle en C₂-C₃₀ non substitué ou substitué par au moins un R₁₀ₐ, ou toute combinaison de ceux-ci,
R₁₀ₐ est :
deutérium, -F, -Cl, -Br, -I, un groupe hydroxyle, un groupe cyano, ou un groupe nitro ; un groupe alkyle en C₁-C₆₀, un groupe alcényle en C₂-C₆₀, un groupe alcynyle en C₂-C₆₀ ou un groupe alcoxy en C₁-C₆₀, chacun non substitué ou substitué par deutérium, -F, -Cl, -Br, -I, un groupe hydroxyle, un groupe cyano, un groupe nitro, un groupe carbocyclique en C₃-C₆₀, un groupe hétérocyclique en C₁-C₆₀, un groupe aryloxy en C₆-C₆₀, un groupe arylthio en C₆-C₆₀, un groupe hétéroaryloxy en C₁-C₆₀, un groupe hétéroarylthio en C₁-C₆₀, -Si(Q₁₁)(Q₁₂)(Q₁₃), -N(Q₁₁)(Q₁₂), -B(Q₁₁)(Q₁₂), -C(=O)(Q₁₁), -S(=O)₂(Q₁₁), - P(=O)(Q₁₁)(Q₁₂), ou toute combinaison de ceux-ci ;
un groupe carbocyclique en C₃-C₆₀, un groupe hétérocyclique en C₁-C₆₀, un groupe aryloxy en C₆-C₆₀, un groupe arylthio en C₆-C₆₀, un groupe hétéroaryloxy en C₁-C₆₀, ou un groupe hétéroarylthio en C₁-C₆₀, chacun non substitué ou substitué par deutérium, -F, -Cl, -Br, -I, un groupe hydroxyle, un groupe cyano, un groupe nitro, un groupe alkyle en C₁-C₆₀, un groupe alcényle en C₂-C₆₀, un groupe alcynyle en C₂-C₆₀, un groupe alcoxy en C₁-C₆₀, un groupe carbocyclique en C₃-C₆₀, un groupe hétérocyclique en C₁-C₆₀, un groupe aryloxy en C₆-C₆₀, un groupe arylthio en C₆-C₆₀, un groupe hétéroaryloxy en C₁-C₆₀, un groupe hétéroarylthio en C₁-C₆₀, -Si(Q₂₁)(Q₂₂)(Q₂₃), -N(Q₂₁)(Q₂₂), -B(Q₂₁)(Q₂₂), -C(=O)(G₂₁), -S(=O)₂(Q₂₁), -P(=O)(Q₂₁)(Q₂₂), ou toute combinaison de ceux-ci ; ou
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), ou - P(=O)(Q₃₁)(Q₃₂), et
Q₁₁ à Q₁₃, Q₂₁ à Q₂₃, Q₃₁ à Q₃₃, Q₁₁₁, et Q₁₁₂ sont chacun indépendamment : hydrogène ; deutérium ; -F ; -Cl ; -Br ; -I ; un groupe hydroxyle ; un groupe cyano ; un groupe nitro ; un groupe alkyle en C₁-C₆₀; un groupe alcényle en C₂-C₆₀; un groupe alcynyle en C₂-C₆₀ ; un groupe alcoxy en C₁-C₆₀; ou un groupe carbocyclique en C₃-C₆₀ ou un groupe hétérocyclique en C₁-C₆₀, chacun non substitué ou substitué par deutérium, -F, un groupe cyano, un groupe alkyle en C₁-C₆₀, un groupe alcoxy en C₁-C₆₀, un groupe phényle, un groupe biphényle ou toute combinaison de ceux-ci.

11. Appareil électronique (200) selon l'une quelconque des revendications 8 à 10, dans lequel le semi-conducteur de type n comprend un composé représenté par la Formule 120 : 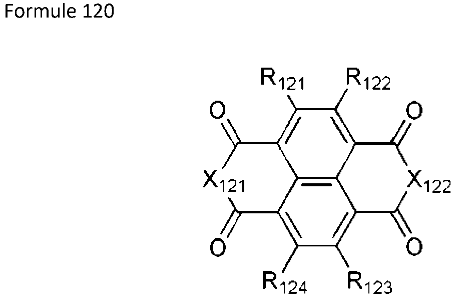 dans lequel, dans la Formule 120,
X₁₂₁ et X₁₂₂ sont chacun indépendamment O ou NR₁₂₅,
R₁₂₁ à R₁₂₄ et R₁₂₅ sont chacun indépendamment hydrogène, deutérium, un halogène, **un** groupe cyano, un groupe nitro, un groupe hydroxy, un groupe alkyle en C₁-C₃₀ substitué ou non substitué par au moins un R₁₀ₐ, un groupe aryle en C₆-C₃₀ substitué ou non substitué par au moins un R₁₀ₐ, un groupe hétéroaryle en C₃-C₃₀ substitué ou non substitué par au moins un R₁₀ₐ, ou toute combinaison de ceux-ci, et
R₁₀ₐ est :
deutérium, -F, -Cl, -Br, -I, un groupe hydroxyle, un groupe cyano, ou un groupe nitro ; un groupe alkyle en C₁-C₆₀, un groupe alcényle en C₂-C₆₀, un groupe alcynyle en C₂-C₆₀ ou un groupe alcoxy en C₁-C₆₀, chacun non substitué ou substitué par deutérium, -F, -Cl, -Br, -I, un groupe hydroxyle, un groupe cyano, un groupe nitro, un groupe carbocyclique en C₃-C₆₀, un groupe hétérocyclique en C₁-C₆₀, un groupe aryloxy en C₆-C₆₀, un groupe arylthio en C₆-C₆₀, un groupe hétéroaryloxy en C₁-C₆₀, un groupe hétéroarylthio en C₁-C₆₀, -Si(O₁₁)(O₁₂)(O₁₃), -N(Q₁₁)(O₁₂), -B(O₁₁)(O₁₂), -C(=O)(Q₁₁), -S(=O)₂(O₁₁), - P(=O)(Q₁₁)(Q₁₂), ou toute combinaison de ceux-ci ;
un groupe carbocyclique en C₃-C₆₀, un groupe hétérocyclique en C₁-C₆₀, un groupe aryloxy en C₆-C₆₀, un groupe arylthio en C₆-C₆₀, un groupe hétéroaryloxy en C₁-C₆₀, ou un groupe hétéroarylthio en C₁-C₆₀, chacun non substitué ou substitué par deutérium, -F, -Cl, -Br, -I, un groupe hydroxyle, un groupe cyano, un groupe nitro, un groupe alkyle en C₁-C₆₀, un groupe alcényle en C₂-C₆₀, un groupe alcynyle en C₂-C₆₀, un groupe alcoxy en C₁-C₆₀, un groupe carbocyclique en C₃-C₆₀, un groupe hétérocyclique en C₁-C₆₀, un groupe aryloxy en C₆-C₆₀, un groupe arylthio en C₆-C₆₀, un groupe hétéroaryloxy en C₁-C₆₀, un groupe hétéroarylthio en C₁-C₆₀, -Si(Q₂₁)(Q₂₂)(Q₂₃), -N(Q₂₁)(Q₂₂), -B(Q₂₁)(Q₂₂), -C(=O)(Q₂₁), -S(=O)₂(Q₂₁), -P(=O)(Q₂₁)(Q₂₂), ou toute combinaison de ceux-ci ; ou
-Si(Q₃₁)(Q₃₂)(Q₃₃), -N(Q₃₁)(Q₃₂), -B(Q₃₁)(Q₃₂), -C(=O)(Q₃₁), -S(=O)₂(Q₃₁), ou - P(=O)(Q₃₁)(Q₃₂), et
Q₁₁ à Q₁₃, Q₂₁ à Q₂₃ et Q₃₁, Q₃₂ sont chacun indépendamment : hydrogène ; deutérium ; -F ; -Cl ; -Br ; -I ; un groupe hydroxyle ; un groupe cyano ; un groupe nitro ; un groupe alkyle en C₁-C₆₀; un groupe alcényle en C₂-C₆₀ ; un groupe alcynyle en C₂-C₆₀ ; un groupe alcoxy en C₁-C₆₀; ou un groupe carbocyclique en C₃-C₆₀ ou un groupe hétérocyclique en C₁-C₆₀, chacun non substitué ou substitué par deutérium, -F, un groupe cyano, un groupe alkyle en C₁-C₆₀, un groupe alcoxy en C₁-C₆₀, un groupe phényle, un groupe biphényle ou toute combinaison de ceux-ci.

12. Appareil électronique (200) selon l'une quelconque des revendications 1 à 11, dans lequel :
(i) une première couche commune (420) est incluse entre la première électrode (410) et la première couche auxiliaire (427), entre la deuxième électrode (511) et la deuxième couche auxiliaire (521), entre la troisième électrode (512) et la troisième couche auxiliaire (522), et entre la quatrième électrode (513) et la quatrième couche auxiliaire (523) ; et/ou
(ii) une seconde couche commune (440) est incluse entre la couche photoactive (430) et l'électrode commune (450), entre la première couche d'émission (531) et l'électrode commune (450), entre la deuxième couche d'émission (532) et l'électrode commune (450), et entre la troisième couche d'émission (533) et l'électrode commune (450).

13. Appareil électronique (200) selon l'une quelconque des revendications 10 à 12, dans lequel la première couche commune (420) comprend au moins une couche sélectionnée parmi une couche d'injection de trous, une couche de transport de trous et une couche de blocage d'électrons.

14. Appareil électronique selon l'une quelconque des revendications 11 à 13, dans lequel la seconde couche commune (440) comprend au moins une couche sélectionnée parmi une couche tampon, une couche de blocage de trous, une couche de transport d'électrons et une couche d'injection d'électrons.

15. Appareil électronique (200) selon l'une quelconque des revendications 1 à 14, dans lequel :
(i) la première couche auxiliaire (427) est en contact direct avec la couche photoactive (430),
la deuxième couche auxiliaire (521) est en contact direct avec la première couche d'émission (531),
la troisième couche auxiliaire (522) est en contact direct avec la deuxième couche d'émission (532), et
la quatrième couche auxiliaire (523) est en contact direct avec la troisième couche d'émission (533) ; et/ou
(ii) un matériau destiné à former la première couche auxiliaire (427), la deuxième couche auxiliaire (521), la troisième couche auxiliaire (522) et la quatrième couche auxiliaire (523) comprend un composé à base de fluorène, un composé à base de carbazole, un composé à base d'arylamine, un composé à base de dibenzofurane, un composé à base de dibenzothiophène, et/ou un composé à base de dibenzosilole ; et/ou
(iii) la pluralité des dispositifs électroluminescents organiques (501, 502, 503) et la photodiode organique (400) forment un pixel, et l'appareil électronique (200) comprend une pluralité des pixels.
